# EUROPEAN PATENT SPECIFICATION

(11) **EP 1 319 659 B1**
(45) Date of publication and mention of the grant of the patent: **11.03.2009**
(21) Application number: 01970120.0
(22) Date of filing: 17.09.2001
(51) Int. Cl.: C07D 237/04, C07D 401/04, C07D 237/14, C07D 401/06, C07D 409/04, C07D 491/052, C07D 491/048, C07D 401/14, C07D 405/14, C07D 403/14, C07D 409/14, C07D 237/24, A61K 31/50, A61P 25/00

(54) **TRIAZINONES AND MEDICINAL USE THEREOF**
TRIAZINONE SOWIE DEREN MEDIZINALE ANWENDUNG
TRIAZINONES AINSI QUE LEUR UTILISATION MEDICALE

(30) Priority: 18.09.2000 JP 2000282636; 22.09.2000 JP 2000289412; 09.11.2000 JP 2000342614; 05.02.2001 GB 0102822; 05.02.2001 GB 0102824
(43) Date of publication of application: 18.06.2003
(73) Proprietor: Eisai R&D Management Co., Ltd., Tokyo 112-8088 (JP)
(72) Inventor: NAGATO, Satoshi, Matsudo-shi, Chiba 271-0076 (JP); KAWANO, Koki, Tsukuba-shi, Ibaraki 305-0045 (JP); ITO, Koichi, Abiko-shi, Chiba 270-1143 (JP); NORIMINE, Yoshihiko, Tsukuba-shi, Ibaraki 305-0061 (JP); UENO, Kohshi, Tsukuba-shi, Ibaraki 305-0042 (JP); HANADA, Takahisa, Tsukuba-shi, Ibaraki 305-0003 (JP); AMINO, Hiroyuki, Tsukuba-shi, Ibaraki 305-0821 (JP); OGO, Makoto, Tsukuba-shi, Ibaraki 305-0035 (JP); HATAKEYAMA, Shinji, Ushiku-shi, Ibaraki 300-1234 (JP); UENO, Masataka, Kitasouma-gun, Ibaraki 302-0121 (JP); GROOM, Anthony, John, Swindon, Wiltshire SN1 2PX (GB); RIVERS, Leanne, Tonbridge, Kent TN12 0SE (GB); SMITH, Terence, Cambridge, Cambridgeshire CB1 2LE (GB)
(74) Representative: HOFFMANN EITLE
(86) International application number: PCT/JP2001/008058
(87) International publication number: WO 2002/022587

(56) References cited:
- WO-A1-00/50408
- WO-A1-99/44995
- JP-A- 5 221 992
- JP-A- 8 225 540
- JP-A- 2000 119 257
- US-A- 5 426 106
- C. RUBAT ET AL.: "synthetic a. pharmaceutical evaluation of N-substituted 4,6-diaryl-3-pyridazinones as analgesic" CHEMICAL AND PHARMACEUTICAL BULLETIN., vol. 37, no. 10, 1989, pages 2832-2835, XP002331600 JPPHARMACEUTICAL SOCIETY OF JAPAN, TOKYO.
- EL-SAYED H. EL-ASHRY: "access to the 2,3-dihydropyridazin-3-one a. as-triazino [3,4-a]phtalazine ring systems" JOURNAL OF HETEROCYCLIC CHEMISTRY., vol. 24, no. 1, 1987, pages 63-68, XP002331601 USHETEROCORPORATION. PROVO.
- ADEL AMER: "dehydration of 2-(2-arylethyl)-2-hydroxy-4-oxopentanoic acids a. their hydrazones to form heterocycles" JOURNAL FUR PRAKTISCHE CHEMIE, CHEMIKER ZEITUNG., vol. 339, no. 1, 1997, pages 20-25, XP008048483 DEWILEY VCH, WEINHEIM.

## Description

### Field of the Invention

The present invention relates to a novel compound, a salt thereof and a hydrate of them, to methods for manufacturing the same, and to use thereof as pharmaceutical preparations. More specifically, it relates to pyridazinone and triazinone compounds useful as non-NMDA receptor inhibitors, particularly as AMPA receptor inhibitors.

### Prior Art

Glutamate and aspartate are important amino acids which participate in nerve functions such as recognition, memory, movement, respiration, cardiovascular adjustment and sensation and are called excitatory neurotransmitters as well. In the expression of their physiological activities, an interaction with a specific receptor is important and, generally, two types of receptors - an ion channel type and a G-protein coupled type - have been known. The former is further classified into N-methyl-D-aspartate (NMDA) receptor, α-amino-3-hydroxy-5-methyl-4-isoxazole propionic acid (AMPA) receptor, kainate receptor, etc. On the other hand, the amino acid as an excitatory neurotransmitter has been known to induce neurotoxicity by, for example, abnormal excitation of central nerves. It has been noted that the said toxicity is as serious as being accompanied by the death of nerve cells causing various nervous diseases. Main nervous diseases which have been known are cerebral ischemia, head injury, spinal cord injury, Alzheimer's disease, Parkinson's disease, amyotrophic lateral sclerosis (ALS), Huntington's chorea, AIDS nervous disturbance, epilepsy, neurodegenaration observed after the state of hypoxia, mental disorder, mobility disturbance, pain, spasticity, nervous disturbance by toxin in food, various neurodegenerative diseases, various mental diseases, chronic pain, migraine, cancer pain and pain caused by diabetic nervous disturbance. They are serious diseases where many mechanisms of onset, etc. have not been clarified yet and pharmaceutical agents which are effective for the therapy have not been found yet but it is believed that they are closely related to excessive release/accumulation of excitatory neurotransmitters, changes in expressing pattern of receptors, etc. For example, it has been reported that glutamate concentration in cerebrospinal fluid and plasma increases in stroke, cerebral ischemia, head injury and spinal cord injury (Castillo, J., Dazalos, A. and Noya, M., Lancet, 1997, 346:79-83; etc.). There is a report that neuropathy occurs when glutamate, NMDA, AMPA, kainate, etc. are excessively applied to nerve cells (Meldrum, B., Brain Res. Reviews, 18, 293, 1993). There are reports that, in Alzheimer's disease, β-amyloid protein enhances the neurotoxicity of glutamate and that it promotes the release of glutamate (Arias, C., Arrieta, I. and Tapia, R., J. Neurosci. Res., 1995, 41:561-566; etc.). In the case of Parkinson's disease, there are reports that L-dopa hydroxide activates the AMPA receptor (Cha, J. J., et. al., Neurosci. Lett., 1991, 132:55-58) and enhances the neurotoxicity (Olney, J. W., et. al., 1990, 108:269-272; Rosenberg, P. A., et. al., Proc. Natl. Acad. Sci. USA, 1991, 88:4865-4869) . There is another report that L-dopa promotes the generation of free radicals resulting in a rise of oxidative stress (Smith, T. S., et. al., Neuroreport, 1994, 5:1009-1011). In the case of Huntington's chorea, it is reported that a substance which inhibits the release of glutamate is effective in improving the symptoms. In the case of ALS, there are many reports showing the participation of glutamate in its pathology. There are some cases where the AIDS patients suffer from recognition nerve function deficiency and, even in such a nerve disease, participation of glutamate is suggested. For example, it is reported that gp 120 which is a glycoprotein in an envelope of HIV virus suppresses the uptake of glutamate by astrocytes (Dreyer, E. B., Eur. J. Neurosci., 1995, 7:2502-2507; Ushijima, H., et. al., Eur. J. Neurosci., 1995, 7:1353-1359) while a substance which inhibits the release of glutamate suppresses the neurodegeneration by gp 120 (Sindou, P., et. al. , J. Neurosci. 1994, 126:133-137; Muller, W. E. G., et. al., Eur. J. Pharmacol. Molec. Pharmacol., 1992, 226:209-214; Lipton, S. A., Neurology, 1992, 42:1403-1405). With regard to allergic encephalomyelitis, there is a report that, in the mice where the said inflammation takes place, enzyme which decomposes glutamate incorporated from outside of cells is deficient (Hardin-Pouzet, H. , Glia. , 1997, 20:79-85) . Olivopontocerebellar atrophy is a disease which is sometimes combined with Parkinson's disease and an antibody to GluR2 which is a subunit constituting the AMPA receptor has been found (Gahring, L. C. , Neurology, 1997, 48:494-500) and the relation between olivopontocerebellar atrophy and AMPA receptor is suggested. With regard to a report for epilepsy, it is reported that, in the mice which are unable to construct the GluR2 in AMPA receptor, Ca²⁺ permeability of the AMPA receptor increases whereby it is apt to cause a sudden onset resulting in death (Brusa, R., Science, 1995, 270:1677-1680). Besides the above, it is reported that NBQX (2,3-dihydroxy-6-nitro-7-sulfamoylbenz [f] quinoxaline; Sheardown, et al., Science, 247, 571, 1990) and other inhibiting compounds to AMPA receptors have antianxiety and anticonvulsant action (J. Pharmacol. Exp. Ther., 260, 742, 1992; Pharmacol. Biochem. Behavior, 1998, 60:119-124) and there are also reports for the connection of AMPA receptor/kainate receptor with urinary disturbance, drug abuse, pain, etc. (J. Pharmacol. Exp. Ther., 280, 894-904, 1997; Neuroscience Letters, 268:127-130, 1999).

It can be expected that the substances showing an antagonistic action to excitatory neurotransmitter receptors are useful for the therapy of the above-mentioned nerve diseases. At present, the usefulness of the substances having an antagonistic action to non-NMDA receptors such as AMPA receptor and kainate receptor is particularly expected. For example, it is reported that inhibitors of the interaction of glutamate with the AMPA and/or kainate receptor complex are useful in treating demyelinating disorders such as encephalitis, acute disseminated encephalomyelitis, acute demyelinating polyneuropathy (Guillain Barre syndrome), chronic inflammatory demyelinating polyneuropathy, multiple sclerosis, Marchifava-Bignami disease, central pontine myelinolysis, Devic syndrome, Balo disease, HIV- or HTLV-myelopathy, progressive multifocal leucoencephalopathy, a secondary demyelinating disorder; for example, CNS lupus erythematodes, polyarteritis nodosa, Sjogren syndrome, sarcoidosis, isolated cerebral vasculitis, etc. as secondary demyelinating disorders, etc. in WO00/01376. With regard to the compound having an inhibitory action to AMPA receptor and kainate receptor, there are reports for the following compounds for example.
(1) Competitive AMPA receptor-inhibiting compounds represented by the following formula. 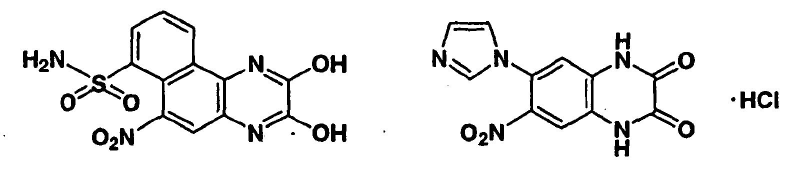 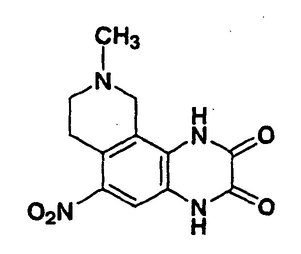
(2) Non-competitive AMPA receptor-inhibiting compounds represented by the following formula. 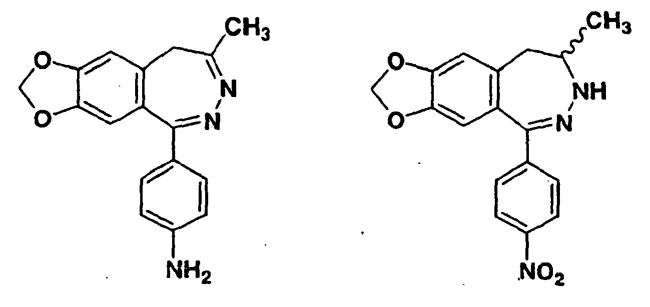 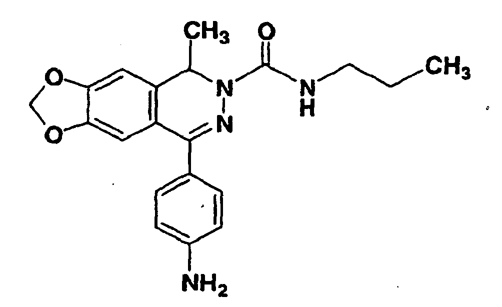
(3) Besides the above, there are reports on competitive AMPA receptor-inhibiting compounds having a quinoxalinedione skeleton in WO 94/25469, WO 96/10023, US 5356902, etc. and there are reports on non-competitive AMPA receptor-inhibiting compounds inWO 95/01357, WO 97/28135, WO 97/28163, WO 97/43276, WO 97/34878, WO 98/38173, EP 802195, DE 19643037, etc.

In WO97/17970, there is a report on a pyridothiazine derivative having an inhibitory action on the neurocytotoxicity of kainate, which is based on non-competitive antagonism against AMPA receptor response. In WO00/27851, there is a report on a condensed pyridazinone derivative having an enhancing action on memorization, which is based on enhancing action of NMDA and inhibitory action on AMPA. In WO00/47567, there is a report on a compound as a heterodiazinone derivative having antagonism against non-NMDA receptor, which is represented by the formula: 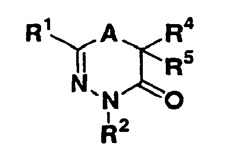 wherein A represents O, S or NR³ (wherein R³ is a hydrogen atom or a lower alkyl group) ; R¹ and R² are independent of each other and each represents an optionally substituted (hetero) aryl group; and R⁴ and R⁵ independently represents a hydrogen, hydroxyl group, halogen, cyano, nitro, lower alkyl, (hetero)aryl group, etc.

In WO99/10331 WO99/10332 and WO00/24719, there is a report on a pyridazinone compound as cyclooxygenase-2 inhibitor etc. , which is represented by the following formula, or a salt, ester or prodrug thereof: 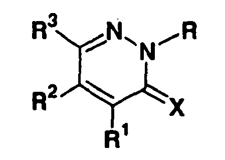 wherein X represents O, S, etc.; R represents an aryl group etc.; and at least one of R¹, R² and R³ represents a phenyl group substituted with a specific group etc., while the two other groups represent an aryl group etc. In WO99/25697, 99/44995 and WO00/50408, there is a report on a pyridazinone derivative as an inhibitor of production of interleukin-1β. In WO00/09488, there is a report on a pyridazinone derivative having an inhibitory action on cell adhesion. In WO97/07104, EP0860435, EP0963978, WO00/34249, US6107250, JP-A 5-25164, DE4423934, etc., there is also a report on a pyridazinone derivative having an antimicrobial activity and a heribicidal action for use in agrochemicals, but the relationship thereof with AMPA receptor/kainate receptor is not described therein and not known. There are some reports on use of triazinone compounds as agrochemicals, but the relationship thereof with AMPA receptor/kainate receptor is not described and not known. The relationship of a pyridazin-3-one derivative having cyclic substituent groups at the 2-, 4- and 6-positions, and a 1,2,4-triazin-3-one derivative, with AMPA receptor/kainate receptor is not known either.

It is desired to provide a compound which exhibits an excellent inhibitory action on AMPA receptor and/or kainate receptor, is highly useful as a pharmaceutical preparation effectively acting in clinical. Thus, an object of the present invention is to investigate and find a compound which inhibits AMPA receptor and/or kainate receptor which suppresses the neurotoxicity of excitatory neurotransmitters and achieves an excellent neuroprotective action as pharmaceutical agents being useful as an agent for treating, preventing or improving various nerve diseases.

### Disclosure of the Invention

Under such circumstances, the present inventors have carried out an intensive study. As a result, they have succeeded for the first time in synthesizing a compound (Compound (VIII)) represented by the following formula, a salt thereof or a hydrate of them, and have found an excellent method for producing the compound, a salt thereof or a hydrate of them. Further, surprisingly, they have found that the above compound (VIII), a salt thereof or a hydrate of them shows an excellent AMPA receptor and/or kainate receptor antagonism, whereupon the present invention has been accomplished.

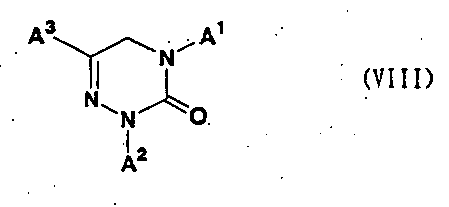

In the formula, A¹, A²and A³ are independent of each other and each represents a C₆₋₁₄ aromatic hydrocarbon cyclic group or a 5- to 14-membered aromatic heterocyclic group, each of which may be substituted; provided that compounds in the following cases (a) to (c) :
(a) the case where each of A¹, A² and A³ is a phenyl group, (b) the case where A¹ is an o,p-dimethylphenyl group; A¹ is an o-methylphenyl group; and A³ is a phenyl group, and (c) the case where A¹ is an o-methylphenyl group; A² is a p-methoxyphenyl group; and A³ is a phenyl group are excluded.

That is, the present invention relates to (1) the compound represented by the above formula (VIII), a salt thereof or a hydrate of them; (2) the compound according to the above (1), a salt thereof or a hydrate of them, wherein A¹, A² and A³ are independent of each other and each represents a phenyl group, pyrrolyl group, pyridyl group, pyridazinyl group, pyrimidinyl group, pyrazinyl group, thienyl group, thiazolyl group, furyl group, naphthyl group, quinolyl group, isoquinolyl group, indolyl group, benzimidazolyl group, benzothiazolyl group, benzoxazolyl group, imidazopyridyl group, or carbazolyl group, each of which may be substituted; (3) the compound according to the above (1), a salt thereof or a hydrate of them, wherein A¹, A² and A³ are independent of each other and each represents a group represented by the formula: 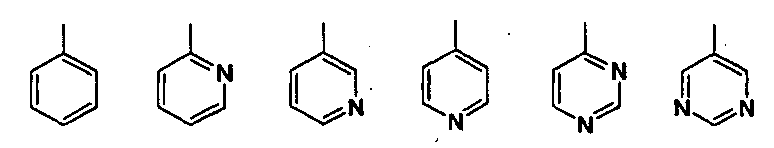 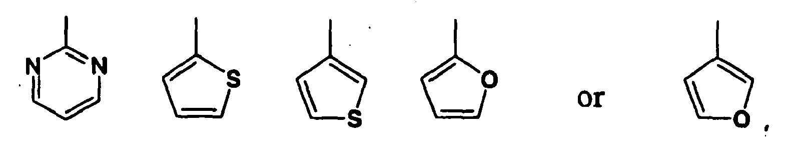 each of which may be substituted. A¹, A² and A³ may be substituted with one or more groups selected from the substituents group b below:
<substituent group b> the group consisting of (a) a hydroxyl group, (b) a halogen atom, (c) a nitrile group, (d) a nitro group, (e) a C₁₋₆ alkyl group, C₃₋₆ alkenyl group or C₂₋₆ alkynyl group, each of which may be substituted with at least one group selected from the group consisting of a hydroxyl group, nitrile group, halogen atom, C₁₋₆ alkylamino group, di(C₁₋₆ alkyl) amino group, C₂₋₆ alkenylamino group, di(C₂₋₆ alkenyl) amino group, C₂₋₆ alkynylamino group, di(C₂₋₆ alkynyl)amino group, N-C₁₋₆ alkyl-N-C₂₋₆ alkenylamino group, N-C₁₋₆ alkyl-N-C₂₋₆ alkynylamino group, N-C₂₋₆ alkenyl-N-C₂₋₆ alkynylamino group, aralkyloxy group, TBDMS oxy group, C₁₋₆ alkylsulfonylamino group, C₁₋₆ alkylcarbonyloxy group, C₁₋₆ alkenylcarbonyloxy group, C₂₋₆ alkynylcarbonyloxy group, N-C₁₋₆ alkylcarbamoyl group, N-C₂₋₆ alkenylcarbamoyl group and N-C₁₋₆ alkynylcarbamoyl group, (f) a C₁₋₆ alkoxy group, C₂₋₆ alkenyloxy group or C₂₋₆ alkynyloxy group, each of which may be substituted with at least one group selected from the group consisting of a C₁₋₆ alkylamino group, aralkyloxy group and hydroxyl group, (g) a C₁₋₆ alkylthio group, C₁₋₆ alkenylthio group or C₂₋₆ alkynylthio group, each of which may be substituted with at least one group selected from the group consisting of a hydroxyl group, nitrile group, halogen atom, C₁₋₆ alkylamino group, aralkyloxy group, TBDMS oxy group, C₁₋₆ alkylsulfonylamino group, C₁₋₆ alkylcarbonyloxy group and C₁₋₆ alkylcarbamoyl group, (h) a carbonyl group substituted with a group selected from the group consisting of a C₁₋₆ alkoxy group, amino group, C₁₋₆ alkylamino group, di (C₁₋₆ alkyl) amino group, C₁₋₆ alkenylamino group, di (C₂₋₆ alkenyl) amino group, C₂₋₆ alkynylamino group, di (C₂₋₆ alkynyl) amino group, N-C₁₋₆ alkyl-N-C₁₋₆ alkenylamino group, N-C₁₋₆ alkyl-N-C₁₋₆ alkynylamino group and N-C₂₋₆ alkenyl-N-C₂₋₆ alkynylamino group, (i) an amino group which may be substituted with one or two groups selected from the group consisting of a C₁₋₆ alkyl group, C₂₋₆ alkenyl group, C₂₋₆ alkynyl group, C₁₋₆ alkylsulfonyl group, C₂₋₆ alkenylsulfonyl group, C₂₋₆ alkynylsulfonyl group, C₁₋₆ alkylcarbonyl group, C₂₋₆ alkenylcarbonyl group and C₂₋₆ alkynylcarbonyl group, (j) a C₁₋₆ alkylsulfonyl group, (k) a C₁₋₆ alkenylsulfonyl group, (1) a C₂₋₆ alkynylsulfonyl group, (m) a C₁₋₆ alkylsulfinyl group, (n) a C₁₋₆ alkenylsulfinyl group, (o) a C₂₋₆ alkynylsulfinyl group, (p) a formyl group, (q) a C₃₋₈ cycloalkyl group or C₃₋₈ cycloalkenyl group, each of which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, (r) a 5 to 14 membered non-aromatic heterocyclic group which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, (s) a C₆₋₁₄ aromatic hydrocarbon cyclic group which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, and (t) a 5- to 14-membered aromatic heterocyclic group which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, and (u) thiol group.

The invention further pertains to (4) the compound according to the above (1) , a salt thereof or a hydrate of them, wherein each of A¹, A² and A³ may be substituted with at least one group selected independently from the group consisting of a halogen atom, nitrile group, hydroxyl group, amino group, formyl group and nitro group; (5) the compound according to the above-mentioned (1), a salt thereof or a hydrate of them, wherein the binding positions of substituent groups on A¹, A² and/or A³ are α-positions of the carbon atoms on A¹, A² and/or A³, each of which are bound directly to the triazinone ring; (6) the compound according to the above (1), a salt thereof or a hydarate of them, which is any one of compounds selected from 2-(2-bromophenyl)-4-(2-methoxyphenyl)-6-(2-pyridyl)-4,5-dihydro-1,2,4-triazin-3(2H)-one, 2-(2-bromophenyl)-4-(2-hydroxyphenyl)-6-(2-pyridyl)-4,5-dihydro-1,2,4-triazin-3(2H)-one, 2-(2-cyanophenyl)-4-(2-methoxyphenyl)-6-(2-pyridyl)-4,5-dihydro-1,2,4-triazin-3(2H)-one, 2-(2-bromophenyl)-4-phenyl-6-(2-pyridyl)-4,5-dihydro-1,2,4-triazin-3(2H)-one, 2-(2-bromophenyl)-6-(2-methoxyphenyl)-4-phenyl-4,5-dihydro-1,2,4-triazin-3(2*H*)-one, 2-(2-bromophenyl)-6-(2-hydroxyphenyl)-4-phenyl-4,5-dihydro-1,2,4-triazin-3(2H)-one, 2-(2-bromophenyl)-6-(2-dimethylaminoethoxyphenyl)-4-phenyl-4,5-dihydro-1,2,4-triazin-3(2H)-one, 2-(2-bromophenyl)-6-(2-methoxyphenyl)-4-(2-pyridyl)-4,5-dihydro-1,2,4-triazin-3(2H)-one, 2-(2-cyanophenyl)-6-(2-hydroxyphenyl)-4-phenyl-4,5-dihydro-1,2,4-triazin-3(2H)-one, 2-(2-bromophenyl)-4-(2,5-dihydroxyphenyl)-6-(2-hydroxyphenyl)-4,5-dihydro-1,2,4-triazin-3(2H)-one, 4-(2,5-dihydroxyphenyl)-6-(2-hydroxyphenyl)-2-phenyl-4,5-dihydro-1,2,4-triazin-3(2*H*)-one, 2-(2-cyanophenyl)-4-(2-hydroxyphenyl)-6-(2-pyridyl)-4,5-dihydro-1,2,4.-triazin-3(2H) -one, 2-(2-cyanophenyl)-6-(2-methoxyphenyl)-4-phenyl-4,5-dihydro-1,2,4-triazin-3(2H)-one, 4-(2-cyanophenyl)-6-(2-methoxyphenyl)-2-phenyl-4,5-dihydro-1,2,4-triazin-3(2H)-one, 2-(2-cyanophenyl)-6-(2-methoxyphenyl)-4-(2-pyridyl)-4,5-dihydro-1,2,4-triazin-3(2*H*)-one, 2-(2-cyanophenyl)-4-phenyl-6-(2-pyridyl)-4,5-dihydro-1,2,4-triazin-3(2*H*)-one, 2-(2-cyanophenyl)-6-(2-pyridyl)-4-(thiophen-3-yl)-4,5-dihydro-1,2,4-triazin-3 (2*H*)-one, 2-(2-cyanophenyl)-6-(2-pyridyl)-4-(2-pyridyl)-4,5-dihydro-1,2,4-triazin-3(2*H*)-one, 2-(2-cyanophenyl)-6-(2-pyridyl)-4-(3-pyridyl)-4,5-dihydro-1,2,4-triazin-3(2*H*)-one, 4₋(2-cyanophenyl)-2-phenyl-6-(2-pyridyl)-4,5-dihydro-1,2,4-triazin-3(2*H*)-one, 2-phenyl-6-(2-pyridyl)-4-(thiophen-3-yl)-4,5-dihydro-1,2,4-triazin-3(2*H*)-one, 2-(2-bromophenyl)-6-(2-pyridyl)-4-(thiophen-3-yl)-4,5-dihydro-1,2,4-triazin-3(2*H*)-one, 4-(2,4-dimethoxyphenyl)-2-phenyl-6-(2-pyridyl)-4,5-dihydro-1,2,4-triazin-3(2*H*)-one, 2-(2-bromophenyl)-6-(2-methoxyphenyl)-4-(thiophen-3-yl)-4,5-dihydro-1,2,4-triazin-3(2*H*)-one, 2-phenyl-6-(2-pyridyl)-4-(3-pyridyl)-4,5-dihydro-1,2,4-triazin-3(2*H*)-one, 2-(2-bromophenyl)-6-(2-pyridyl)-4-(3-pyridyl)-4,5-dihydro-1,2,4-triazin-3(2*H*)-one, 2-(2-bromophenyl)-4-(2-cyanophenyl)-6-(2-pyridyl)-4,5-dihydro-1,2,4-triazin-3(2*H*)-one, 2-(2-bromophenyl)-4,6-diphenyl-4,5-dihydro-1,2,4-triazin-3(2*H*)-one, 4-(2-bromophenyl)-2,6-diphenyl-4,5-dihydro-1,2,4-triazin-3(2*H*)-one, 2-(2-bromophenyl)-4-(2-bromophenyl)-6-phenyl-4,5-dihydro-1,2,4-triazin-3(2*H*)-one, 4-(2-bromophenyl)-6-(2-methoxyphenyl)-2-phenyl-4,5-dihydro-1,2,4-triazin-3(2*H*)-one, 2-(2-bromophenyl)-4-(2,5-dimethoxyphenyl)-6-(2-methoxyphenyl)-4,5-dihydro-1,2,4-triazin-3(2*H*)-one, 4-(2,5-dimethoxyphenyl)-6-(2-methoxyphenyl)-2-phenyl-4,5-dihydro-1,2,4-triazin-3(2*H*)-one, 2-(2-bromophenyl)-6-(2-pyridyl)-4-(2-pyridyl)-4,5-dihydro-1,2,4-triazin-3(2*H*)-one, 2-phenyl-4-phenyl-6-(2-pyrimidinyl)-4,5-dihydro-1,2,4-triazin-3(2*H*)-one, 2-(2-bromophenyl)-4-(4-biphenyl)-6-(2-pyridyl)-4,5-dihydro-1,2,4-triazin-3(2*H*)-one, 2-(2-bromophenyl)-4-(3-nitrophenyl)-6-(2-pyridyl)-4,5-dihydro-1,2,4-triazin-3(2*H*)-one, 2-(2-bromophenyl)-4-(4-fluorophenyl)-6-(2-pyridyl)-4,5-dihydro-1,2,4-triazin-3(2*H*)-one, 2-(2-bromophenyl)-4-(3-formylphenyl)-6-(2-pyridyl)-4,5-dihydro-1,2,4-triazin-3 (2*H*)-one, 2-(2-bromophenyl)-4-(3-tolyl)-6-(2-pyridyl)-4,5-dihydro-1,2,4-triazin-3(2*H*)-one,2-(2-bromophenyl)-4-(4-thiomethoxyphenyl)-6-(2-pyridyl)-4,5-dihydro-1,2,4-triazin-3(2*H*)-one, 2-(2-bromophenyl)-4-(2-chloropyridin-5-yl)-6-(2-pyridyl)-4,5-dihydro-1,2,4-triazin-3(2*H*) -one, 2-(2-cyanophenyl)-4-(3-nitrophenyl)-6-(2-pyridyl)-9,5-dihydro-1,2,4-triazin-3(2*H*)-one, 2-(2-cyanophenyl)-4-(3-aminophenyl)-6-(2-pyridyl)-4,5-dihydro-1,2,4-triazin-3(2*H*)-one, and 2-(2-chlorophenyl)-4-phenyl-6-(2-pyrimidinyl)-4, 5-dihydro-1,2,4-triazin-3(2*H*)-one; (7) a pharmaceutical composition comprising a compound represented by formula (VIII) as an active ingredient; (8) the use of the compounds according to the above (1), a salt thereof or a hydrate of them for producing an agent for treating or preventing a disease selected from multiple sclerosis and epilepsy.

The compound according to the present invention may be a pharmaceutically acceptable salt thereof or a pharmacologically acceptable hydrate thereof.

The pharmaceutical composition of the present invention can contain a pharmacologically acceptable carrier.

The present invention provides a process for treating or preventing a disease in which any AMPA receptor or a kainate receptor is participated, by-administering a pharmacologically effective dose of the compound represented by the above formula (I), a salt thereof or a hydrate of them to a patient.

The present invention provides use of the compound represented by the above formula (VIII), a salt thereof or a hydrate of them for producing an agent for treating or preventing a disease in which an AMPA receptor or a kainate receptor is participated, namely multiple sclerosis and epilepsy.

Hereinafter, the meanings of symbols, terms, etc. used in this specification are described, and the present invention is described in detail.

As "acute neurodegenerative disease" in the present invention, for example, cerebrovascular disorders at acute stage (subarachnoid hemorrhage, cerebral infarction and the like), head injury, spinal cord injury, and neuropathies due to hypoxia or hypoglycemia; and the like are mentioned. As "chronic neurodegenerative disease" , for example, Alzheimer's disease, Parkinson's disease, Huntington's chorea, amyotrophic lateral sclerosis, spinocerebellar degeneration and the like are mentioned. As "infectious encephalomyelitis" , for example, HIV encephalomyelitis is mentioned, and as "demyelinating disease", for example, encephalitis, acute disseminated encephalomyelitis, multiple sclerosis, acute dimyelinating polyneuropathy, Guillain-Barre syndrome, chronic inflammatory demyelinating polyneuropathy, Marchifava-Bignami disease, central pontine myelinolysis, neuromyelitis optica, Devic disease, Balo disease, HIV myelopathy, HTLV myelopathy, progressive multifocal leukoencephalopathy, secondary demyelinating disease and the like are mentioned. As "the secondary demyelinating disease" mentioned above, for example, CNS lupus erythematodes, polyarteritis nodosa, Sjoegren's syndrome, sarcoidosis, isolated cerebral vasculitis and the like are mentioned.

Incidentally, in the specification of this application, although structural formula of a compound may express a certain isomer for the sake of convenience, the present invention covers all isomers such as geometrical isomers resulted from the structure of the compound, optical isomers due to asymmetric carbon, rotamers, stereo isomers and tautomers as well as a mixture of isomers and the present invention is not limited to the description of the formula given for the sake of convenience but may be another isomer or may be a mixture. Accordingly, although it is possible that an asymmetric carbon atom is present in a molecule and accordingly that optically active substance and racemic substance may be present, the present invention is not limited thereto but covers any of them. Further, crystal polymorphism may be present but, again, there is no limitation but any of single crystal form or a mixture will do. The compound (VIII) or its salt related to the present invention may be an anhydride or a hydrate, and either of them are included in the scope of claim for patent in the present invention. The metabolite which is generated by decomposing the compound (VIII) related to the present invention *in vivo,* and the prodrug of the compound (VIII) or its salt related to the present invention produce are also included in the scope of claim for patent in the present invention.

The "halogen atom" used in this specification includes a fluorine atom, chlorine atom, bromine atom and iodine atom, and the atom is preferably a fluorine atom, chlorine atom or bromine atom.

The "C₁₋₆ alkyl group" used in this specification refers to an alkyl group containing 1 to 6 carbon atoms, and preferable examples thereof include linear or branched alkyl groups such as a methyl group, ethyl group, n-propyl group, iso-propyl group, n-butyl group, iso-butyl group, sec-butyl group, tert-butyl group, n-pentyl group, 1,1-dimethylpropyl group, 1,2-dimethylpropyl group, 2,2-dimethylpropyl group, 1-ethylpropyl group, 2-ethylpropyl group, n-hexyl group, 1-methyl-2-ethylpropyl group, 1-ethyl-2-methylpropyl group, 1,1,2-trimethylpropyl group, 1-propylpropyl group, 1-methylbutyl group, 2-methylbutyl group, 1,1,-dimethylbutyl group, 1,2-dimethylbutyl group, 2,2-dimethylbutyl group, 1,3-dimethylbutyl group, 2,3-dimethylbutyl group, 2-ethylbutyl group, 2-methylpentyl group or 3-methylpentyl group.

The "C₂₋₆ alkenyl group" used in this specification refers to an alkenyl group containing 2 to 6 carbon atoms, and preferable examples thereof include a vinyl group, allyl group, 1-propenyl group, 2-propenyl group, isopropenyl group, 2-methyl-1-propenyl group, 3-methyl-1-propenyl group, 2-methyl-2-propenyl group, 3-methyl-2-propenyl group, 1-butenyl group, 2-butenyl group, 3-butenyl group, 1-pentenyl group, 1-hexenyl group, 1,3-hexanedienyl group, 1,6-hexanedienyl group, etc.

The "C₂₋₆ alkynyl group" used in this specification refers to an alkynyl group containing 2 to 6 carbon atoms, and preferable examples thereof include an ethynyl group, 1-propynyl group, 2-propynyl group, 1-butynyl group, 2-butynyl group, 3-butynyl group, 3-methyl-1-propynyl group, 1-ethynyl-2-propynyl group, 2-methyl-3-propynyl group, 1-pentynyl group, 1-hexynyl group, 1,3-hexanediynyl group, 1,6-hexanediynyl group, etc.

The "C₁₋₆ alkoxy group" used in this specification refers to an alkoxy group containing 1 to 6 carbon groups, and preferable examples thereof include a methoxy group, ethoxy group, n-propoxy group, iso-propoxy group, sec-propoxy group, n-butoxy group, iso-butoxy group, sec-butoxy group, tert-butoxy group, n-pentyloxy group, iso-pentyloxy group, sec-pentyloxy group, n-hexoxy group, iso-hexoxy group, 1,1-dimethylpropoxy group, 1,2-dimethylpropoxy group, 2,2-dimethylpropoxy group, 2-ethylpropoxy group, 1-methyl-2-ethylpropoxy group, 1-ethyl-2-methylpropoxy group, 1,1,2-trimethylpropoxy group, 1,1-dimethylbutoxy group, 1,2-dimethylbutoxy group, 2,2-dimethylbutoxy group, 2,3-dimethylbutoxy group, 1,3-dimethylbutoxy group, 2-ethylbutoxy group, 1,3-dimethylbutoxy group, 2-methylpentoxy group, 3-methylpentoxy group, hexyloxygroup, etc.

The "C₂₋₆, alkenyloxy group" used in this specification refers to an alkenyloxy group containing 2 to 6 carbon atoms, and preferable examples thereof include a vinyloxy group, allyloxy group, 1-propenyloxy group, 2-propenyloxy group, isopropenyloxy group, 2-methyl-1-propenyloxy group, 3-methyl-1-propenyloxy group, 2-methyl-2-propenyloxy group, 3-methyl-2-propenyloxy group, 1-butenyloxy group, 2-butenyloxy group, 3-butenyloxy group, 1-pentenyloxy group, 1-hexenyloxy group, 1,3-hexanedienyloxy group, 1,6-hexanedienyloxy group, etc.

The "C₃₋₈ cycloalkyl group" used in this specification refers to a cycloalkyl group containing 3 to 8 carbon atoms, and preferable examples thereof include a cyclopropyl group, cyclobutyl group, cyclopentyl group, cyclohexyl group, cycloheptyl group, cyclooctyl group, etc. The "C₃₋₈ cycloalkane" refers to a cyclic structure corresponding to the above-described "C₃₋₈ cycloalkyl group", and preferable examples thereof also correspond to examples of the above-described "C₃₋₈ cycloalkyl group".

The "C₃₋₈ cycloalkenyl group" used in this specification refers to a C₃₋₈ cycloalkenyl group composed of 3 to 8 carbon atoms, and preferable examples thereof include cyclopropen-1-yl, cyclopropen-3-yl, cyclobuten-1-yl, cyclobuten-3-yl, 1,3-cyclobutadien-1-yl, cyclopenten-1-yl, cyclopenten-3-yl, cyclopenten-4-yl, 1,3-cyclopentadien-1-yl, 1,3-cyclopentadien-2-yl, 1,3-cyclopentadien-5-yl, cyclohexen-1-yl, cyclohexen-3-yl, cyclohexen-4-yl, 1,3-cyclohexadien-1-yl, 1,3-cyclohexadien-2-yl, 1,3-cyclohexadien-5-yl, 1,4-cyclohexadien-3-yl, 1,4-cyclohexadien-1-yl, cyclohepten-1-yl, cyclohepten-3-yl, cyclohepten-4-yl, cyclohepten-5-yl, 1,3-cyclohepten-2-yl, 1,3-cyclohepten-1-yl, 1,3-cycloheptadien-5-yl, 1,3-cycloheptadien-6-yl, 1,4-cycloheptadien-3-yl, 1,4-cycloheptadien-2-yl, 1,4-cycloheptadien-1-yl, 1,4-cycloheptadien-6-yl, 1,3,5-cycloheptatrien-3-yl, 1,3,5-cycloheptatrien-2-yl, 1,3,5-cycloheptatrien-1-yl, 1,3,5-cycloheptatrien-7-yl, cycloocten-1-yl, cycloocten-3-yl, cycloocten-4-yl, cycloocten-5-yl, 1,3-cyclooctadien-2-yl, 1,3-cyclooctadien-1-yl, 1,3-cyclooctadien-5-yl, 1,3-cyclooctadien-6-yl, 1,4-cyclooctadien-3-yl, 1,4-cyclooctadien-2-yl, 1,4-cyclooctadien-1-yl, 1,4-cyclooctadien-6-yl, 1,4-cyclooctadien-7-yl, 1,5-cyclooctadien-3-yl, 1,5-cyclooctadien-2-yl, 1,3,5-cyclooctatrien-3-yl, 1,3,5-cyclooctatrien-2-yl, 1,3,5-cyclooctatrien-1-yl, 1,3,5-cyclooctatrien-7-yl, 1,3,6-cyclooctatrien-2-yl, 1,3,6-cyclooctatrien-1-yl, 1,3,6-cyclooctatrien-5-yl, 1,3,6-cyclooctatrien-6-yl group, and the like. The "C₃₋₈ cycloalkene" refers to a cyclic structure corresponding to the above-mentioned "C₃₋₈ cycloalkenyl group", and preferable examples also correspond to examples of the above-described "C₃₋₈ cycloalkenyl group".

The "5 to 14 membered non-aromatic heterocyclic group" used in the present invention means a mono-cyclic type, di-cyclic type, or tri-cyclic type 5 to 14 membered non-aromatic heterocyclic group which contains one or more of hetero atoms selected from a group which consists of nitrogen atom, sulfur atom and oxygen atom. Preferable examples in the group include, for example, pyrrolidinyl group, pyrrolinyl group, piperidyl group, piperazinyl group, imidazolidinyl group, pyrazolidinyl group, morpholinyl group, tetrahydrofuryl group, tetrahydropyranyl group, dihydrofuryl group, dihydropyranyl group, imidazolinyl group, oxazolinyl group, and the like. Further, a group derived from a pyridone ring and a non-aromatic condensed ring (for example, a group derived from a phthalimide ring, a succinimide ring, and the like) are also included in the non-aromatic heterocyclic group.

The "C₆₋₁₄ aromatic hydrocarbocyclic group" and the "aryl group" used in the present invention mean an aromatic hydrocarbocyclic group having which is composed of 6 to 14 carbon atoms, and a mono-cyclic group, and a condensed group of a di-cyclic group, a tri-cyclic group and the like are also included. Specific examples in the group include phenyl group, indenyl group, 1-naphthyl group, 2-naphthyl group, azulenyl group, heptalenyl group, biphenyl group, indathenyl group, acenaphthyl group, fluorenyl group, phenalenyl group, phenanthrenyl group, anthracenyl group, cyclopentacyclooctenyl group, benzocyclooctenyl group etc. Further, the "C₆₋₁₄ aromatic hydrocarbon ring" means a cyclic structure corresponding to the above-mentioned "C₆₋₁₄ aromatic hydrocarbon cyclic group", and preferable examples also correspond to examples of the above-described "C₆₋₁₄ aromatic hydrocarbon cyclic group".

The "5 to 14 membered aromatic heterocyclic group" and the "heteroaryl group" used in the present invention mean a mono-cyclic type, di-cyclic type, or tri-cyclic type 5 to 14 membered aromatic heterocyclic group which contains one or more of hetero atoms selected from a group which consists of nitrogen atom, sulfur atom and oxygen atom. Preferable examples in the group include aromatic heterocyclic groups containing nitrogen such as pyrrolyl group, pyridyl group, pyridazinyl group, pyrimidinyl group, pyrazinyl group, triazolyl group, tetrazolyl group, benzotriazolyl group, pyrazolyl group, imidazolyl group, benzimidazolyl group, indolyl group, isoindolyl group, indolizinyl group, prenyl group, indazolyl group, quinolyl group, iso-quinolyl group, quinoliziyl group, phthalazyl group, naphthylidinyl group, quinoxalyl group, quinazolinyl group, cynnolinyl group, pteridinyl group, imidazotriazinyl group, pyrazinopyridazinyl group, acridinyl group, phenanthridinyl group, carbazolyl group, carbazolinyl group, perimidinyl group, phenanthrolinyl group, phenacinyl group, imidazopyridinyl group, imidazopyrimidinyl group, pyrazolopyridinyl group etc; aromatic heterocyclic groups containing sulfur such as thienyl group or benzothienyl group; aromatic heterocyclic groups containing oxygen such as furyl group, pyranyl group, cyclopentapyranyl group, benzofuryl group or iso-benzofuryl group; and aromatic heterocyclic groups containing 2 or more of different hetero atoms such as thiazolyl group, iso-thiazolyl group, benzothiazolyl group, benzothiadiazolyl group, phenothiazinyl group, isoxazolyl group, furazanyl group, phenoxazinyl group, oxazolyl group, isoxazoyl group, benzoxazolyl group, oxadiazolyl group, pyrazoloxadiazolyl group, imidazothiazolyl group, thienofuranyl group, furopyrrolyl group or pyridoxadinyl group, etc. The "5- to 14-membered aromatic heterocyclic ring" means a cyclic structure corresponding to the above-mentioned "5- to 14-membered aromatic heterocyclic group", and preferable examples also correspond to examples of the above-described "5- to 14-membered aromatic heterocyclic group".

The "C₅₋₈ hydrocarbon ring" in this specification refers to a ring selected from C₅₋₈ cycloalkane, C₅₋₈ cycloalkene and C₆₋₈ aromatic hydrocarbon ring. The preferable ring is not particularly limited, and includes the preferable examples of the C₅₋₈ cycloalkane, C₅₋₈ cycloalkene and C₆₋₈ aromatic hydrocarbon ring as defined above.

The "5- to 8-membered heterocyclic ring" in this specification refers to a ring selected from a 5- to 8-membered non-aromatic heterocyclic ring and aromatic heterocyclic ring, and the preferable ring is not particularly limited, and includes the preferable examples of the 5- to 8-membered non-aromatic heterocyclic ring and aromatic heterocyclic ring defined above.

The groups indicated by A¹, A² and A³ in the compound (VIII) in the present invention indicate independently an optionally substituted C₆₋₁₄ aromatic hydrocarbocyclic group or an optionally substituted 5 to 14 membered aromatic heterocyclic group, and each of the groups has the same meanings as the above definitions, respectively. The preferable group in A¹, A² and A³ is not specifically limited, but the more preferable group includes phenyl group, pyrrolyl group, pyridyl group, pyridazinyl group, pyrimidinyl group, pyrazinyl group, thienyl group, thiazolyl group, furyl group, naphthyl group, quinolyl group, iso-quinolyl group, indolyl group, benzimidazolyl group, benzothiazolyl group, benzoxazolyl group, imidazopyridyl group, and carbazolyl group, which may be substituted. The more preferable group includes a group represented by the formula: 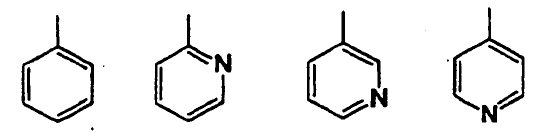 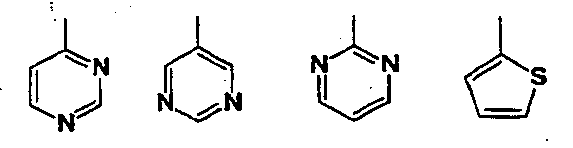 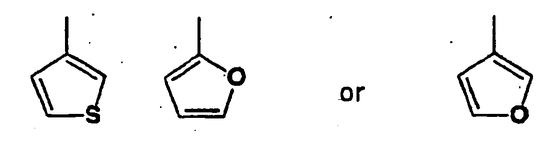 which may optionally have substituents respectively, etc., and the most preferable group includes a group represented by the formula: 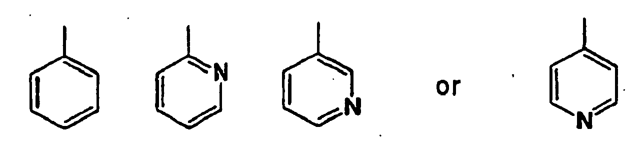 which may optionally have substituents respectively, etc.

Examples of the preferable group in the "substituent" of the groups indicated by A¹, A² and A³ in the compound (VIII) include a group such as hydroxy group, a halogen atom, nitrile group, nitro group, a C₁₋₆ alkyl group, C₂₋₆ alkenyl group, C₂₋₆ alkynyl group, C₁₋₆ alkoxy group, C₂₋₆ alkenyloxy group, C₂₋₆ alkynyloxy group, C₁₋₆ alkylthio group, C₂₋₆ alkenylthio group, C₂₋₆ alkynylthio group, amino group, a substituted carbonyl group, C₁₋₆ alkylsulfonyl group, C₂₋₆ alkenylsulfonyl group, C₂₋₆ alkynylsulfonyl group, C₁₋₆ alkylsulfinyl group, C₂₋₆ alkenylsulfinyl group, C₂₋₆ alkynylsulfinyl group, formyl group, aralkyl group, heteroarylalkyl group, aralkyloxy group, heteroarylalkyloxy group, C₃₋₈ cycloalkyl group, C₃₋₈ cycloalkenyl group, 5 to 14 membered non-aromatic heterocyclic group, C₆₋₁₄ aromatic hydrocarbon group, 5 to 14 membered aromatic heterocyclic group etc., which may be substituted, respectively.

Examples of the preferable group in the above-mentioned "substituents" of the groups indicated by A¹, A² and A³ include fluorine atom, chlorine atom, bromine atom, iodine atom etc., and the more preferable example includes fluorine atom, chlorine atom and bromine atom. Examples of the preferable group in the "C₁₋₆alkyl group which may optionally have substituents" include methyl group, ethyl group, n-propyl group, iso-propyl group, n-butyl group, iso-butyl group, tert-butyl group, n-pentyl group, iso-pentyl group, neopentyl group, n-hexyl group, 1-methylpropyl group, 1,2-dimethylpropyl group, 2-ethylpropyl group, 1-methyl-2-ethylpropyl group, 1-ethyl-2-methylpropyl group, 1,1,2-trimethylpropyl group, 1-methylbutyl group, 2-methylbutyl group, 1,1-dimethylbutyl group, 2,2-dimethylbutyl group, 2-ethylbutyl group, 1,3-dimethylbutyl group, 2-methylpentyl group, 3-methylpentyl group etc, each of which may be substituted. Examples of the preferable group in the "C₂₋₆ alkenyl group which may optionally have substituents" include a vinyl group, allyl group, 1-propenyl group, iso-propenyl group, 1-buten-1-yl group, 1-buten-2-yl group, 1-buten-3-yl group, 2-buten-l-yl group, 2-buten-2-yl group etc., each of which may be substituted. Examples of the preferable group in the "C₂₋₆ alkynyl group which may optionally have substituents respectively" include an ethynyl group, 1-propynyl group, 2-propynyl group, butynyl group, pentynyl group, hexynyl group etc., each of which may be substituted. Further, preferable examples of the "substituents" in the "which may optionally have substituents" include 1 or more groups selected from hydroxy group, nitrile group, a halogen atom, an N-C₁₋₆ alkylamino group, an N,N-di-C₁₋₆ alkylamino group, an N-C₂₋₆ alkenylamino group, an N,N-di-C₂₋₆ alkenylamino group, an N-C₂₋₆ alkynylamino group, an N,N-di-C₂₋₆ alkynylamino group, a C₆₋₁₄ aromatic hydrocarbocyclic group (for example, phenyl group etc.), a 5 to 14 membered aromatic heterocyclic group (for example, thienyl group, furyl group, pyridyl group, pyridazinyl group, pyrimidinyl group, pyrazinyl group etc.), an aralkyloxy group, a heteroaryloxy group, a TBDMS-oxy group, a C₁₋₆ alkylsulfonylamino group, a C₂₋₆ alkenylsulfonylamino group, a C₂₋₆ alkynylsulfonylamino group, a C₁₋₆ alkylcarbonyloxy group, a C₂₋₆ alkenylcarbonyloxy group, a C₂-₆ alkynylcarbonyloxy group, a C₁₋₆ alkylcarbamoyl group, a C₂₋₆ alkenylcarbamoyl group, a C₂₋₆ alkynylcarbamoyl group, and the like.

Preferable examples in the "C₁₋₆ alkoxy group which may optionally have substituents" include methoxy group, ethoxy group, n-propoxy group, iso-propoxy group, sec-propoxy group, n-butoxy group, iso-butoxy group, sec-butoxy group, tert-butoxy group, n-pentoxy group, iso-pentoxy group, sec-pentoxy group, tert-pentoxy group, n-hexoxy group, iso-hexoxy group, 1,2-dimethylpropoxy group, 2-ethylpropoxy group, 1-methyl-2-ethylpropoxy group, 1-ethyl-2-methylpropoxy group, 1,1,2-trimethylpropoxy group, 1,1-dimethylbutoxy group, 2,2-dimethylbutoxy group, 2-ethylbutoxy group, 1,3-dimethylbutoxy group, 2-methylpentoxy group, 3-methylpentoxy group, hexyloxy group etc. Preferable examples in the "C₂₋₆ alkenyloxy group which may optionally have substituents" include vinyloxy group, allyloxy group, 1-propenyloxy group, iso-propenyloxy group, 1-buten-1-yloxy group, 1-buten-2-yloxy group, 1-buten-3-yloxy group, 2-buten-1-yloxy group, 2-buten-2-yloxy group etc. Preferable examples in the "C₂₋₆ alkynyloxy group which may optionally have substituents" include ethynyloxy group, 1-propynyloxy group, 2-propynyloxy group, butynyloxy group, pentynyloxy group, hexynyloxy group etc. Further, preferable examples of the "substituents" in the "which may optionally have substituents" include 1 or more groups selected from an C₁₋₆ alkylamino group, an aralkyloxy group, hydroxy group, and the like.

Respectively preferable examples in the "C₁₋₆ alkylthio group which may optionally have substituents", "C₂₋₆ alkenylthio group which may optionally have substituents" and "C₂₋₆ alkynylthio group which may optionally have substituents" include a C₁₋₆ alkylthio group (for example, methylthio group, ethylthio group, n-propylthio group, iso-propylthio group, n-butylthio group, iso-butylthio group, tert-butylthio group, n-pentylthio group, iso-pentylthio group, neopentylthio group, n-hexylthio group etc.), a C₂₋₆ alkenylthio group (for example, vinylthio group, allylthio group, 1-propenylthio group, iso-propenylthio group, 1-buten-1-ylthio group, 1-buten-2-ylthio group, 1-buten-3-ylthio group, 2-buten-1-ylthio group, 2-buten-2-ylthio group etc.) and a C₂₋₆ alkynylthio group (for example, ethynylthio group, 1-propynylthio group, 2-propynylthio group, butynylthio group, pentynylthio group, hexynylthio group etc.), which may be optionally substituted by 1 or more groups selected from the group comsisting of hydroxy group, a halogen atom, nitrile group and nitro group.

Preferable examples in the "carbonyl group which was substituted" include a group which is represented by the formula -CO-W (examples of W in the formula include a C₁₋₆ alkyl group, a C₂₋₆ alkenyl group, a C₂₋₆ alkynyl group, a C₁₋₆ alkoxy group, amino group, an N-C₁₋₆ alkylamino group, an N, N-di (C₁₋₆ alkyl) amino group, an N-C₂₋₆ alkenylamino group, an N,N-di (C₂₋₆ alkenyl) amino group, an N-C₂₋₆ alkynylamino group, an N,N-di (C₂₋₆ alkynyl) amino group, an N-C₁₋₆ alkyl-N-C₂₋₆ alkenylamino group, an N-C₁₋₆ alkyl-N-C₂₋₆ alkynylamino group, an N-C₂₋₆ alkenyl-N-C₂₋₆ alkynylamino group etc.).

Examples of the "substituents" in the "amino group which may optionally have substituents" include 1 or 2 groups selected from a C₁₋₆ alkyl group, C₂₋₆ alkenyl group, C₂₋₆ alkynyl group, C₁₋₆ alkylsulfonyl group, C₂₋₆ alkenylsulfonyl group, C₂₋₆ alkynylsulfonyl group, C₁₋₆ alkylcarbonyl group, C₂₋₆ alkenylcarbonyl group, C₂₋₆ alkynylcarbonyl group etc., which may be substituted, respectively. Preferable examples in the "substituents" of the C₁₋₆ alkyl group, C₂₋₆ alkenyl group, C₂₋₆ alkynyl group, C₁₋₆ alkylsulfonyl group, C₂₋₆ alkenylsulfonyl group, C₂₋₆ alkynylsulfonyl group, C₁₋₆ alkylcarbonyl group, C₂₋₆ alkenylcarbonyl group and C₂₋₆ alkynylcarbonyl group include hydroxy group, a halogen atom, nitrile group, a C₁₋₆alkoxy group, a C₁₋₆alkylthio group etc. Specifically preferable examples in the "amino group which may optionally have substituents" in particular include methylamino group, ethylamino group, n-propylamino group, iso-propylamino group, n-butylamino group, iso-butylamino group, tert-butylamino group, n-pentylamino group, iso-pentylamino group, neopentylamino group, n-hexylamino group, 1-methylpropylamino group, 1,2-dimethylpropylamino group, 2-ethylpropylamino group, 1-methyl-2-ethylpropylamino group, 1-ethyl-2-methylpropylamino group, 1,1,2-trimethylpropylamino group, 1-methylbutylamino group, 2-methylbutylamino group, 1,1-dimethylbutylamino group, 2,2-dimethylbutylamino group, 2-ethylbutylamino group, 1,3-dimethylbutylamino group, 2-methylpentylamino group, 3-methylpentylamino group, N,N-dimethylamino group, N,N-diethylamino group, N,N-di(n-propyl)amino group, N,N-di(iso-propyl)amino group, N,N-di(n-butyl)amino group, N,N-di(iso-butyl)amino group, N,N-di(tert-butyl)amino group, N,N-di(n-pentyl)amino group, N,N-di(iso-pentyl)amino group, N,N-di(neopentyl)amino group, N,N-di(n-hexyl)amino group, N,N-di(1-methylpropyl)amino group, N,N-di(1,2-dimethylpropyl)amino group, N-methyl-N-ethylamino group, N-ethyl-N-(n-propyl)amino group, N-methyl-N-(iso-propyl)amino group, vinylamino group, allylamino group, (1-propenyl)amino group, iso-propenylamino group, (1-buten-1-yl)amino group, (1-buten-2-yl)amino group, (1-buten-3-yl)amino group, (2-buten-1-yl)amino group, (2-buten-2-yl)amino group, N,N-divinylamino group, N,N-diallylamino group, N,N-di(1-propenyl)amino group, N,N-di(iso-propenyl)amino group, N-vinyl-N-allylamino group, ethynylamino group, 1-propynylamino group, 2-propynylamino group, butynylamino group, pentynylamino group, hexynylamino group, N,N-diethynylamino group, N,N-di(1-propynyl)amino group, N,N-di(2-propynyl)amino group, N,N-dibutynylamino group, N,N-dipentynylamino group, N,N-dihexynylamino group, hydroxymethylamino group, 1-hydroxyethylamino group, 2-hydroxyethylamino group, 3-hydroxy-n-propylamino group, methylsulfonylamino group, ethylsulfonylamino group, n-propylsulfonylamino group, iso-propylsulfonylamino group, n-butylsulfonylamino group, tert-butylsulfonylamino group, vinylsulfonylamino group, allylsulfonylamino group, iso-propenylsulfonylamino group, iso-pentenylsulfonylamino group, ethynylsulfonylamino group, methylcarbonylamino group, ethylcarbonylamino group, n-propylcarbonylamino group, iso-propylcarbonylamino group, n-butylcarbonylamino group, tert-butylcarbonylamino group, vinylcarbonylamino group, allylcarbonylamino group, iso-propenylcarbonylamino group, iso-pentenylcarbonylamino group, ethynylcarbonylamino group etc.

Respectively preferable examples in the "C₁₋₆ alkylsulfonyl group which may optionally have substituents", "C₂₋₆ alkenylsulfonyl group which may optionally have substituents", "C₂₋₆ alkynylsulfonyl group which may optionally have substituents", "C₁₋₆ alkylsulfinyl group which may optionally have substituents", "C₂₋₆ alkenylsulfinyl group which may optionally have substituents" and "C₂₋₆ alkynylsulfinyl group which may optionally have substituents" include methylsulfonyl group, ethylsulfonyl group, n-propylsulfonyl group, iso-propylsulfonyl group, n-butylsulfonyl group, tert-butylsulfonyl group, vinylsulfonyl group, allylsulfonyl group, iso-propenylsulfonyl group, iso-pentenylsulfonyl group, ethynylsulfonyl group, methylsulfinyl group, ethylsulfinyl group, n-propylsulfinyl group, iso-propylsulfinyl group, n-butylsulfinyl group, tert-butylsulfinyl group, vinylsulfinyl group, allylsulfinyl group, iso-propenylsulfinyl group, iso-pentenylsulfinyl group, ethynylsulfinyl group etc.

Preferable examples in the "aralkyl group" and "heteroarylalkyl group" include benzyl group, phenethyl group, naphthylmethyl group, naphthylethyl group, pyridylmethyl group, pyridylethyl group, thienylmethyl group, thienylethyl group etc., preferable examples in the "aralkyloxy group" include benzyloxy group, phenethyloxy group, phenylpropoxy group, naphthylmethyloxy group, naphthylethyloxy group, naphthylpropyloxy group etc., and preferable examples in the "heteroarylalkyloxy group" include pyridylmethyloxy group, pyrazinylmethyloxy group, pyrimidinylmethyloxy group, pyrrolylmethyloxy group, imidazolylmethyloxy group, pyrazolylmethyloxy group, quinolylmethyloxy group, iso-quinolylmethyloxy group, furfuryloxy group, thienylmethyloxy group, thiazolylmethyloxy group etc.

Preferable examples in the "C₃₋₈, cycloalkyl group which may optionally have substituents" and "C₃₋₈ cycloalkenyl group which may optionally have a substituent" include a C₃₋₈ cycloalkyl group (for example, cyclopropyl group, cyclobutyl group, cyclopentyl group, cyclohexyl group, cycloheptyl group, and the like) and a C₃₋₈ cycloalkenyl group (for example, cyclopropenyl group, cyclobutenyl group, cyclopentenyl group, cyclohexenyl group, cycloheptenyl group, and the like) which may be optionally substituted respectively by 1 or more groups selected from hydroxy group, a halogen atom, nitrile group, a C₁₋₆ alkyl group (for example, methyl group, ethyl group, n-propyl group, iso-propyl group, n-butyl group, iso-butyl group, tert-butyl group, n-pentyl group, iso-pentyl group, neopentyl group, n-hexyl group etc.), a C₁₋₆ alkoxy group (for example, methoxy group, ethoxy group, n-propoxy group, iso-propoxy group, sec-propoxy group, n-butoxy group, iso-butoxy group, sec-butoxy group, tert-butoxy group, n-pentoxy group, iso-pentoxy group, sec-pentoxy group, tert-pentoxy group, n-hexoxy group etc.), a C₁₋₆ alkoxy C₁₋₆ alkyl group, an aralkyl group (for example, benzyl group, phenethyl group, naphthylmethyl group, naphthylethyl group etc.), and the like.

Preferable examples of the "5 to 14 membered non-aromatic heterocyclic group", "C₆₋₁₄ aromatic hydrocarbocyclic group" and "5 to 14 membered aromatic heterocyclic group" in "an optionally substituted 5 to 14 membered non-aromatic heterocyclic group", "an optionally substituted C₆₋₁₄ aromatic hydrocarbocyclic group" and "an optionally substituted 5 to 14 membered aromatic heterocyclic group" are not specifically limited, but the more preferable "5 to 14 membered non-aromatic heterocyclic group" includes pyrrolidinyl group, pyrrolinyl group, piperidinyl group, piperazinyl group, imidazolinyl group, pyrazolyl group, imidazolidinyl group, morpholinyl group, phthalimidoyl group, a succinimidoyl group etc.; the more preferable "C₆₋₁₄ aromatic hydrocarbocyclic group" includes phenyl group, indenyl group, naphthyl group, azulenyl group, heptalenyl group, biphenyl group etc.; the more preferable "5 to 14 membered aromatic heterocyclic group" includes pyrrolyl group, pyridyl group, pyridazinyl group, pyrimidinyl group, pyrazinyl group, pyrazolyl group, imidazolyl group, thienyl group, furyl group, thiazolyl group, iso-thiazolyl group, quinolyl group, iso-quinolyl group, indolyl group, benzimidazolyl group, benzothiazolyl group, benzoxazolyl group, carbazolyl group, dioxinyl group etc., respectively. Further, preferable examples of the "substituents" in the "which may optionally have substituents" include 1 or more groups selected from hydroxy group, a halogen atom (for example, fluorine atom, chlorine atom, bromine atom, iodine atom etc.), nitrile group, a C₁₋₆ alkyl group (for example, methyl group, ethyl group, n-propyl group, iso-propyl group, n-butyl group, iso-butyl group, tert-butyl group, n-pentyl group, iso-pentyl group, neopentyl group, n-hexyl group etc.) , a C₁₋₆ alkoxy group (methoxy group, ethoxy group, n-propoxy group, iso-propoxy group, sec-propoxy group, n-butoxy group, iso-butoxy group, sec-butoxy group, tert-butoxy group, n-pentoxy group, iso-pentoxy group, sec-pentoxy group, tert-pentoxy group, n-hexoxy group etc.), a C₁₋₆ alkoxy C₁₋₆ alkyl group (for example, methoxymethyl group, methoxyethyl group, ethoxymethyl group, ethoxyethyl group etc.), an aralkyl group (for example, benzyl group, phenethyl group, naphthylmethyl group, naphthylethyl group etc.), and the like. Further, an amino group, a cyclic amino group, and an alkoxyamino group which may optionally have substituents are also preferable as the substituents.

There is no particular limitation for "a salt" in the specification of the present application so far as it forms a salt with the compound of the present invention and is a pharmacologically acceptable one. Preferably, salt with a hydrogen halide (such as hydrofluoride, hydrochloride, hydrobromide or hydroiodide), salt with an inorganic acid (such as sulfate, nitrate, perchlorate, phosphate, carbonate or bicarbonate), salt with an organic carboxylic acid (such as acetate, trifluoroacetate, oxalate, maleate, tartrate, fumarate or citrate), salt with an organic sulfonic acid (such as methanesulfonate, trifluoromethanesulfonate, ethanesulfonate, benzenesulfonate, toluenesulfonate or camphor-sulfonate), salt with an amino acid (such as aspartate or glutamate), salt with a quaternary amine, salt with an alkaline metal (such as sodium salt or potassium salt) and salt with an alkaline earth metal (such as magnesium salt or calcium salt). More preferred examples of the "pharmacologically acceptable salt" are hydrochloride, oxalate etc.

compound (VIII) according to the present invention can be produced by a known method or its analogous method. Typical production methods are shown below. The "room temperature" mentioned in the following typical production methods, Reference Examples and Examples refers to 0 to about 40°C.

As the compound according to the present invention which is represented by the above formula (VIII), the compound represented by the following formula (I-4) wherein Z is N can be produced by converting an α-haloketone derivative (vii) into an α-ketone derivative azide (viii), then condensing (viii) with a hydrazine derivative (ii) to synthesize an α-azide hydrazide derivative (ix), further reducing (ix) to convert it into an α-aminohydrazide derivative (x) and then into a triazinone ring (xi), and introducing a substituent group to the 4-position of the ring, as shown in the reaction scheme:
Production Method 4 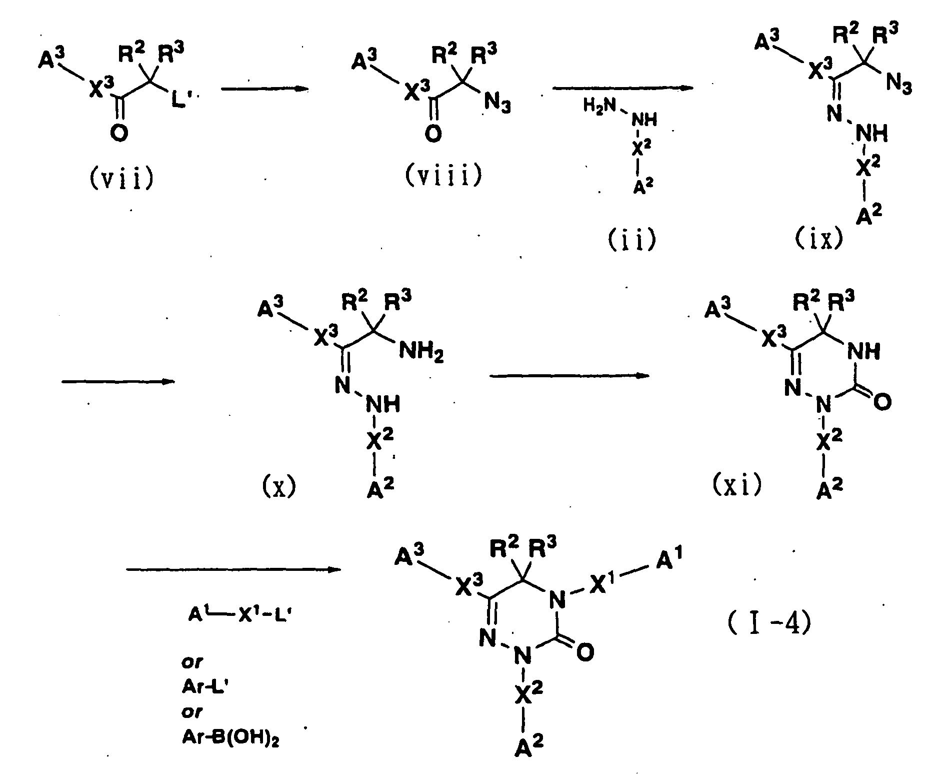 wherein A¹, A², A³ have the same meanings as defined above; X¹, X², X³ are a single bond and R² and R³ are H; L' represents a halogen atom such as chlorine atom, bromine atom or iodine atom; and Ar represents an optionally substituted aromatic ring or an optionally substituted heterocycle.

The azidating reagent used in the azidation reaction in producing (viii) varies depending on the starting material, the solvent used etc., and is not particularly limited insofar as it is inert to the reaction. The preferable examples include sodium azide, lithium azide etc. The azidation reaction is conducted preferably in the presence of a solvent from viewpoints of operativeness, stirring, safety and the like. The solvent used varies depending on the starting material, reagents etc., and is not particularly limited insofar as it is inert to the reaction and dissolves the starting material to a certain degree. The preferable examples include dimethylformamide, chloroform, dichloromethane, etc. The reaction temperature varies depending on the reagents used, the solvent etc., but usually the reaction is carried out at room temperature or less, preferably under ice-cooling, from viewpoint of safety.

The hydrazine derivative (ii) used in production of (ix) may be a salt, and is not particularly limited insofar as the reaction is not inhibited. From viewpoints of safety and availability, e.g. hydrochloride is preferable. The solvent used varies depending on the starting material, reagents etc., and is not particularly limited insofar as it is inert to the reaction and dissolves the starting material to a certain degree. Preferable examples include ethanol, toluene, chloroform, etc. The reaction temperature varies depending on the reagents used, the solvent etc., but usually the reaction is carried out at room temperature or by heating under reflux. Further, an acid catalyst such as p-toluene sulfonic acid or camphor sulfonic acid can be added as an additive in this reaction to give good results such as reduction in reaction time, improvement in yield, etc.

The conditions for reducing the azide group for production of (x) are not particularly limited insofar the conditions are gentle, and for this reduction, triphenylphosphine is preferably used. The solvent used varies depending on the starting material, reagents etc., and is not particularly limited insofar as it is inert to the reaction and dissolves the starting material to a certain degree Preferable examples include tetrahydrofuran, chloroform, toluene, etc. The reaction temperature varies depending on the reagents used, the solvent etc., but usually the reaction is carried out at room temperature or by heating under reflux, preferably from 60°C to 120°C.

For cyclization of the triazinone ring in production of (xi), it is preferable that (x) is reacted with a carbonylation reagent such as triphosgene, 1,1'-carbonyl diimidazole or diethyl carbonate, preferably triphosgene, in the presence of a base such as triethylamine. The solvent used varies depending on the starting material, reagents etc., and is not particularly limited insofar as it is inert to the reaction and dissolves the starting material to a certain degree. Preferable examples include tetrahydrofuran, acetonitrile, etc. The reaction temperature varies depending on the reagents used, the solvent etc., but usually the reaction is carried out under ice-cooling or by heating under reflux.

In the "step of introducing a substituent group to the 4-position of the triazinone derivative (xi) " as the final step for production of the compound (I-4) according to the present invention, a typical method for introducing an aryl group is, for example, Ullman reaction with a halogen aryl derivative. The reaction conditions are not particularly limited, and for example, the reaction is carried out in the presence of copper, copper bromide, copper iodide etc. with a base such as potassium carbonate, sodium carbonate, potassium acetate, sodium acetate, etc. in the system in a solvent under stirring. The solvent used varies depending on the starting material, reagents etc., and is not particularly limited insofar as it is inert to the reaction and dissolves the starting material to a certain degree. Preferable examples include dimethylformamide, dichlorobenzene, nitrobenzene, amyl alcohol, etc. The reaction temperature varies depending on the reagents used, the solvent etc., but usually the reaction can be finished in a short time by heating under reflux. An alternative method of introducing a substituent group to the 5-position of the triazinone derivative (xi) is a method of subjecting (xi) and an arylboronic acid derivative (Ar-B (OH)₂ in the reaction scheme above) to coupling reaction in the presence of a base with a copper compound. Preferably the arylboronic acid derivative used is, for example, an optionally substituted phenylboronic acid derivative or an optionally substituted heterocyclic boronic acid derivative. The base used varies depending on the other reagents used, the solvent used etc., and is not particularly limited insofar as the reaction is not inhibited. Preferable examples include triethylamine, pyridine, tetramethylethylenediamine, etc. Preferably the copper compound used is, for example, copper acetate, di-µ-hydroxo-bis[(N,N,N',N'-tetramethylethylene diamine) copper (II)] chloride or the like. This reaction is conducted preferably in the presence of a solvent. The solvent used varies depending on the starting material, reagents etc., and is not particularly limited insofar as it is inert to the reaction and dissolves the starting material to a certain degree. Preferable examples include dichloromethane, tetrahydrofuran, ethyl acetate, dimethylformamide, etc. Further, this reaction can be conducted in an oxygen atmosphere or an air stream to give good results such as reduction in reaction time, improvement in yield, etc. A base such as sodium hydride, tert-butoxy potassium etc. can be added to further improve yield.

### Production Method 5

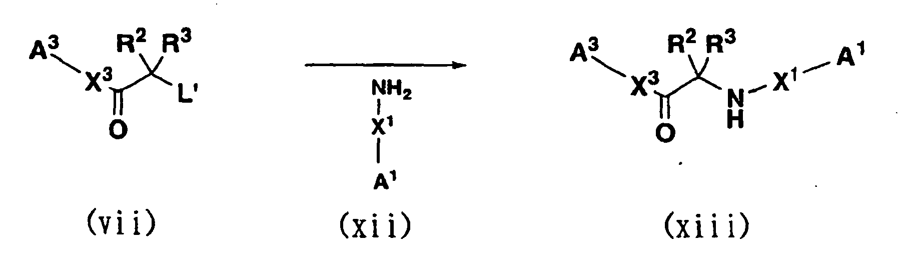

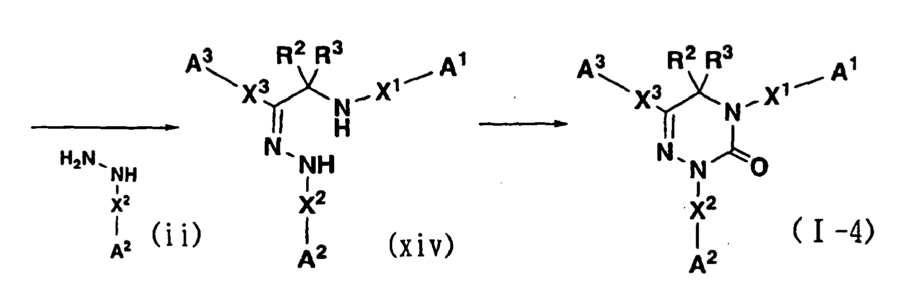

wherein X¹, X², X³, A¹, A², A³, R², R³ and L' have the same meanings as defined above. The compound represented by the above formula (I-4) according to the present invention can also be produced according to Production Method 5 shown in the above reaction scheme.

That is, an intermediate α-aminoketone derivative (xiii) is produced by condensation reaction of an α-haloketone derivative (vii) with an amine derivative (xii). From viewpoints of operativeness and stirring, this step is conducted preferably in a solvent in the presence of an organic base such as triethylamine, an inorganic base such as potassium carbonate, or an excess of an amine derivative (xii). The solvent used varies depending on the starting material, reagents etc., and is not particularly limited insofar as it is inert to the reaction and dissolves the starting material to a certain degree. Preferable examples include ethanol, acetone, tetrahydrofuran, etc. By adding potassium iodide, sodium iodide etc., good results such as reduction in reaction time, improvement in yield, etc. can be obtained.

An α-aminohydrazide derivative (xiv) is produced by condensation reaction of the α-aminoketone derivative (xiii) with a hydrazine derivative (ii). The substituted hydrazine derivative used may be a salt, and is not particularly limited insofar as the reaction is not inhibited. From viewpoints of stability and availability, it is preferably a hydrochloride. The solvent used varies depending on the starting material, reagents etc., and is not particularly limited insofar as it is inert to the reaction and dissolves the starting material to a certain degree. Preferable examples include ethanol, toluene, chloroform etc. The reaction temperature varies depending on the type of used reagent, solvent, catalyst etc., but usually the reaction is carried out at room temperature or by heating under reflux. An acid catalyst such as p-toluene sulfonic acid or camphor sulfonic acid can be added as an additive to give good results such as reduction in reaction time, improvement in yield, etc.

The "triazinone ring cyclization" which is the final step (i.e. the step from the intermediate (xiv) to (I-4)) for production of the compound (I-4) of the present invention is carried out by reacting (xiv) preferably with a carbonylation reagent such as triphosgene or a carbonylation reagent such as 1,1' -carbonyl diimidazole or diethyl carbonate in the presence of a base such as triethylamine. The reaction is carried out more preferably using triphosgene. The solvent used varies depending on the starting material, reagents etc., and is not particularly limited insofar as it is inert to the reactoin and dissolves the starting material to a certain degree. Preferable examples include tetrahydrofuran, acetonitrile etc. The reaction temperature varies depending on the reagents used, the solvent etc., but usually the reaction is carried out under ice-cooling or by heating under reflux.

### Production Method 6

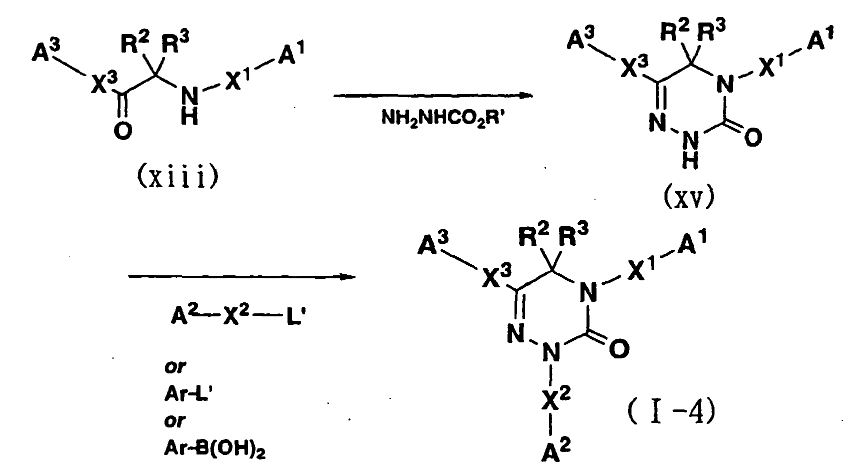

wherein X¹, X², X³, A¹, A², A³, R², R³, L' and Ar have the same meanings as defined above; and R' represents a C₁₋₆ alkyl or benzyl group.

The compound according to the present invention which is represented by the formula (I-4) can also be produced by condensation reaction of a hydrazinocarboxylate (NH₂NHCO₂R' in the reaction scheme) with the aminoketone derivative (xiii) produced in the above-mentioned Production Method 5, to synthesize a triazine derivative (xv), followed by introducing a substituent group to the 2-position of (xv).

The condensation reaction of (xiii) with the hydrazinocarboxylate (NH₂NHCO₂R' in the reaction scheme) is conducted preferably in the presence of a solvent from viewpoints of operativeness and stirring. The solvent used varies depending on the starting material, reagents etc., and is not particularly limited insofar as it is inert to the reaction and dissolves the starting material to a certain degree. Preferable examples include ethanol, toluene, xylene, etc. The reaction temperature varies depending on the reagents, solvent, catalyst etc., and is not particularly limited, but usually the reaction is carried out at room temperature or by heating under reflux, preferably at 40 to 120°C.

In the "step of introducing a substituent group to the 2-position of the triazinone derivative (xv) " as the final step for production of the compound (I-4) according to the present invention, a typical method for introducing an aryl group is, for example, Ullman reaction with a halogen aryl derivative. The reaction conditions are not particularly limited, and for example, the reaction is carried out in the presence of copper, copper bromide, copper iodide, etc. with a base such as potassium carbonate, sodium carbonate, potassium acetate, sodium acetate, etc. in a solvent under stirring. The solvent used varies depending on the starting material, reagents etc. , and is not particularly limited insofar as it is inert to the reaction and dissolves the starting material to a certain degree. Preferable examples include dimethylformamide, dichlorobenzene, nitrobenzene, amyl alcohol, etc. The reaction temperature varies depending on the reagents used, the solvent etc., but usually the reaction can be finished in a short time by heating under reflux. An alternative method of introducing the substituent group to the 2-position of the triazinone derivative (xv) is a method of subjecting (xv) and an arylboronic acid derivative (Ar-B (OH)₂ in the reaction scheme above) to coupling reaction in the presence of a base with a copper compound. Preferable examples of the arylboronic acid derivative used include an optionally substituted phenylboronic acid derivative or an optionally substituted heterocyclic boronic acid derivative. The base used varies depending on the starting material, the solvent used etc., and is not particularly limited insofar as it is inert to the reaction. Preferable examples include triethylamine, pyridine, tetramethylethylenediamine, etc. Preferable examples of the copper compound used include copper acetate, di-µ-hydroxo-bis[(N,N,N',N'-tetramethylethylenediamine) copper (II)] chloride, and the like. This reaction is conducted preferably in the presence of a solvent, and the solvent used varies depending on the starting material, reagents etc., and is not particularly limited insofar as it is inert to the reaction and dissolves the starting material to a certain degree. Preferable examples include dichloromethane, tetrahydrofuran, ethyl acetate, dimethylformamide, etc. Further, this reaction can be conducted in an oxygen atmosphere or an air stream to give good results such as reduction in reaction time, improvement in yield, etc.

When A¹, A² and/or A³ in the compound (VIII) in this invention have a substituent group, the substituent group can be easily converted by a known method or its analogous method. For example, (1) when the substituent group is a nitro group and, although there is no particular limitation for the method and for the resulting substance, a method of changing to an amine derivative by a reduction reaction may be exemplified. Although there is usually no particular limitation for the reduction condition, preferred conditions are a method where iron, zinc or tin is used under acidic conditions, a hydrogenation method where palladium, rhodium, ruthenium, platinum or a complex thereof is used as a catalyst. When the amine derivative produced by the said reduction reaction is used, it is possible to further change to an amide compound, a carbamate compound, a sulfonamide compound, a halogen compound, a substituted amine compound etc., easily. (2) When the substituents group is an alkoxy group, an example for changing to a functional group from an alkoxy group is a method to change to an alcohol derivative by means of deprotection. The alcohol derivative which is prepared by the said method may be easily changed to an ester compound by a dehydrating condensation with carboxylic acid derivative or by a reaction with an acid chloride or may be easily changed to an ether compound by a Mi tsunobu reaction or by a condensation reaction with a halogen compound. (3) When the substituent is an aldehyde group, various reactions are known for changing to a functional group from an aldehyde group and, although there is no particular limitation for the method therefor and the resulting substance by the change, an example is a method of changing to a carboxylic acid derivative by an oxidation reaction. The carboxylic acid derivative prepared by the said method may be easily changed further to an ester compound, a ketone compound, etc. In addition, starting from the said carboxylic acid derivative, it is possible to easily manufacture an alcohol derivative by a reduction reaction, an amine derivative by a reductive amination reaction, a secondary alcohol compound by an addition reaction with an organic metal reagent and various alkyl derivatives by a Wittig reaction. (4) When the substituent group is a halogen atom, an example for changing to a functional group from a halogen atom as a substituent is a method of changing to a nitrile derivative by a substitution reaction. Besides the above, it is also possible to easily change to various kinds of compounds via, for example, an organolithium compound, an organomagnesium compound, an organotin compound or an organoboronic acid derivative etc.

Described above are typical examples of the method of producing the compound (VIII) according to the present invention, and the starting compound and various reagents in production of the compound of the invention may be in the form of salts or hydrates, vary depending on the starting material, the solvent used etc. , and are not particularly limited so long as it is inert to the reaction. As a matter of course, the solvent used varies depending on the starting material, reagents etc., and is not particularly limited insofar as it is inert to the resaction and dissolves the starting material to a certain degree. When the compound (VIII) of the present invention is obtained in a free substance, it may be changed to a state of a salt by conventional methods. Further, various isomers (for example, a geometrical isomer, an enantiomer based on an asymmetric carbon, a rotamer, a stereoisomer, a tautomer, and the like) which are obtained for the compound (VIII) related to the present invention are purified by using usual separation procedures, for example, such as recrystallization, a diastereomer salt method, an enzymolysis method, various chromatographies (for example, thin layer chromatography, column chromatography, gas chromatography, and the like), and can be separated.

The compound according to the present invention which is represented by the above formula (VIII), a salt thereof or a hydrate of them can be used as they are, or mixed with pharmacologically acceptable carriers known per se, and formed into pharmaceutical preparations by a conventional method. As the preferable preparation forms, tablets, diluted powder, fine granules, granules, coated tablets, capsules, syrup, troche, inhalation preparation, suppositories, injections, ointments, eye ointments, eye drops, nasal preparations, ear drops, cataplasm, lotions etc. may be proposed. In the manufacture of the pharmaceutical preparations, it is possible to use commonly used fillers, binders, disintegrating agent, lubricants, coloring agents, corrigents and, if necessary, stabilizers, emulsifiers, absorption promoters, surfactant, pH adjusting agents, antiseptics, antioxidants, etc. and, after compounding with the ingredients commonly used as materials for the pharmaceutical preparations, it is made into pharmaceutical preparations by a common method.

Examples of the components thereof are animal and plant oil such as soybean oil, beef tallow or synthetic glyceride; hydrocarbon such as liquid paraffin, squalane or solid paraffin; ester oil such as octyldodecyl myristate or isopropyl myristate; higher alcohol such as cetostearyl alcohol or behenyl alcohol; silicone resin; silicone oil; surfactant such as polyoxyethylene fatty acid ester, sorbitan fatty acid ester, glycerol fatty acid ester, polyoxyethylene sorbitan fatty acid ester, polyoxyethylene hydrogenated castor oil or polyoxyethylene-polyoxypropylene block copolymer; water-soluble high-molecular substance such as hydroxyethyl cellulose, polyacrylic acid, carboxyvinyl polymer, polyethylene glycol, polyvinylpyrrolidone or methylcellulose; lower alcohol such as ethanol or isopropanol; polyhydric alcohol such as glycerol, propylene glycol, dipropylene glycol or sorbitol; saccharide such as glucose or sucrose; inorganic powder such as silicic acid anhydride, aluminum magnesium silicate or aluminum silicate; pure water and the like. Applicable examples of a filler are lactose, corn starch, pure sugar, glucose, mannitol, sorbitol, crystalline cellulose, silicon dioxide etc. ; those of a binder are polyvinyl alcohol, polyvinyl ether, methyl cellulose, ethyl cellulose, gum arabic, tragacanth, gelatin, shellac, hydroxypropyl methyl cellulose, hydroxypropyl cellulose, polyvinylpyrrolidone, polypropylene glycol-polyoxyethylene block copolymer, meglumine, calcium citrate, dextrin, pectin etc.; those of a disintegrating agent are starch, agar, gelatin powder, crystalline cellulose, calcium carbonate, sodium bicarbonate, calcium citrate, dextrin, pectin, carboxymethyl cellulose calcium etc.; those of a lubricant are magnesium stearate, talc, polyethylene glycol, silica, hydrogenated plant oil etc.; those of a coloring agent are those which are allowed to add to pharmaceuticals; those of a corrigent are cocoa powder, menthol, aromatic powder, peppermint oil, borneol and cinnamon powder; and those of an antioxidant are those which are permitted to be added to pharmaceuticals, such as ascorbic acid, α-tocopherol and the like, are respectively used.

In the manufacture of preparations for oral use, the compound of the present invention or a pharmacologically acceptable salt is mixed with a filler and, if necessary, further with a binder, a disintegrating agent, a lubricant, a coloring agent, a corrigent, etc. and the mixture is made into diluted powder, fine particles, granules, tablets, coated tablets, capsules, etc. by a common method.

In case of tablets and coated tablets, there is of course no problem that such tablets and granules are sugar-coated, gelatin-coated, or appropriately coated upon necessity.

In case of the manufacture of liquid preparations such as syrup, injection preparations and eye drops, a pH adjusting agent, a solubilizer, an isotonizing agent, etc. and, if necessary, a solubilizing aid, a stabilizer, buffer, suspending agent, antioxidant etc. are added, and then made into pharmaceutical preparations by a common method. It can be made as a freeze drying product, and a injections can be dosed in vena, subcutis, and muscle. Preferable examples in a suspending agent include methyl cellulose, polysorbate 80, hydoxyethyl cellulose, gum arabic, tragacanth powder, sodium carboxymethyl cellulose, polyoxyethylene sorbitan monolaurate, and the like; preferable examples in a resolving aid include polyoxyethylene hardened castor oil, polysorbate 80, nicotinic acid amide, polyoxyethylene sorbitan monolaurate, and the like; preferable examples in a stabilizer include sodium sulfite, meta sodium sulfite, ether, and the like; preferable examples in a preservative include methyl p-oxybenzoate, ethyl p-oxybenzoate, sorbic acid, phenol, cresol, chlorocresol and the like.

In case of external use, there is no particular limitation for a method of manufacturing a pharmaceutical preparation, but a common method is used for the manufacture. Thus, with regard to a base material used, various materials which are commonly used for pharmaceuticals, quasi drugs, cosmetics, etc. may be used. Specific examples of the base material used are animal/plant oil, mineral oil, ester oil, waxes, higher alcohols, fatty acids, silicone oil, surfactant, phospholipids, alcohols, polyhydric alcohols, water-soluble high-molecular substances, clay minerals and pure water and, if necessary, it is possible to add pH adjusting agent, antioxidant, chelating agent, antiseptic antifungal, coloring agent, perfume, etc. If necessary, it is further possible to compound other components such as a component having a differentiation-inducing action, blood flow promoter, bactericide, antiinflammatory agent, cell activator, vitamins, amino acid, moisturizer and keratin solubilizing agent.

The pharmaceutical preparation comprising the compound (VIII) according to the present invention, a salt thereof or a hydrate of them, as an active ingredient is useful for treatment and prevention in mammalians (e.g., humans, mice, rats, guinea pigs, rabbits, dogs, horses, monkeys etc.), particularly in treatment and prevention in humans. Dose of the pharmaceutical preparation according to the present invention varies depending on degree of symptom, age, sex, body weight, dosage form, type of salt, sensitivity to the pharmaceuticals, specific type of the disease, etc. In humans, the pharmaceutical preparation is given daily in one portion or in divided portions into an adult in a dose of usually about 30 µg to 10 g, preferably 100 µg to 10 g, more preferably 100 µg to 5 g for oral administration, or about 30 µg to 10 g for injection.

In accordance with the present invention, it is possible to provide a novel compound (VIII) which shows an excellent inhibiting action to AMPA receptor and/or kainate receptor and is useful as pharmaceutical agents. Further, a useful production process for producing the compound or its salt and a production intermediate could be provided. According to this process, the compound relating to the present invention can be obtained in high yield, and the highly safe compound can be obtained. The compound (VIII) of the present invention suppress the neurotoxicity of excitatory neurotransmitters and is able to achieve an excellent neuroprotecting action as a pharmaceutical agent. Accordingly, the compounds of the present invention are useful as therapeutic, preventive and improving agents for epilepsy and multiple sclerosis.

### Examples

Reference Examples, Examples (further pharmacologically acceptable salts thereof, hydrates thereof, and pharmaceutical preparations or pharmaceutical composition comprising them) and Test Example shown below are described merely for illustrative purposes, and the compounds of the invention are not limited to the following examples in any case. The present invention can be carried out to the maximum by those skilled in the art by making various modifications not only to the following examples but also to claims in this specification, and such modifications fall under the claims in this specification.

### Reference Example 1

### 1-(2-Pyridyl)-3-(3-methoxyphenyl)-2-propen-1-one

Potassium tert-butoxide (2.4 g) was added to a solution of 2-acetylpyridine (25 g) and 3-methoxybenzaldehyde (28 g) in tetrahydrofuran (150 ml), followed by stirring for 5 hours. The reaction mixture was partitioned between ethyl acetate and water, and the organic layer was washed with water, dried and concentrated. The residue was purified by silica gel column (ethyl acetate-hexane system), to give the title compound (17.2 g) as a yellow solid.
¹H-NMR(400MHz, CDCl₃); δ (ppm) 3.87 (s, 3H), 6.96-6.99 (m, 1H), 7.24-7.26(m, 1H), 7.32-7.34(m, 2H), 7.50(ddd, 1H), 7.88(dt, 1H), 7.91(d, 1H), 8.19(td, 1H), 8.28(d, 1H), 8.75(ddd, 1H).

### Reference Example 2

### 2-(3-Methoxyphenyl)-4-(2-pyridyl)-4-oxobutanenitrile

According to J. Chem. Soc. (1958) 4193, the title compound (16.7 g) was obtained as a brown oil from 1-(2-pyridyl)-3-(3-methoxyphenyl)-2-propen-1-one (17.2 g).
¹H-NMR(400MHz, CDC1₃) ; δ(ppm) 3.80(dd, 1H), 3.82(s, 3H), 4.00(dd, 1H), 4.50(dd, 1H), 6.86(dd, 1H), 6.97(t, 1H), 7.00-7.03(m, 1H), 7.29(t, 1H), 7.50(ddd, 1H), 7.86(td, 1H), 8.07(td, 1H), 8.65(ddd, 1H).

### Reference Example 3

### 2-(3-Methoxyphenyl)-4-(2-pyridyl)-4-oxobutyric acid

According to J. Heterocyclic. Chem., 25, 799 (1988), the title compound (12.3 g) was obtained as a brown solid from 2-(3-methoxyphenyl)-4-(2-pyridyl)-4-oxobutanenitrile (16.7
¹H-NMR(400MHz, CDCl₃); δ (ppm) 3.52-3.58 (m, 1H), 3.77 (dd, 1H), 3.79(s, 1H), 8.55(dd, 1H), 6.82(ddd, 1H), 6.85-6.89(m, 1H), 6.94 (t, 1H), 6.98 (d, 1H), 7.47 (ddd, 1H), 7.83 (dt, 1H), 8.02 (d, 1H), 8.67(ddd, 1H).

### Reference Example 4

### Ethyl 4-(2-methoxyphenyl)-2-(2-pyridyl)-4-oxobutylate

60% sodium hydride (1.5 g) was added to a solution of ethyl 2-pyridylacetate (5.5 g) in dimethylformamide (50 ml) under ice-cooling, followed by stirring. After 1 hour, 2-methoxyphenacyl bromide (7.7 g) was added thereto, and the mixture was stirred for 1 hour under ice-cooling and then stirred overnight at room temperature. The reaction mixture was evaporated, and partitioned between ethyl acetate and water. The organic layer was washed with water, dried and concentrated. The residue was purified by silica gel column (ethyl acetate-hexane system), to give the title compound (6.6 g) as a reddish brown solid.
¹H-NMR(400MHz, CDC1₃) ; δ (ppm) 1.20(1, 3H), 3.59(dd, 1H), 3.88(s, 3H), 3.95(dd, 1H), 4.11-4.20(m, 2H), 4.51(dd, 1H), 6.94-7.00(m, 2H), 7.15-7.18 (m, 1H), 7.36 (dt, 1H), 7.43-7.47 (m, 1H), 7.65(td; 1H), 7.73(dd, 1H), 8.54-8.56(m, 1H).

### Reference Example 5

### 4-Phenyl-2,3,4,4a-tetrahydro-5H-(1)benzopyrano[4,3-c]pyridazin-3-one

4-Oxo-4*H*-2,3-dihydro-1-benzopyran-3-acetic acid (4.00 g) synthesized according to the method described in Example 1 was dissolved in ethanol (60 ml), and hydrazine monohydrate (0.68 g) was added thereto, followed by heating under reflux for 3 hours. After cooling as it was to room temperature, the resulting crystals were separated by filtration, to give the title compound (1.95 g, 49%).
¹H-NMR (400MHz, DMSO-d₆); δ (ppm) 3.65-3.84 (m, 3H), 4.00 (dd, 1H), 6.91(dd, 1H), 7.04(ddd, 1H), 7.27-7.41(m, 6H), 7.90(dd, 1H), 11.18(s, 1H).

### Reference Example 6

### 3-Chloro-6-methoxy-5-tributyltin pyridazine

2.5 M butyl lithium (19.4 ml) was added to a solution of diisopropylamine (6.7ml) in tetrahydrofuran (60 ml) at -40°C in a nitrogen atmosphere. After stirring for 20 minutes under ice-cooling, a solution of 3-chloro-6-methoxypyridazine (5.76 g) and tributyltin chloride (15.56 g) in tetrahydrofuran (30 ml) was added dropwise thereinto at -72°C and stirred for 1 hour. Water was added thereto, and the mixture was extracted with ethyl acetate. The organic layer was washed with water, dried and concentrated. Then, the residue was purified by silica gel column chromatography (ethyl acetate/hexane system), to give the title compound (12.77 g) as a pale yellow oil.
¹H-NMR (400MHz, CDCl₃); δ (ppm) 0.89 (t, 9H), 1.10-1.15 (m, 6H), 1.27-1.36 (m, 6H), 1.48-1.53(m, 6H), 4.06(s, 3H), 7.38(s, 1H).

### Reference Example 7

### 3-Chloro-6-methoxy-5-phenylpyridazine

3-Chloro-6-methoxy-5-tributyltin pyridazine (3.20 g), bromobenzene (11.57 g), tetrakis(triphenylphosphine)palladium (428 mg) and copper (I) iodide (70 mg) were added to xylene (130 ml), followed by stirring at 120°C for 2 hours in a nitrogen atmosphere. The reaction mixture was purified by silica gel column chromatography (ethyl acetate/hexane system), to give the title compound (1.10 g) as a colorless oil.
¹H-NMR (400MHz, CDCl₃); δ (ppm) 4.16 (s, 3H), 7.40 (s, 1H), 7.47-7.50(m, 3H), 7.60-7.63(m, 2H).

### Reference Example 8

### 6-Chloro-4-phenyl-3(2H)-pyridazinone

A reaction mixture of 3-chloro-6-methoxy-5-phenylpyridazine (267 mg) and conc. hydrochloric acid (2 ml) was heated under reflux for 2 hours. After concentrating, the residue was purified by silica gel column chromatography (ethyl acetate), to give the title compound (159 mg) as a colorless solid.
¹H-NMR (400MHz, CDCl₃); δ (ppm) 7.38 (s, 1H), 7.47-7.49 (m, 3H), 7.81-7.84(m, 2H), 11.34(brs, 1H).

### Reference Example 9

### 6-Chloro-2-(2-cyanophenyl)-4-phenyl-3(2H)-pyridazinone

A suspension of 6-chloro-4-phenyl-3(2*H*)-pyridazinone (80 mg), 2-(2-cyanophenyl)-1,3,2-dioxaborinate (144 mg), copper (II) acetate (35 mg), triethylamine (107 µl) and pyridine (62 µl) in methylene chloride (5 ml) was stirred for 4 days in an oxygen atmosphere. The reaction mixture was partitioned between aqueous ammonia and ethyl acetate, and the organic layer was washed with water, dried and concentrated. Then, the residue was purified by silica gel column chromatography (ethyl acetate/hexane system) to give the title compound (83 mg) as a colorless solid.
¹H-NMR (400MHz, CDCl₃); δ (ppm) 7.43 (s, 1H), 7.46-7.50 (m, 3H), 7.56(dt, 1H), 7.68(ddd, 1H), 7.76(ddd, 1H), 7.81-7.84(m, 3H).

### Reference Example 10

### 3-Methoxy-4-phenyl-6- (2-pyrimidinyl)pyridazine

2-Tributylstannylpyrimidine (2.10 g) prepared according to Tetrahydron 50, 275-284 (1994), 3-chloro-6-methoxy-5-phenylpyridazine (800 mg) and tetrakis(triphenylphosphine)palladium (208 mg) were added to xylene (10 ml), followed by stirring at 120°C for 2 hours in a nitrogen atmosphere. The reaction mixture was purified by NH-silica gel column chromatography (ethyl acetate/hexane system), to give the title compound (403 mg) as a brown solid.
¹H-NMR (400MHz, CDCl₃); δ (ppm) 4.28 (s, 3H), 7.37 (t, 1H), 7.46-7.53 (m, 3H), 7.73-7.76 (m, 2H), 8.52 (s, 1H), 8.94 (d, 2H).

### Reference Example 11

### 4-Phenyl-6-(2-pyrimidinyl)-3(2H)-pyridazinone

A solution of 3-methoxy-4-phenyl-6-(2-pyrimidinyl)pyridazine (1.07 g) in 5 N hydrochloric acid (15 ml) was heated under reflux for 2 hours. After neutralizing with 5 N aqueous sodium hydroxide solution, the resulting precipitates were collected by filtration and washed with ethyl acetate, to give the title compound (609 mg) as a colorless solid.
¹H-NMR (40OMHz, CDCl₃); δ (ppm) 7.38 (t, 1H), 7.47-7.53 (m, 3H), 7.95-7.98(m, 2H), 8.60(s, 1H), 8.95(d, 2H).

### Reference Example 12

### 2-(Azide acetyl) pyridine

Acetyl pyridine (2.46 g) was dissolved in acetic acid (4 ml), and bromine (1.1 ml) was added gradually dropwise thereto under heating at 70°C. After cooling the reaction solution as it was to room temperature, the resulting crystals were collected by filtration. An aqueous sodium bicarbonate solution was added to the crude crystals (4.8 g), followed by extracting with ethyl acetate. The organic layer was washed with water and dried over sodium sulfate anhydride. After the drying agent was filtered off, the filtrate was evaporated. The residue (4.06 g) was dissolved in dimethylformamide (50 ml), sodium azide (1.5 g) was added thereto, and the mixture was stirred at room temperature for 2 hours. Water was added thereto, followed by extracting with ethyl acetate. The organic layer was washed with water and dried over sodium sulfate anhydride. After the drying agent was filtered off, the filtrate was evaporated, to give the title compound as a brown oil (2.74 g, 83%).
¹H-NMR (400MHz, CDCl₃); δ (ppm) 4.87 (s, 2H), 7.51-7.55 (m, 1H), 7.87-7.91 (m, 1H), 8.09 (d, J=8.0 Hz, 1H), 8.66 (dt, J=4.8 Hz, 0.8 Hz, 1H).

### Reference Example 13

### 2-Pyridyl-aminomethyl-2'-bromophenylhyrazone

2-(Azide acetyl) pyridine (2.7 g) was dissolved in ethanol (50 ml) and 2-bromophenylhydrazine (3.4 g) was added thereto, followed by stirring overnight at room temperature. The reaction mixture was evaporated, and the residue was dissolved in tetrahydrofuran (40 ml). Triphenylphosphine (4.94 g) was added thereto, followed by stirring at room temperature for 3 hours. Water (1 ml) was added thereto, and the mixture was stirred for 1 hour and then heated overnight at 80°C. After cooling to room temperature, the mixture was evaporated. The residue was purified by silica gel column chromatography (hexane-ethyl acetate system), to give the title compound (2.69 g, 53%) as a brown oil.
¹H-NMR (400MHz, CDCl₃); δ (ppm) 1.84 (brs, 2H), 4.41 (s, 2H), 6.74 (td, J=8.4 Hz, 1.2 Hz, 1H), 7.16-7.17 (m, 1H), 7.25-7.30 (m, 1H), 7.45 (dd, J=8.0 Hz, 1.2 Hz, 1H), 7.62 (dd, J=8.4 Hz, 1.6 Hz, 1H), 7.67 (td, J=8.0 Hz, 0.8 Hz, 1H), 8.14 (d, J=8.0 Hz, 1H), 8.50-8.51 (m, 1H), 11.39 (brs, 1H).

### Reference Example 14

### 2-(2-Bromophenyl)-6-(2-pyridyl)-4,5-dihydro-1,2,4-triazin-3-(2H)-one

2-Pyridyl-aminomethyl-2'-bromophenylhydrazone (2.69 g) was dissolved in tetrahydrofuran anhydride (100 ml), followed by adding triphosgene (1.31 g) and triethylamine (2.7 ml) under ice-cooling while raising the temperature of the mixture gradually from 0°C to room temperature, the mixture was stirred overnight. The insoluble matters were filtered off, and the filtrate was evaporated. The residue was purified by NH-silica gel column chromatography (hexane-ethyl acetate system), to give the title compound (1.00 g, 31%) as a brown powder.
¹H-NMR (400MHz, CDCl₃); δ (ppm) 4.79 (s, 2H), 5.50 (s, 1H), 7.23-7.30 (m, 2H), 7.39-7.48 (m, 1H), 7.53 (dd, J=7.8 Hz, 1.8 Hz, 1H), 7.67-7.72 (m, 2H), 8.04 (d, J-8.0 Hz, 1H), 8.57 (ddd, J=4.8 Hz, 1.8 Hz, 1.0 Hz, 1H).
ESI-Mass; 331 [M⁺+H]

### Example 85

### 2-(2-Bromophenyl)-4-(2-methoxyphenyl)-6-(2-pyridyl)-4,5-dihydro-1,2,4-triazin-3(2H)-one

2-(2-Bromophenyl)-6-(2-pyridyl)-4,5-dihydro-1,2,4-triazin-3(*2H*)-one (70 mg) was dissolved in tetrahydrofuran anhydride (10 ml), and copper acetate (77 mg), sodium hydride (25 mg) and 2-methoxyphenylboronic acid (77 mg) were added thereto, followed by stirring at room temperature for 1 hour. Sodium hydride (25 mg) and 2-methoxyphenylboronic acid (50 mg) were further added thereto and stirred at room temperature for 6 hours. Then, the organic layer was partitioned by adding ethyl acetate and aqueous ammonia thereto. The organic layer was washed with water and dried over anhydrous sodium sulfate. After the drying agent was filtered off, the filtrate was evaporated. The residue was purified by silica gel column chromatography (hexane-ethyl acetate system), to give the title compound (55 mg, 60%) as a colorless powder.
¹H-NMR (400MHz, CDCl₃); δ (ppm) 3.85 (s, 3H), 5.02 (s, 2H) 6.94-6.99 (m, 2H), 7.20-7.31 (m, 3H), 7.35-7.42 (m, 2H), 7.62 (dd, J=7.6Hz, 1.6 Hz, 1H), 7.66-7.73 (m, 2H), 8.09-8.11 (m,1H), 8.54 (ddd. J=5.0 Hz, 1.8 Hz, 1.6 Hz, 1H).

### Example 86

### 2-(2-Bromophenyl)-4-(2-hydroxyphenyl)-6-(2-pyridyl)-4,5-dihydro-1,2,4-triazin-3(2H)-one

1 M boron tribromide in methylene chloride (0.3 ml) was added to a solution of 2-(2-bromophenyl)-4-(2-methoxy phenyl)-6-(2-pyridyl)-4,5-dihydro-1,2,4-triazin-3(2*H*)-one (48 mg) in dichloromethane (10 ml) under ice-cooling and stirred for 5 hours. The organic layer was partitioned by adding an aqueous saturated sodium bicarbonate solution and dichloromethane to the reaction solution, washed with water and dried over anhydrous sodium sulfate. After the drying agent filtered off, the filtrate was evaporated. The residue was purified by silica gel column chromatography (hexane-ethyl acetate system), to give the title compound (36 mg, 78%) as a colorless amorphous.
¹H-NMR (400MHz, CDCl₃); δ (ppm) 5.23 (brs, 2H), 7.02-7.10 (m, 2H), 7.20-7.36 (m, 4H), 7.46 (td, J=8.0 Hz, 1.2 Hz, 1H), (dd, J=8.0 Hz, 1.8 Hz, 1H), 7.71-7.76 (m, 2H), 8.09 (d, J=8.0 Hz, 1H), 8.59 (d, J=5.2 Hz, 1H).
ESI-Mass; 423 [M⁺+H]

### Example 87

### 2-(2-Cyanophenyl)-4-(2-methoxyphenyl)-6-(2-pyridyl)-4,5-dihydro-1,2,4-triazin-3(2H)-one

2-(2-Bromophenyl)-4-(2-methoxyphenyl)-6-(2-pyridyl)-4,5-dihydro-1,2,4-triazin-3(2*H*)-one (99 mg) was dissolved in dimethylformamide (20 ml), and copper cyanide (51 mg) was added thereto, followed by stirring at 150°C for 3 hours. After cooling the reaction solution to room temperature, the organic layer was partitioned by adding aqueous ammonia (20 ml) and ethyl acetate thereto. The organic layer was washed with water and dried over anhydrous sodium sulfate. After the drying agent was filtered off, the residue was purified by silica gel column chromatography (hexane-ethyl acetate system), to give the title compound (72 mg, 83%) as a colorless amorphous.
¹H-NMR (400MHz, CDCl₃) ; δ (ppm) 3.87 (s, 3H), 5.04 (s, 2H), 6.97-7.01 (m, 2H), 7.25-7.39 (m, 4H), 7.61-7.66 (m, 1H), 7.72-7.78 (m, 3H), 8.22 (d, J=8.0 Hz, 1H), 8.54 (ddd, J=4.8Hz, 1.6 Hz, 1.2 Hz, 1H).
ESI-Mass; 384 [M⁺+H]

### Example 88

### 2-(2-Bromophenyl)-4-phenyl-6-(2-pyridyl)-4,5-dihydro-1,2,4-triazin-3(2H)-one

2-(2-Bromophenyl)-6-(2-pyridyl)-4,5-dihydro-1,2,4-triazin-3(2*H*)-one (12 mg) was dissolved in anhydrous dichloromethane (30 ml), and triethylamine (0.1 ml), copper acetate (13.2 mg) and phenylboronic acid (13.3 mg) were added thereto, followed by stirring at room temperature for 48 hours. Sodium hydride (3 mg) and phenylboronic acid (10 mg) were further added thereto and stirred at room temperature for 5 hours. Then, the organic layer was partitioned by adding aqueous ammonia and ethyl acetate thereto. The organic layer was washed with water and dried over anhydrous sodium sulfate. After the drying agent was filtered off, the residue was purified by NH silica gel column chromatography (hexane-ethyl acetate system), to give the title compound (11 mg, 75%) as a colorless powder.
¹H-NMR (400MHz, CDCl₃); δ (ppm) 5.15 (brs, 2H), 7.21-7.31 (m, 3H), 7.36-7.46 (m, 3H), 7.47-7.52 (m, 2H), 7.62 (dd, J=8.0Hz, 1.8 Hz, 1H), 7.68-7.74 (m, 2H), 8.10 (dt, J=8.0 Hz, 1.4 Hz, 1H), 8.57 (ddd, J=5.0 Hz, 1.8 Hz, 0.8 Hz, 1H).
ESI-Mass; 407 [M*+H]

### Example 89

### 2-(2-Bromophenyl)-6-(2-methoxyphenyl)-4-phenyl-4,5-dihydro-1,2,4-triazin-3(2H) -one

According to the method of Horst Gnichtel, Widah I. Salem und Lothar Waretschek; Liebigs Ann. Chem. (1978) 2033-2043, the synthesis was carried out as follows. 2-Bromo-2'-methoxyacetophenone (1.33 g) and aniline (1.06 g) were dissolved in ethanol (20 ml) and stirred at room temperature for 72 hours. After the insoluble matters were filtered off, the filtrate was evaporated. The residue was purified by silica gel column chromatography (hexane-ethyl acetate system). The resulting colorless oily acetophenone derivative (1.12 g) was dissolved in ethanol (20 ml), and 2-bromophenyl hydrazine (930 mg) was added thereto, followed by stirring at room temperature overnight. The reaction solution was evaporated, and the residue (1.84 g) was dissolved in tetrahydrofuran (20 ml). Triphosgene (459 mg) and triethylamine (1.4 ml) were added thereto under ice-cooling, and the mixture was stirred for 3 hours while the temperature of the mixture was gradually raised to room temperature. The reaction solution was evaporated, and the residue was purified by silica gel column chromatography (hexane-ethyl acetate), to give the title compound (438 mg, 17%) as a colorless amorphous.
¹H-NMR (400MHz, CDCl₃); δ (ppm) 3.87 (s, 3H), 4.85 (s, 2H), 6. 92 (d, J=8.4 Hz, 1H), 7.01 (t, J=7.4 Hz, 1H), 7.18-7.25 (m, 2H), 7.27-7.46 (m, 6H), 7.64-7.68 (m, 2H), 7.71 (dt, J=7.6 Hz, 1.6 Hz, 1H).
ESI-Mass; 436 [M⁺+H]

### Example 90

### 2-(2-Bromophenyl)-6-(2-hydroxyphenyl)-4-phenyl-4,5-dihydro-1,2,4-triazin-3(2H)-one

According to the method described in Example 86, the title compound (135 mg, 95%) was obtained form 2-(2-bromophenyl)-6-(2-methoxyphenyl)-4-phenyl-4,5-dihydro-1,2,4-triazin-3(2*H*)-one (147 mg).
¹H-NMR (400MHz, CDCl₃); δ (ppm) 4.98 (s, 2H), 6.91 (t, J=8.0 Hz, 1H), 7.00 (dd, J=8.0 Hz, 0.8 Hz, 1H), 7.25-7.35 (m, 4H), 7.40-7.48 (m, 5H), 7.58 (dd, J=8.0 Hz, 1.6 Hz, 1H), 7.69 (dd, J=7.8 Hz, 1.4 Hz, 1H), 10.96 (s, 1H).
ESI-Mass; 424 [M*+H]

### Example 91

### 2-(2-Bromophenyl)-6-(2-dimethylaminoethoxyphenyl)-4-phenyl-4,5-dihydro-1,2,4-triazin-3(2H)-one

2-(2-Bromophenyl)-6-(2-hydroxyphenyl)-4-phenyl-4,5-dihydro-1,2,4-triazin-3(2*H*)-one (100 mg) was dissolved in dimethylformamide (20 ml), and potassium carbonate (66 mg) was added thereto. An excess amount of dimethylaminoethyl chloride was added dropwise thereto and stirred at 120°C overnight. After cooling the reaction solution to room temperature, the organic layer was partitioned by adding water and ethyl acetate thereto. The organic layer was washed with water and dried over anhydrous sodium sulfate. After the drying agent was filtered off, the filtrate was evpaorated. The residue was purified by silica gel column chromatography (hexane-ethyl acetate system), to give the title compound (65 mg. 55%) as colorless crystals.
¹H-NMR (400MHz, CDCl₃); δ (ppm) 2.26 (s, 6H), 2.65 (t, J=5.8 Hz, 2H), 4.11 (t, J=5.8 Hz, 2H), 4.93 (s, 2H), 6.91 (d, J=8.0 Hz, 1H), 6.98 (td, J=7.6 Hz, 0.8 Hz, 1H), 7.16-7.25 (m, 2H), 7.33-7.39 (m, 3H), 7.40-7.46 (m, 3H), 7.66 (td, J=8.0 Hz, 1.6 Hz, 2H), 7.72 (dd, J=7.8 Hz, 1.8 Hz, 1H).
ESI-Mass; 495 [M⁺+H]

### Example 92

### 2-(2-Bromophenyl)-6-(2-methoxyphenyl)-4-(2-pyridyl)-4,5-dihydro-1,2,4-triazin-3(2H)-one

2-(2-Bromophenyl)-6-(2-methoxyphenyl)-4,5-dihydro-1,2,4-triazin-3(2*H*)-one (193 mg) synthesized according to the method described in Examples 85 to 87 was dissolved in dimethylformamide (20 ml), and 2-bromopyridine (300 mg), potassium carbonate (185 mg) and copper iodide (20.4 mg) were added thereto, followed by heating at 130°C for 5 hours. After cooling to room temperature, the organic layer was partitioned by adding aqueous ammonia (20 ml) and ethyl acetate thereto. The organic layer was washed with water and dried over anhydrous sodium sulfate. After the drying agent was filtered off, the filtrate was evaporated. The residue was purified by NH silica gel column chromatography (hexane-ethyl acetate system), to give the title compound (126 mg, 54%) as a colorless powder.
¹H-NMR (400MHz, CDCl₃) ;δ (ppm) 3.84 (s, 3H), 5.10 (brs, 2H), 6.86-6.97 (m, 3H), 7.14-7.18 (m, 1H), 7.29-7.38 (m, 2H), 7.54-7.63 (m, 4H), 7.92-7.95 (m, 1H), 8.29-8.31 (m, 1H).
ESI-Mass; 437 [M⁺+H]

The title compounds of Examples 93 to 96 were synthesized according to the method described in the above-mentioned Example 86.

### Example 93

### 2-(2-Cyanophenyl)-6-(2-hydroxyphenyl)-4-phenyl-4,5-dihydro-1,2,4-triazin-3(2H)-one

¹H-NMR (400MHz, CDCl₃); δ (ppm) 5.00 (s, 2H), 6.91-6.96 (m, 1H), 7.02 (dd, J=8.4 Hz, 0.8 Hz, 1H), 7.27-7.49 (m, 8H), 7.70-7.73 (m, 2H), 7.73-7.77 (m, 1H), 10.83 (s, 1H).
ESI-Mass; 369 [M⁺ +H]

### Example 94

### 2-(2-Bromophenyl)-4-(2,5-dihydroxyphenyl)-6-(2-hydroxyphenyl)-4,5-dihydro-1,2,4-triazin-3(2H)-one

¹H-NMR (400MHz, DMSO-d₆); δ (ppm) 4.79 (s, 2H), 6.59 (dd, J=8.8 Hz, 2.8 Hz, 1H), 6.70-6.74 (m, 2H), 6.89 (dd, J= 13.2 Hz, 0,8 Hz, 2H), 7.28-7.37 (m, 2H), 7.49-7.57 (m, 2H), 7.66 (dd, J=7.8 Hz, 1.0 Hz, 1H), 7.76 (d, J=8.2 Hz, 1H), 8.92 (s, 1H), 9.09 (brs, 1H), 10.52 (brs, 1H).
ESI-Mass; 454 [M⁺+H]

### Example 95

### 4-(2,5-Dihydroxyphenyl)-6-(2-hydroxyphenyl)-2-phenyl-4,5-dihydro-1,2,4-triazin-3(2H)-one

¹H-NMR (400MHz, DMSO-d₆); δ (ppm) 4.78 (s, 2H), 6.60 (dd, J=8.8 Hz, 2.8 Hz, 1H), 6.70-6.75 (m, 2H), 6.91 (dd, J=12.8 Hz, 7.6 Hz, 2H), 7.23-7.34 (m, 2H), 7.39-7.45 (m, 2H), 7.54-7.61 (m, 3H), 8.92 (s, 1H), 9.04 (s, 1H), 10.74 (s, 1H).

### Example 96

### 2-(2-Cyanophenyl)-4-(2-hydroxyphenyl)-6-(2-pyridyl)-4,5-dihydro-1,2,4-triazin-3(2H)-one

¹H-NMR (400MHz, CDCl₃); δ (ppm) 3.87 (s, 2H), 6.69-6.82 (m, 1H), 6.94-7.10 (m, 2H), 7.22-7.52 (m, 5H), 7.62-7.66 (m, 1H), 7.72-7.76 (m, 1H), 7.98-8.05 (m, 1H), 8.54-8.67 (m, 1H).
ESI-Mass; 370 [M⁺+H]

The title compounds of Examples 97 to 103 were synthesized according to the method described in the above-mentioned Example 87.

### Example 97

### 2-(2-Cyanophenyl)-6-(2-methoxyphenyl)-4-phenyl-4,5-dihydro-1,2,4-triazin-3(2H)-one

¹H-NMR (400MHz, CDCl₃); δ (ppm) 3.87 (s, 3H), 4.86 (s, 2H) 6.93 (d, J=8.4 Hz, 1H), 7.04 (td, J=7.2 Hz, 0.8 Hz, 1H), 7.20-7.27 (m, 1H), 7.34-7.45 (m, 6H), 7.65 (td, J=7.8 Hz, 1.6 Hz, 1H), 7.71 (dd, J-7.6 Hz, 1.6 Hz, 1H), 7.77-7.81 (m, 2H).
ESI-Mass; 383 [M⁺+H]

### Example 98

### 4-(2-Cyanophenyl)-6-(2-methoxyphenyl)-2-phenyl-4,5-dihydro-1,2,4-triazin-3(2H)-one

¹H-NMR (400MHz, CDCl₃) δ (ppm) 3.85 (s, 3H), 4.85 (s, 2H), 6.90-6.94 (m, 1H), 6.99-7.06 (m, 1H), 7.20-7.26 (m, 1H), 7.33-7.43 (m, 4H), 7.49-7.52 (m, 1H), 7.62-7.77 (m, 5H).
ESI-Mass; 383 [M⁺+H]

### Example 99

### 2-(2-Cyanophenyl)-6-(2-methoxyphenyl)-4-(2-pyridyl)-4,5-dihydro-1,2,4-triazin-3(2H)-one

¹H-NMR (400MHz, CDCl₃); δ (ppm) 3.91 (s, 3H), 5.21 (s, 2H), 6.95 (d, J=8.4 Hz, 1H), 7.00-7.08 (m, 2H), 7.39-7.44 (m, 2H), 7.64-7.80 (m, 5H), 7.98-8.01 (m, 1H), 8.39 (ddd, J=4.8 Hz, 1.8 Hz, 0.8 Hz, 1H)
ESI-Mass; 384 [M⁺+H]

### Example 100

### 2-(2-Cyanophenyl)-4-phenyl-6-(2-pyridyl)-4,5-dihydro-1,2,4-triazin-3(2H)-one

¹H-NMR (400MHz, CDCl₃); δ (ppm) 5.17 (s, 2H), 7.24-7.33 (m, 2H), 7.38-7.44 (m, 3H), 7.46-7.50 (m, 2H), 7.65-7.69 (m, 1H), 7.74-7.79 (m, 3H), 8.19-8.22 (m, 1H), 8.57 (ddd, J=4.8 Hz, 1.6Hz, 0.8 Hz, 1H).
ESI-Mass; 354 [M⁺+H]

### Example 101

### 2-(2-Cyanophenyl)-6-(2-pyridyl)-4-(thiophene-3-yl)-4,5-dihydro-1,2,4-triazin-3(2H)-one

¹H-NMR (400MHz, CDCl₃) ; δ (ppm) 5.19 (s, 2H), 7.28-7.35 (m, 3H), 7.43 (td, J-7.6 Hz, 1.2 Hz, 1H), 7.47 (dd, J-5.2 Hz, 1.6 Hz, 1H), 7.66-7.79 (m, 4H), 8.19 (dt, J=8.0 Hz, 1.0 Hz, 1H), 8.59-8.61 (m, 1H).

### Example 102

### 2-(2-Cyanophenyl)-6-(2-pyridyl)-4-(2-pyridyl)-4,5-dihydro-1,2,4-triazin-3(2H)-one

¹H-NMR (400MHz, CDCl₃) ; δ (ppm) 5.47 (s, 2H), 7.10 (ddd, J=7.0 Hz, 4.6 Hz, 1.0 Hz, 1H), 7.30-7.34 (m, 1H), 7.38-7.52 (m, 2H), 7.64-7.79 (m, 4H), 7.99-8.01 (m, 1H), 8.18 (dd, J=8.0 Hz, 0.8 Hz, 1H), 8. 47-8.49 (m, 1H), 8.64-8.66 (m, 1H).
ESI-Mass; 355 [M⁺+H]

### Example 103

### 2-(2-Cyanophenyl)-6-(2-pyridyl)-4-(3-pyridyl)-4,5-dihydro-1,2,4-triazin-3(2H)-one

¹H-NMR (400MHz, CDCl₃); δ (ppm) 5.22 (s, 2H), 7.32-7.38 (m, 2H), 7.44 (td, J=7.6 Hz, 1.2 Hz, 1H), 7.70 (td, J-7.4 Hz, 1.6 Hz, 1H), 7.74-7.80 (m, 3H), 7.88 (ddd, J=8.4 Hz, 2.8 Hz, 1.2 Hz, 1H), 8.19 (d, J=8.0 Hz, 1H), 8.50-8.53 (m, 1H), 8.59 (ddd, J=4.8 Hz, 1.6 Hz, 0.8 Hz, 1H), 8. 78-8.82 (m, 1H).
ESI-Mass; 355 [M⁺+H]

The title compounds of Examples 104 to 111 were synthesized according to the method described in the above-mentioned Example 88.

### Example 104

### 4-(2-Cyanophenyl)-2-phenyl-6-(2-pyridyl)-4,5-dihydro-1,2,4-triazin-3(2H)-one

¹H-NMR (400MHz, CDCl₃); δ (ppm) 5.15 (s, 2H), 7.21-7.33 (m, 2H), 7.36-7.46 (m, 3H), 7.52-7.57 (m, 1H), 7.65-7.79 (m, 5H), 8.18 (d, J=8.4 Hz, 1H), 8.54-8.56 (m, 1H).
ESI -Mass ; 354 - [M⁺+H]

### Example 105

### 2-phenyl-6-(2-pyridyl)-4-(thiophen-3-yl)-4,5-dihydro-1,2,4-triazin-3(2H)-one

¹H-NMR (400MHz, CDCl₃) ; δ (ppm) 5.14 (s, 2H), 7.24-7.28 (m, 1H), 7.28-7.34 (m, 3H), 7.42-7.47 (m, 2H), 7.50 (dd, J=5.2 Hz, 1.2 Hz, 1H), 7.52-7.65 (m, 2H), 7.76 (td, J=7.8 H,z 2.0 Hz, 1H), 8.16 (d, J=8.0 Hz, 1H), 8.59-8.61 (m, 1H).
ESI-Mass; 335 [M⁺+H]

### Example 106

### 2-(2-Bromophenyl)-6-(2-pyridyl)-4-(thiophen-3-yl)-4,5-dihydro-1,2,4-triazin-3(2H)-one

¹H-NMR (400MHz, CDCl₃); δ (ppm) 5.18 (brs, 2H), 7.24-7.32 (m, 4H), 7.45 (t, J=7.6 Hz, 1H), 7.52-7.54 (m, 1H), 7.59-7.61 (m, 1H), 7.69-7.74 (m, 2H), 8.08 (dd, J=7.8 Hz, 1.0 Hz, 1H), 8.59-8.61 (m, 1H).
ESI-Mass; 415 [M⁺+H]

### Example 107

### 4-(2,4-Dimethoxyphenyl)-2-phenyl-6-(2-pyridyl)-4,5-dihydro-1,2,4-triazin-3(2H)-one

¹H-NMR (400MHz, CDCl₃); δ (ppm) 3. 82 (s, 3H), 3.83 (s, 3H), 4.96 (s, 2H), 6.49 (dd, J=8.4 Hz, 2.8 Hz, 1H), 6.53 (d, J=2.8 Hz, 1H), 7.20-7.23 (m, 3H), 7.36-7.42 (m, 2H), 7.66-7.67 (m, 2H), 7.75 (td, J=8.0 Hz, 1.6 Hz, 1H), 8.18 (d, J=8.4 Hz, 1H), 8.54 (d, J-4.8 Hz, 1H).
ESI-Mass; 389 [M⁺+H]

### Example 108

### 2-(2-Bromophenyl)-6-(2-methoxyphenyl)-4-(thiophen-3-yl)-4,5-dihydro-1,2,4-triazin-3(2H)-one

¹H-NMR (400MHz, CDCl₃); δ (ppm) 3.92 (s, 3H), 4.89 (s, 2H), 6.95 (d, J=8.4 Hz, 1H), 7.10 (t, J=7.6 Hz, 1H), 7.14-7.16 (m, 1H), 7.21-7.29 (m, 3H), 7.38-7.62 (m, 3H), 7.62-7.64 (m, 1H), 7.67-7.70 (m, 1H) .

### Example 109

### 2-Phenyl-6-(2-pyridyl)-4-(3-pyridyl)-4,5-dihydro-1,2,4-triazin-3(2H)-one

²H-NMR (400MHz, CDCl₃) ;δ (ppm) 5.16 (s, 2H), 7.27-7.36 (m, 3H), 7.45 (t, J=7.8 Hz, 2H), 7.63-7.66 (m, 2H), 7.77 (td, J=8.0 Hz, 1.8 Hz, 1H), 7.86 (ddd, J=8.2 Hz, 2.8 Hz, 1.6 Hz, 1H), 8.19 (dt, J=8.0 Hz, 1.0 Hz, 1H), 8.48 (dd, J=4.8 Hz, 1.6 Hz, 1H), 8.58 (ddd, J=4.8 Hz, 1.8 Hz, 0.8 Hz, 1H), 8.78 (d, J=2.0 Hz, 1H).
ESI-Mass; 330 [M⁺+H]

### Example 110

### 2-(2-Bromophenyl)-6-(2-pyridyl)-4-(3-pyridyl)-4,5-dihydro-1,2,4-triazin-3(2H)-one

¹H-NMR (400MHz, CDCl₃); δ (ppm) 5.20 (brs, 2H), 7.26-7.35 (m, 2H), 7.43-7.48 (m, 2H), 7.60-7.76 (m, 3H), 7.89 (ddd, J=7.8Hz, 2.8 Hz, 1.4 Hz, 1H), 8.10 (d, J=8.0 Hz, 1H), 8.48 (dd, J=4.8 Hz, 1.6 Hz, 1H), 8.59 (d, J=4.8 Hz, 1H), 8.80 (d, J=2.4 Hz, 1H).
ESI-Mass; 410 [M⁺+H]

### Example 111

### 2-(2-Bromophenyl)-4-(2-cyanophenyl)-6-(2-pyridyl)-4,5-dihydro-1,2,4-triazin-3(2H)-one

¹H-NMR (400MHz, CDCl₃); δ (ppm) 5.19 (s, 2H), 7.24-7.32 (m, 2H), 7.41 (dd, J=7.6 Hz, 1.2 Hz, 1H), 7.45 (dd, J=7.6 Hz, 1.2 Hz, 1H), 7.57 (dd, J=8.0 Hz, 0.8 Hz, 1H), 7.64-7.75 (m, 5H), 8.11 (d, J=8.0 Hz, 1H), 8.55 (ddd, J=5.0 Hz, 1.8 Hz, 0.8 Hz, 1H).

The title compounds of Examples 112 to 117 were synthesized according to the method described in the above-mentioned Example 89.

### Example 112

### 2-(2-Bromophenyl)-4,6-diphenyl-4,5-dihydro-1,2,4-triazin-3(2H)-one

¹H-NMR (400MHz, CDCl₃); δ (ppm) 4.92 (s, 2H), 7.22-7.27 (m, 2H), 7.39-7.47 (m, 8H), 7.62-7.65 (m, 1H), 7.67-7.70 (m, 1H), 7.72-7.75 (m, 2H).

### Example 113

### 4-(2-Bromophenyl)-2,6-diphenyl-4,5-dihydro-1,2,4-triazin-3(2H)-one

¹H-NMR (400MHz, CDCl₃) δ (ppm) 4.67 (d, J=15.4Hz, 1H) , 4.94 (d, J=15.4 Hz, 1H) ; 7.21-7.31 (m, 3H), 7.36-7.46 (m, 6H), 7.49 (dd, J=8.0Hz_{.} 1.6Hz, 1H), 7.67 -7.71 (m, 2H), 7.73-7.77 (m, 2H).
ESI-Mass; 406 [M⁺+H]

### Example 114

### 2-(2-Bromophenyl)-4-(2-bromophenyl)-6-phenyl-4,5-dihydro-1,2,4-triazin-3(2H)-one

¹H-NMR (400MHz, CDCl₃); δ (ppm) 4.74 (d, J=15.6Hz, 1H), 5.44 (d, J=15.6Hz, 1H), 6.78-6.82 (m, 1H), 6.95-6.98 (m, 1H), 7.12-7.30 (m, 3H), 7.38-7.53 (m, 4H) , 7.59-7.74 (m, 3H) , 8.54-8.58 (m, 1H).
ESI-Mass; 486 [M⁺+H]

### Example 115

### 4-(2-Bromophenyl)-6-(2-methoxyphenyl)-2-phenyl-4,5-dihydro-1,2,4-triazin-3(2H)-one

¹H-NMR (400MHz, CDCl₃) ; δ (ppm) 3.82 (s, 3H) , 4.55-4.82 (m, 2H), 6.88-6.98 (m, 1H), 7.04 (t, J=7.6 Hz, 1H), 7.13-7.27 (m, 3H), 7.32-7.41 (m, 3H), 7.45 (td, J=7.8 Hz, 1.6 Hz, 1H), 7.61-7.71 (m, 3H), 7.76 (dd, J=7.6 Hz, 1.6 Hz, 1H).
ESI-Mass; 436 [M⁺+H]

### Example 116

### 2-(2-Bromophenyl)-4-(2,5-dimethoxyphenyl)-6-(2-methoxyphenyl)-4,5-dihydro-1,2,4-triazin-3(2H)-one

¹H-NMR (400MHz, CDCl₃) ; δ (ppm) 3.75 (s, 6H), 3.82 (s, 3H), 4.70 (s, 2H), 6.80 (dd, J=9.0 Hz, 3.0 Hz, 1H), 6.87 (d, J=8.8 Hz, 1H), 6.89 (d, J=8.4 Hz, 1H), 6.96 (d, J=2.8 Hz, 1H), 7.00 (td, J=7.4 Hz, 0.4 Hz, 1H), 7.17-7.20 (m, 1H), 7.35-7.40 (m, 2H), 7.64 (dd, J=4.8 Hz, 1.6 Hz, 1H), 7.66 (dd, J=4.8 Hz, 1.6 Hz, 1H), 7.73 (dd, J=7.6 Hz, 1.6 Hz, 1H).

### Example 117

### 4-(2,5-Dimethoxyphenyl)-6-(2-methoxyphenyl)-2-phenyl-4,5-dihydro-1,2,4-triazin-3(2H)-one

¹H-NMR (400MHz, CDCl₃) ; δ (ppm) 3.74 (s, 3H), 3.76 (s, 3H) , 3.81 (s, 3H), 4.66 (s, 2H), 6.83 (dd, J= 9.0Hz, 3.0Hz, 1H), 6.86-6.94 (m, 3H), 7.03 (t, J=7.6 Hz, 1H), 7.17 (t, J=7.4Hz, 1H), 7.32-7.41 (m, 3H), 7.67-7.71 (m, 2H), 7.75 (dd, J=7.6 Hz, 1.6 Hz, 1H).
ESI-Mass; 418 [M⁺+H]

### Example 118

### 2-(2-Bromophenyl)-6-(2-pyridyl)-4-(2-pyridyl)-4,5-dihydro-1,2,4-triazin-3(2H)-one

The title compound was synthesized according to the method described in Example 92 above.
¹H-NMR (400MHz, CDCl₃) ; δ (ppm) 5.16 (m, 1H) 5.25-5.51 (m, 1H) , 7.06-7.09 (m, 1H), 7.27-7.32 (m, 1H), 7.37-7.52 (m, 2H), 7.59-7.74 (m, 4H), 8.03 (d, J=8.4 Hz, 1H), 8.08-8.11 (m, 1H), 8.46-8.49 (m, 1H), 8.63-8.66 (m, 1H).
ESI-Mass; 408 [M⁺+H]

### Example 119

### 2-Phenyl-4-phenyl-6- (2-pyrimidinyl) -4.5-dihydro-1,2,4-triazin-3(2H)-one

### 119-1) N-Methoxycarbonyl-N-phenylglycine

N-Phenylglycine (7.2 g) was dissolved in t-butyl methyl ether (120 ml), and 1 N aqueous sodium hydroxide (105 ml) was added thereto. The mixture was cooled to 0°C, and methyl chlorocarbonate (6 ml) was added dropwise thereto under vigorous stirring and then stirred at room temperature overnight. The organic layer was removed, and an aqueous saturated sodium dihydrogen phosphate solution was added to the aqueous layer, followed by extracting with ethyl acetate. The organic layer was washed with water and dried over anhydrous sodium sulfate. After the drying agent was filtered off, the filtrate was evaporated. The residue was purified by silica gel column chromatography (hexane-ethyl acetate system), to give the title compound (9.1 g, 92%) as colorless crystals. ¹H-NMR (400MHz, CDCl₃); δ (ppm) 3.27 (brs, 3H), 4.39 (m, 2H), 7.24-7.39 (m, 5H).

### 119-2) N-Methoxycarbonyl-N-phenylamino-2-ethanol

N-Methoxycarbonyl-N-phenylglycine (395 mg) was dissolved in tetrahydrofuran anhydride (50 ml), cooled to 0°C, and 1.0 M borane-tetrahydrofuran complex in tetrahydrofuran (2.4 ml) was added dropwise in a nitrogen atmosphere. After stirring at 0°C for 2 hours, 1.0 M borane-tetrahydrofuran complex in tetrahydrofuran (2. 4 ml) was further added dropwise. This procedure was repeated twice. After stirring at 0°C for 4 hours, methanol (40 ml) was added dropwise, and after stirring at the same temperature for 2 minutes, the mixture was evaporated. Ethyl acetate was added thereto, and the reaction solution was washed with an aqueous saturated sodium bicarbonate solution and dried over anhydrous sodium sulfate. After the drying agent was filtered off, the filtrate was evaporated, to give the title compound as a colorless oil (420 mg, quantitative).
¹H-NMR (400MHz, CDCl₃); δ (ppm) 3.68 (brs, 3H), 3.76 (t, 2H), 3.83 (dt, 2H), 7.15-7.39 (m, 5H).

### 119-3) N-Methoxycarbonyl-N-phenyl-aminoacetaldehyde

N-Methoxycarbonyl-N-phenylamino-2-ethanol (420 mg) was dissolved in dimethyl sulfoxide (13 ml), and triethylamine (5 ml) was added thereto, followed by cooling to 0°C. Sulfur trioxide (500 mg) was added little by little thereto under stirring vigorously at the same temperature, followed by stirring at room temperature overnight. Water was added thereto, and the reaction solution was extracted with ethyl acetate. The organic layer was washed with an aqueous saturated ammonium chloride solution and an aqueous saturated sodium hydroxide solution, and dried over anhydrous sodium sulfate. After the drying agent was filtered off, the filtrate was evaporated. The residue was purified by silica gel column chromatography (hexane-ethyl acetate system), to give the title compound as a brown oil (191 mg, 46%).
¹H-NMR (400MHz, CDCl₃); δ (ppm) 3.72 (s, 3H), 4.40 (s, 2H), 7.24-7.39 (m, 5H), 9.70 (s, 1H).

### 119-4) N-Phenyl-2-(N"-phenyl-N"-methoxycarbonylamino)ethanehydrazonoyl bromide

N-Methoxycarbonyl-N-phenylaminoacetaldehyde (500 mg) was dissolved in ethanol (20 ml) , and phenylhydrazine (280 mg) was added thereto, and the mixture was stirred overnight in a nitrogen atmosphere. The reaction solution was evaporated, and from N-methoxycarbonyl-N-phenylaminoacetaldehyde phenylhydrazone obtained as the residue, the title compound (158 mg) was obtained as a reddish brown oil, according to the method in Tetrahedron Vol. 52, pp. 661-668, 1996.
¹H-NMR (400MHz, CDCl₃) ; δ (ppm) 3.75 (s, 3H) , 4.79 (s, 2H), 6.85 (d, 2H), 7.22-7.38 (m, 8H), 7.68 (s, 1H).

### 119-5) (Z)-2'-(N-Phenyl-N-methoxycarbonylamino)-2-acetylpyrimidine phenylhydrazone

N-Phenyl-2-(N"-phenyl-N"-methoxycarbonylamino)ethane hydrazonoyl bromide (158 mg) was dissolved in xylene (10 ml), and 2-trinormalbutyl stannyl pyrimidine (241 mg), tetrakis (triphenyl phosphine) palladium (25 mg) and copper iodide (5 mg) were added thereto, followed by stirring at 110°C for 4 hours in a nitrogen atmosphere. After cooling to room temperature, the mixture was extracted with ethyl acetate. The organic layer was washed with water and dried over anhydrous sodium sulfate. After the drying agent was filtered off, the filtrate was evaporated. The residue was purified by silica gel column chromatography (hexane-ethyl acetate system), to give the title compound (37 mg, 23%) as brown crystals.
¹H-NMR (400MHz, CDCl₃) ; δ (ppm) 3.73 (s, 3H), 5.10 (s, 2H). 7.01-7.11 (m, 2H), 7.15-7.20 (m, 2H), 7.25-7.30 (m, 3H), 7.35-7.39 (m, 4H), 8.81 (d, 2H), 13.30 (s, 1H).

### 119-6) (E)-2'-(N-phenyl-N-methoxycarbonylamino)-2-acetylpyridine phenylhydrazone

(Z)-2'-(N-Phenyl-N-methoxycarbonylamino)-2-acetylpyrimidine phenylhydrazone (5 mg) was dissolved in 4 N hydrochloric acid-ethyl acetate (0.5 ml) at 0°C. After stirring at room temperature for 2 minutes, the reaction solution was neutralized by adding an aqueous saturated sodium bicarbonate solution. The reaction solution was extracted with ethyl acetate, and the organic layer was washed with water and dried over anhydrous sodium sulfate. After the drying agent was filtered off, the filtrate was evaporated. The residue was purified by silica gel column chromatography (hexane-ethyl acetate system), to give the title compound (5 mg) as brown crystals.
¹H-NMR- (400MHz, CDCl₃) ; δ (ppm) 3.74 (s, 3H) , 5.28 (s, 2H), 6.95 (t, 1H), 7.06-7.08 (m, 2H) , 7.16-7.21 (m, 3H) , 7.26-7.30 (m, 3H), 7.41-7.44(m, 2H), 8.49 (d, 2H), 10.45 (s, 1H).

### 119-7) 2-Phenyl-4-phenyl-6-(2-pyrimidinyl)-4,5-dihydro-1,2,4-triazin-3(2H)-one

(E)-2'-(N-Phenyl-N-methoxycarbonylamino)-2-acetylpyrimidine phenylhydrazone (5 mg) was dissolved in ethanol (3 ml), and sodium ethylate (1.1 mg) was added thereto at 0°C, followed by stirring at room temperature for 1 hour. After heating at 110°C for 1 minute, it was cooled to room temperature. An aqueous saturated ammonium chloride solution and water were added thereto, and the reaction solution was extracted with ethyl acetate. The organic layer was washed with water and dried over anhydrous sodium sulfate. After the drying agent was filtered off, the filtrate was evaporated. The residue was purified by silica gel column chromatography (hexane-ethyl acetate system), to give the title compound (2 mg) as colorless crystals.
¹H-NMR (400MHz, CDCl₃) ; δ (ppm) 5.12(s, 2H), 7.25-7.30 (m, 2H), 7.32 (t, 1H), 7.40-7.47 (m, 4H), 7.51-7.57 (m, 4H), 8.85 (d, 2H).

The title compounds of Examples 120 to 126 were synthesized according to the above-mentioned Example 85.

### Example 120

### 2-(2-Bromophenyl)-4-(4-biphenyl)-6-(2-pyridyl)-4,5-dihydro-1,2,4-triazin-3(2H)-one

¹H-NMR (400MHz, CDCl₃); δ (ppm) 5.20 (brs, 2H), 7.19-7.75 (m, 15H), 8.11 (d, 1H), 8.57-8.59 (m, 1H).

### Example 121

### 2-(2-Bromophenyl)-4-(3-nitrophenyl)-6-(2-pyridyl)-4,5-dihydro-1,2,4-triazin-3(2H)-one

¹H-NMR (400MHz, CDCl₃); δ (ppm) 5.23 (brs, 2H), 7.22-7.35 (m,2H), 7.47 (td, 1H), 7.55 (t, 1H), 7.60 (dd, 1H), 7.70-7.78 (m, 2H), 7.91 (dd, 1H), 8.08-8.12 (m, 2H), 8.38 (t, 1H), 8.60 (m, 1H). ESI-Mass; 452 [M⁺+H]

### Example 122

### 2-(2-Bromophenyl)-4-(4-fluorophenyl)-6-(2-pyridyl)-4,5-dihydro-1,2,4-triazin-3(2H)-one.

¹H-NMR (400MHz, CDCl₃); δ (ppm) 5.12 (brs, 2H), 7.06-7.11 (m, 2H), 7.24-7.29 (m, 1H), 7.30 (ddd, 1H), 7.41-7.49 (m, 3H), 7.60 (dd, 1H), 7.68-7.75 (m, 2H), 8.10 (d, 1H), 8.56-8.58 (m, 1H).

### Example 123

### 2-(2-Bromophenyl)-4-(3-formylphenyl)-6-(2-pyridyl)-4,5-dihydro-1,2,4-triazin-3(2H)-one

²H-NMR (400MHz, CDCl₃); δ (ppm) 5.20 (brs, 2H), 7.18-7.34 (m, 2H), 7.46 (td, 1H), 7.57 (t, 1H), 7.63 (dd, 1H), 7.56-7.70 (m, 3H), 7.83 (ddd, 1H) , 8.02 (t, 1H) , 8.11 (dt, 1H) , 8.58-8.59 (m, 1H) , 10.03 (s, 1H) .

### Example 124

### 2-(2-Bromophenyl)-4-(3-tolyl)-6-(2-pyridyl)-4,5-dihydro-1,2,4-triazin-3(2H)-one

¹H-NMR (400MHz, CDCl₃) δ (ppm) 2.37 (s, 3H), 5.13 (brs, 2H), 7.04 (t, 1H), 7.23-7.33 (m, 5H), 7.44 (td, 1H), 7.62 (dd, 1H), 7.68-7.70(m, 1H), 7.73 (dd, 1H) , 8. 10 (d, 1H), 8.56-8.58 (m, 1H).

### Example 125

### 2-(2-Bromophenyl)-4-(4-thiomethoxyphenyl)-6-(2-pyridyl)-4,5-dihydro-1,2,4-triazin-3(2H)-one

²H-NMR (400MHz, CDCl₃) ; δ (ppm) 2.50 (s, 3H), 5.13 (brs, 2H), 7.26-7.32 (m, 3H), 7.42-7.47 (m, 2H), 7.53-7.57 (m, 1H), 7.63 (dd, 1H), 7.70-7.76 (m, 3H), 8.11 (d, 1H), 8.57-8.60 (m, 1H).

### Example 126

### 2-(2-Bromophenyl)-4-(2-chloropyridin-5-yl)-6-(2-pyridyl)-4,5-dihydro-1,2,4-triazin-3(2H)-one

¹H-NMR (400MHz, CDCl₃); δ (ppm) 5.18 (brs, 2H), 6.99-8.11 (m, 10H), 8.56-8.60 (m, 1H).

### Example 127

### 2-(2-Cyanophenyl)-4-(3-nitrophenyl)-6-(2-pyridyl)-4,5-dihydro-1,2,4-triazin-3(2H)-one

According to the above-mentioned Example 87, the title compound was synthesized from 2-(2-bromophenyl)-4-(3-nitrophenyl)-6-(2-pyridyl)-4,5-dihydro-1,2,4-triazin-3(2H)-one.
²H-NMR (400MHz, CDCl₃) ; δ (ppm) 5.24 (brs, 2H), 7.36 (ddd, 1H), 7.46 (td, 1H), 7.59 (t, 1H), 7.69-7.81 (m, 4H), 7.90 (ddd, 1H), 8.12 (ddd, 1H), 8.20 (dt, 1H), 8.38 (t, 1H), 8.60 (ddd, 1H).
ESI-Mass; 399 [M⁺+H]

### Example 128

### 2-(2-Cyanophenyl)-4-(3-aminophenyl)-6-(2-pyridyl)-4,5-dihydro-1,2,4-triazin-3(2H)-one

2-(2-Cyanophenyl)-4-(3-nitrophenyl)-6-(2-pyridyl)-4,5-dihydro-1,2,4-triazin-3(2*H*)-one (15 mg) was dissolved in methanol (3 ml), 10% palladium-carbon powder (hydrate) (21 mg) was added thereto, and the mixture was stirred for 4 hours at room temperature in a hydrogen atmosphere. The palladium-carbon powder was filtered off, and the filtrate was evaporated. The resulting residue was purified by silica gel column chromatography (hexane-ethyl acetate system), to give the title compound (13 mg) as colorless crystals.
¹H-NMR (400MHz, CDCl₃) δ (ppm) 3.72 (brs, 2H), 5.13 (s, 2H), 6.58 (ddd, 1H), 6.82-6.85 (m, 2H) , 7.19 (t, 1H), 7.32 (ddd, 1H) , 7.40 (td, 1H), 7.67 (td, 1H), 7.73-7.78 (m, 3H), 8.20 (dt, 1H), 8.58 (ddd, 1H).
ESI-Mass; 369 [M⁺+H]

### Example 129

### 2- (2-Chlorophenyl) -4-phenyl-6- (2-pyrimidinyl) -4, 5-dihydro-1,2,4-triazin-3(2H)-one.

The title compound was synthesized according to the above-mentioned Example 119.
¹H-NMR (400MHz, CDCl₃); δ (ppm) 5.8 (brs, 2H), 7.25 (tt, 1H), 7.28-7.44 (m, 5H), 7.47-7.51 (m, 3H), 7.65 (dd, 1H), 8.84 (d, 2H).
ESI-Mass; 364 [M⁺+H]

The chemical structures of the compounds of the above-mentioned Examples are shown below. Each symbol in the table corresponds to the symbol for each substituent group in the structural formula shown in the title of the table. Each substituent group is bound directly via a single bond having a substituent-free terminal, as shown in the structural formula in the table. "Me" in the table means a methyl group.

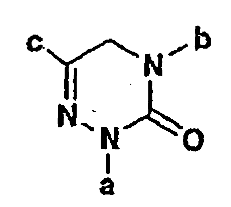

| Example | a | b | c |
|---|---|---|---|
| 85 | 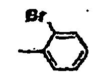 | 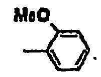 | 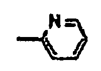 |
| 86 | 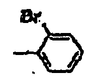 | 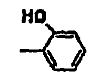 | 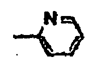 |
| 87 | 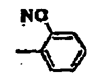 | 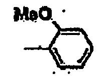 | 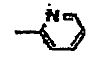 |
| 88 | 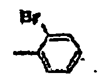 | 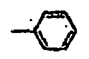 | 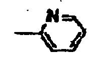 |
| 89 | 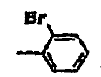 | 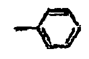 | 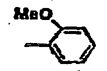 |
| 90 | 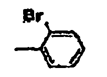 | 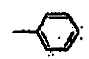 | 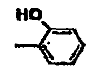 |
| 91 | 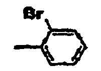 | 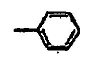 | 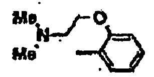 |
| 92 | 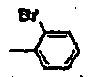 | 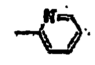 | 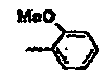 |
| 93 | 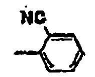 | 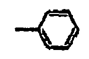 | 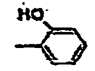 |
| 94 | 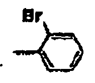 | 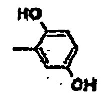 | 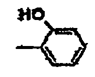 |
| 95 | 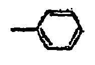 | 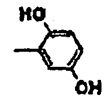 | 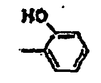 |
| 96 | 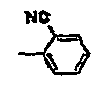 | 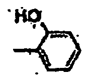 | 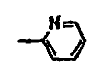 |
| 97 | 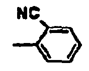 | 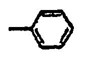 | 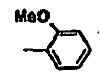 |
| 98 | 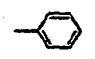 | 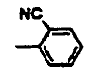 | 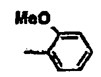 |
| 99 | 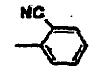 | 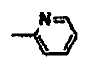 | 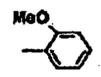 |
| 100 | 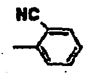 | 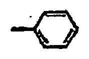 | 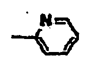 |
| 101 | 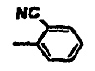 | 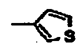 | 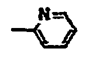 |
| 102 | 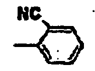 | 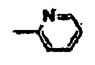 | 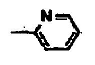 |
| 103 | 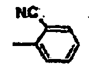 | 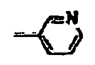 | 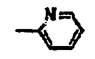 |
| 104 | 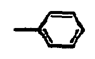 | 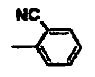 | 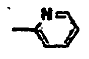 |
| 105 | 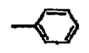 | 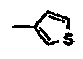 | 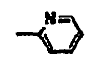 |
| 106 | 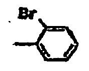 | 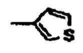 | 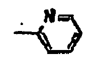 |
| 107 | 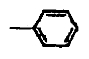 | 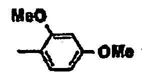 | 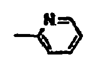 |
| 108 | 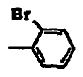 | 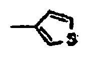 | 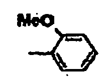 |
| 109 | 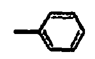 | 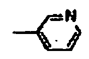 | 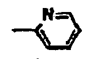 |
| 110 | 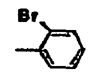 | 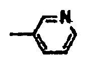 | 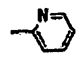 |
| 111 | 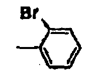 | 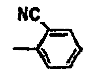 | 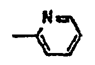 |
| 112 | 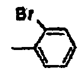 | 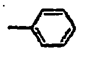 | 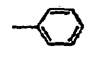 |
| 113 | 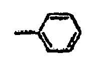 | 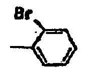 | 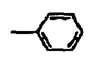 |
| 114 | 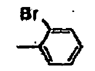 | 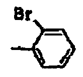 | 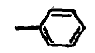 |
| 115 | 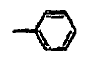 | 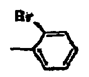 | 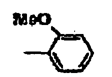 |
| 116 | 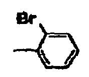 | 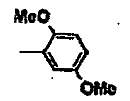 | 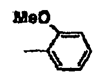 |
| 117 | 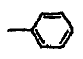 | 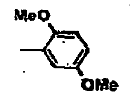 | 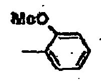 |
| 118 | 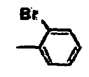 | 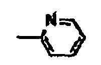 | 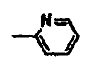 |
| 119 | 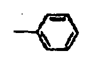 | 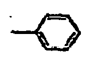 | 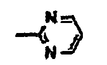 |
| 120 | 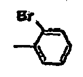 | 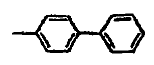 | 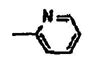 |
| 121 | 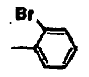 | 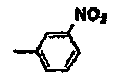 | 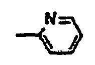 |
| 122 | 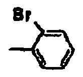 | 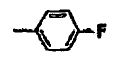 | 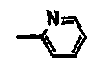 |
| 123 | 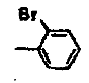 | 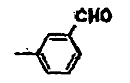 | 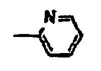 |
| 124 | 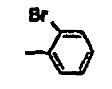 | 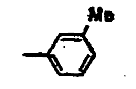 | 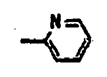 |
| 125 | 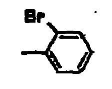 | 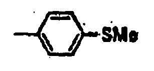 | 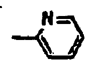 |
| 126 | 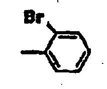 | 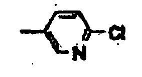 | 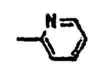 |
| 127 | 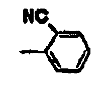 | 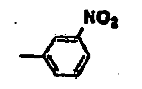 | 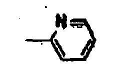 |
| 128 | 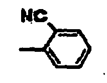 | 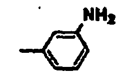 | 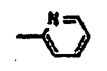 |
| 129 | 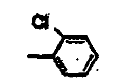 | 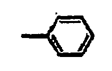 | 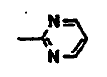 |

### Test Example 1

### Suppressing Action to Calcium Influx into Nerve Cells Induced by AMPA

The suppressing action of the compounds of the present invention to calcium influx into nerve cells induced by AMPA was investigated using the primary culture system of nerve cells of cerebral cortex of embryo of rat.

### Culturing Conditions:

Cerebral cortex was cut out from the brain of rat of gestational 18 days and treated with trypsin and DNase to disperse the cells. The cells were flown by MEM containing 10% of serum, sown in a culture bottle and astrocytes were proliferated. The astrocytes were re-dispersed by trypsin and sown in a 96-well plate. After incubation for one week, it was confirmed that the astrocytes covered all over the bottom and then the nerve cells of cerebral cortex which was dispersed by the above method were sown thereupon. After incubation for 24 hours, the medium was changed, the incubation was carried out for one week and, after that, the medium was changed to that containing 1µM of MK-801 . Nerve cells which were incubated for not shorter than 8 to 10 days were used.

### Test Method:

Calcium influx into the cells was measured using Fura2-AM which was a calcium-sensitive fluorescent dye. It was treated in a medium containing Fura2-AM for 1 hour, incorporated into the cells, exchanged to a Tyrode solution containing 1µM MK-801 and stimulation was carried out using 2µM AMPA. Change in the amount of calcium flown into the cells were measured as the change in the fluorescent intensity at the exciting wave length of 340/380 nm. Effect of the test compound was evaluated using the reaction resulted in the AMPA added to a Tyrode solution containing no compound as a control. As the control compound, GYKI52466 (Le Peillet, et al., Brain Res., 571, 115, 1992) was used.

### Results:

The compound (VIII) according to the present invention significantly inhibited the calcium influx into nerve cells induced by AMPA (Table 1). The IC₅₀ of GYKI52466 was 9.02 µM.

**Table 1**

| Ex. No. | IC₅₀(µM) |
|---|---|
| 85 | 0.2 |
| 86 | 0.1 |
| 87 | 0.05 |
| 88 | 0.1 |
| 89 | 5.0 |
| 90 | 0.9 |
| 91 | 0.3 |
| 92 | 0.1 |
| 93 | 0.03 |
| 94 | 0.9 |
| 95 | 0.05 |
| 96 | 0.6 |
| 97 | 0.7 |
| 98 | 0.4 |
| 99 | 0.07 |
| 100 | 0.05 |
| 101 | 0.1 |
| 102 | 0.1 |
| 103 | 0.1 |
| 104 | 0.5 |
| 105 | 0.2 |
| 106 | 0.1 |
| 107 | 0.3 |
| 108 | 4.0 |
| 109 | 0.3 |
| 110 | 0.1 |
| 111 | 0.4 |
| 112 | 0.3 |
| 113 | 7.1 |
| 115 | 7.2 |
| 118 | 0.03 |
| 119 | 4.3 |
| 121 | 0.4 |
| 1 22 | 0.2 |
| 123 | 0.3 |
| 124 | 0.2 |
| 125 | 0.6 |
| 127 | 0.4 |
| 128 | 0.1 |
| 129 | 3.0 |

### Test Example 2

### Anticonvulsant Action Induced by AMPA

A test compound was suspended in a 0.5% methyl cellulose solution or in sesame oil and was orally administered (25 mg/kg) to male mice of ddy strain. After 30 minutes or 1 hour from the oral administration, AMPA was continuously injected (2 nmole/5µl/minute/mouse) into lateral ventricle to induce the convulsions. The effect was judged by a time-extending action until the convulsion takes place by a continuous injection of AMPA.

### Results:

The compound (VIII) according to the present invention showed an excellent anticonvulsant action. For example, the compounds of Examples 88, 97, 100, 102 and 103 significantly inhibited the convulsion induced by AMPA.

### Test example 3

### Middle cerebral artery occlusion model

The usefulness of the compound related to the present invention in the remedy of cerebral vascular accident acute stage was confirmed by the test below. Namely, the cerebral bloodstream of middle cerebral artery was blocked by inserting a nylon suture thread of 4-0 specification whose edge was crashed with flame, by 17mm from the branch of internal carotid artery, through internal carotid artery from the external carotid artery of a male Sprange Dawley rat, and cerebral infarction was prepared (Zea Longa et al., Stroke 20: 84 - 91, 1989). The size of the cerebral infarction was evaluated by preparing the intersection slice of brain having a thickness of 2mm and measuring the area of a portion which was not stained by TTC staining. The effect of the tested substance was carried out in this model by comparing the infarction size between a group treated with a solvent and a group treated with the tested substance.

As a result, the compound (VIII) according to the present invention revealed an excellent effect as the therapeutic agent of cerebral vascular accident acute stage.

### Test Example 4

### Antimethamphetamine effect

(S)-(+)-N,α-dimethylphenetylamine (hereinafter, referred to as "methamphetamine") was dosed intraperitoneal injection to a rat or mouse to which the tested compound was dosed, and a quantity of active movement was measured using an active movement measuring apparatus (SCANET SV-10; manufactured by TOYO Sangyo Co., Ltd.). The activity as the therapeutic agent of schizophrenia was evaluated using the hyperdynamic effect control of movement caused by methamphetamine as an index (K.E.Vanover, Psychopharmacology 136: 123-131, 1998). The effect of the tested substance was confirmed by the control effect of a quantity of movement accentuation in comparison with the group dosed with a solvent.

As a result, the compound (VIII) according to the present invention revealed an excellent anti-methamphetamine effect.

### Test Example 5

### Intercolliculus decerebrated rigidity model

An animal model in which the myotony of limbs was provoked was prepared by electrically sectioning between the colliculus superior and the colliculus inferior of a rat. Myorelaxation effect was evaluated based on the effect of controlling the increase of muscle discharge which is generated when the posterior limbs in this model are moved back and forth. The effect of the tested substance was confirmed by the changes of muscle discharge amount before dosing the tested substance and muscle discharge amount after dosing it.

The compound (VIII) according to the present invention revealed an excellent myorelaxation effect.

### Test Example 6

### Light dark test

A mouse is put in a dark box which is composed of two light and dark boxes which are linked by a tunnel, and items below were recorded concerning the behavior of the mouse for 5 minutes after that..
1. A time for remaining in the light and dark boxes.
2. Times by which the mouse went and came back between the light box and the dark box.
3. Times by which the mouse went until the entrance of the light box.

The antianxiety effect of the tested compound was detected as the elongation of the time remaining in the light box, the increase of times by which the mouse went and came back between the light box and the dark box, and the increase of times by which the mouse went until the entrance of the light box, for the group dosed with a solvent (Hascoet M., Bourin M., Pharm. Biochem. Behav. 60: 645-653, 1998).

The compound (VIII) according to the present invention showed an excellent antianxiety effect.

### Test Example 7

### 6-OHDA-induced hemi-parkinsonian rat model of Parkinson's disease

10mg/kg of L-Dihydroxyphenylalanine (L-DOPA) (twice per day) was intraperitaneously dosed every day to the rat whose one side of nigra was destroyed by injecting 6-hydroxydopamine (6-OHDA) into medial forebrain bundle. The increase of contralateral rotation to the intact side was provoked (C.Marin et al, Synapse 36 (4) :267-274, 2000). After the solvent or the tested compound was dosed to the rat, influence on the provoked rotation was studied.

The compound (VIII) of the present invention as the test sample delayed the time until the maximum rotatory response after dosing L-DOPA, and increased the time of showing rotation which is a half or more of the maximum rotational number.

### Test Example 8

### Acetic acid writhing model

Anguishing condition under which the lower half of mice's body was twisted, its abdomen was dented and its hind legs were extended was provoked by injecting 0.6% acetic acid in saline into the abdomen of the mice. After the tested compound and the solvent were dosed, the acetic acid in saline was injected into the abdomen, and analgesic effect was evaluated by comparing the times of these abnormal actions within an observation time (5 to 15 minutes after the dose of acetic acid) which occur after the dosing (Basic Pharmacology Experiment, edited by Kazuhiko Kubota, pages 45-47, Nankoh-do).

As a result, it could be confirmed that the compound (VIII) according to the present invention controls the times of the abnormal actions significantly and has an excellent analgesic effect.

### Test Example 9

### Vomiting model induced by cisplatin

A intravenous catheter was buried in a ferret, and the rat was postoperatively recovered. Then, vomiting reaction was provoked by injecting 10mg/kg of cis-diaminedichloroplatinum (cisplatin) (A.Fink-Jensen et al., Neuroscience Letters 137: 173-177, 1992). Cisplatin (10mg/kg) was injected a ferret which was treated with the tested compound or the solvent, then the ferret was put in an observation cage, and times of the rhythmical contraction of abdomen (defined as vomiting) and times until vomiting occurs during the observation period of 240 minutes were measured.

As a result, the compound (VIII) according to the present invention extended the latent time and reduced the vomiting times significantly.

### Test example 10

### Experimental autoimmune encephalomyelitis model

Female Lewis rats (205 ± 10g) obtained from Charles River, Kent UK, were housed in pairs under environmentally controlled conditions (6:00a.m.-6:00p.m. light/dark cycle; 22-24°C; 45-55% humidity) and allowed free access to food and water. Experimental groups consisted of 9-12 animals. Rats were immunized with 20-50 µl of inoculum containing 50 µg guinea pig myelin basic protein (MBP: final concentration 2 mg/ml), emulsified in Freund's complete adjuvant (CFA; Sigma, UK) containing Mycobacterium tuberculosis H37Ra (final concentration 5.5 mg/ml; Difco Laboratories, UK). Animals were weighed and monitored daily and clinical disease scored as (0) no clinical signs; (1) flaccid tail and weight loss; (2) hind limb hypotonia with further weight loss; (3) complete hind limb paralysis; (4) paraplegia and (5) death. In addition, intermediate scores were assigned to animals which showed a loss of tonicity in the distal half of the tail (score = 0.5), paralysis of one hind limb (score = 2.5) or complete hind limb paralysis witch forelimb weakness (score = 3.5). During the period of compound administration (10-16 days post immunisation; dpi) animals were scored 15h after injection of vehicle or compound to avoid any acute effect of treatment on disease score. Compounds were dissolved/suspended in 0.5% methyl cellulose using a hand held-Polytron homogeniser (PT1200; 2 min). Rats were dosed p.o. with either methyl cellulose vehicle (2.5 ml/kg) or compound at 5, 10 and 20 mg/kg.

### Results:

The compound of the invention is improved in view of experimental autoimmune encephalomyelitis. The compound (VIII) according to the present invention showed a superior effect to the vehicle-administered group.

## Claims

1. A compound represented by the following formula, a salt thereof or a hydrate of them: 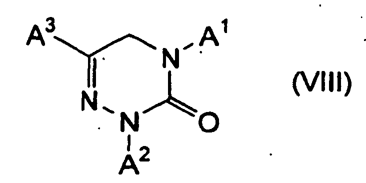 wherein A¹, A² and A³ are independent of each other and each represents a C₆₋₁₄ aromatic hydrocarbon cyclic group or a 5-to 14-membered aromatic heterocyclic group, each of which may be substituted with one or more groups selected from the group consisting of (1) a hydroxyl group, (2) a halogen atom, (3) a nitrile group, (4) a nitro group, (5) a C₁₋₆ alkyl group, C₂₋₆ alkenyl group or C₂₋₆ alkynyl group, each of which may be substituted with at least one group selected from the group consisting of a hydroxyl group, nitrile group, halogen atom, C₁₋₆ alkylamino group, di(C₁₋₆ alkyl) amino group, C₂₋₆ alkenylamino group, di (C₂₋₆ alkenyl) amino group, C₂₋₆ alkynylamino group, di(C₂₋₆ alkynyl)amino group, N-C₁₋₆ alkyl-N-C₂₋₆ alkenylamino group, N-C₁₋₆ alkyl-N-C₂₋₆ alkynylamino group, N-(C₂₋₆ alkenyl-N-C₂₋₆ alkynylamino group, aralkyloxy group, TBDMS oxy group, C₁₋₆ alkylsulfonylamino group, C₁₋₆ alkylcarbonyloxy group, C₂₋₆ alkenylcarbonyloxy group, C₂₋₆ alkynylcarbonyloxy group, N-C₁₋₆ alkylcarbamoyl group, N-C₂₋₆ alkenylcarbamoyl group and N-C₂₋₆ alkynylcarbamoyl group, (6) a C₁₋₆ alkoxy group, C₂₋₆ alkenyloxy group or C₂₋₆ alkynyloxy group, each of which may be substituted with at least one group selected from the group consisting of a C₁₋₆ alkylamino group, aralkyloxy group and hydroxyl group, (7) a C₁₋₆ alkylthio group, C₂₋₆ alkenylthio group or C₂₋₆ alkynylthio group, each of which may be substituted with at least one group selected from the group consisting of a hydroxyl group, nitrile group, halogen atom, C₁₋₆ alkylamino group, aralkyloxy group, TBDMS oxy group, C₁₋₆ alkylsulfonylamino group, C₁₋₆ alkylcarbonyloxy group and C₁₋₆ alkylcarbamoyl group, (8) a carbonyl group substituted with a group selected from the group consisting of a C₁₋₆ alkoxy group, amino group, C₁₋₆ alkylamino group, di (C₁₋₆ alkyl) amino group, C₂₋₆ alkenylamino group, di (C₂₋₆ alkenyl) amino group, C₂₋₆ alkynylamino group, di (C₂₋₆ alkynyl)amino group, N-C₁₋₆ alkyl-N-C₂₋₆ alkenylamino group, N-C₁₋₆ alkyl-N-C₂₋₆ alkynylamino group and N-C₂₋₆ alkenyl-N-C₂₋₆ alkynylamino group, (9) an amino group which may be substituted with one or two groups selected from the group consisting of a C₁₋₆ alkyl group, C₂₋₆ alkenyl group, C₂₋₆ alkynyl group, C₁₋₆ alkylsulfonyl group, C₂₋₆ alkenylsulfonyl group, C₂₋₆ alkenylsulfonyl group, C₁₋₆ alkylcarbonyl group, C₂₋₆ alkenylcarbonyl group and C₂₋₆ alkynylcarbonyl group, (10) a C₁₋₆ alkylsulfonyl group, (11) a C₂₋₆ alkenylsulfonyl group, (12) a C₂₋₆ alkynylsulfonyl group, (13) a C₁₋₆ alkylsulfinyl group, (14) a C₂₋₆ alkenylsulfinyl group, (15) a C₂₋₆ alkynylsulfinyl group, (16) a formyl group, (17) a C₃₋₈ cycloalkyl group or C₃₋₈ cycloalkenyl group, each of which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, (18) a 5-to 14-membered non-aromatic heterocyclic group which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, (19) a C₆₋₁₄ aromatic hydrocarbon cyclic group which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, and (20) a 5- to 14-membered aromatic heterocyclic group which may be substituted with at least one group selected from the group consisting of a hydroxyl group, halogen atom, nitrile group, C₁₋₆ alkyl group, C₁₋₆ alkoxy group, C₁₋₆ alkoxy-C₁₋₆ alkyl group and aralkyl group, and (21) thiol group,
provided that compounds in the following cases (1) to (3) are excluded:
(1) the case where each of A¹, A² and A³ is a phenyl group,
(2) the case where A¹ is an o,p-dimethylphenyl group; A² is an o-methylphenyl group; and A³ is a phenyl group, and
(3) the case where A¹ is an o-methylphenyl group; A² is a p-methoxyphenyl group; and A³ is a phenyl group.

2. The compound according to claim 1, a salt thereof or a hydrate of them, wherein A¹, A² and A³ are independent of each other and each represents a phenyl group, pyrrolyl group, pyridyl group, pyridazinyl group, pyrimidinyl group, pyrazinyl group, thienyl group, thiazolyl group, furyl group, naphthyl group, quinolyl group, isoquinolyl group, indolyl group, benzimidazolyl group, benzothiazolyl group, benzoxazolyl group, imidazopyridyl group or carbazolyl group, each of which may be substituted.

3. The compound according to claim 1, a salt thereof or a hydrate of them, wherein A¹, A² and A³ are independent of each other and each represents a group represented by the formula : 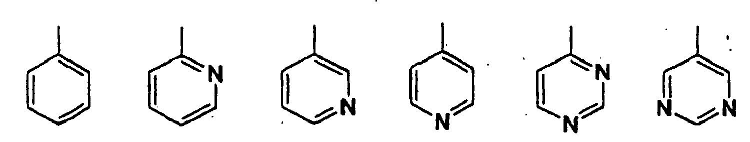 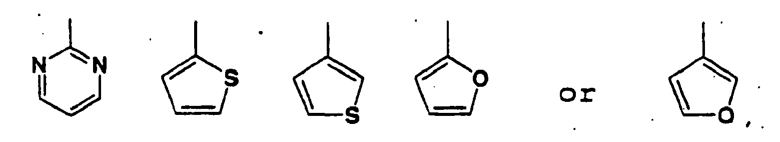 each of which may be substituted.

4. The compound according to claim 1, a salt thereof or a hydrate of them, wherein each of A¹, A² and A³ may be substituted with at least one group selected independently from the group consisting of a halogen atom, nitrile group, hydroxyl group, amino group, formyl group and nitro group.

5. The compound according to claim 1, a salt thereof or a hydrate of them, wherein the binding positions of substituents groups on A¹, A² and/or A³ are α-positions of the carbon atoms on A¹, A² and/or A³, each of which are bound directly to the triazinone ring.

6. The compound according to claim 1, a salt thereof or a hydrate of them, which is a compound selected from:
2-(2-bromophenyl)-4-(2-methoxyphenyl)-6-(2-pyridyl)-4,5-dihydro-1,2,4-triazin-3(2*H*)-one, 2-(2-bromophenyl)-4-(2-hydroxyphenyl)-6-(2-pyridyl)-4,5-dihydro-1,2,4-triazin-3(2*H*)-one, 2-(2-cyanophenyl)-4-(2-methoxyphenyl)-6-(2-pyridyl)-4,5-dihydro-1,2,4-triazin-3(2*H*)-one, 2-(2-bromophenyl)-4-phenyl-6-(2-pyridyl)-4,5-dihydro-1,2,4-triazin-3(2*H*)-one, 2-(2-bromophenyl)-6-(2-methoxyphenyl)-4-phenyl-4,5-dihydro-1,2,4-triazin-3(2*H*)-one, 2-(2-bromophenyl)-6-(2-hydroxyphenyl)-4-phenyl-4,5-dihydro-1,2,4-triazin-3(2H)-one, 2-(2-bromophenyl)-6-(2-dimethylaminoethoxyphenyl)-4-phenyl-4,5-dihydro-1,2,4-triazin-3(2*H*)-one, 2-(2-bromophenyl)-6-(2-methoxyphenyl)-4-(2-pyridyl)-4,5-dihydro-1,2,4-triazin-3(2H)-one, 2-(2-cyanophenyl)-6-(2-hydroxyphenyl)-4-phenyl-4,5-dihydro-1,2,4-triazin-3(2*H*)-one, 2-(2-bromophenyl)-4-(2,5-dihydroxyphenyl)-6-(2-hydroxyphenyl)-4,5-dihydro-1,2,4-triazin-3(2*H*)-one, 4-(2,5-dihydroxyphenyl)-6-(2-hydroxyphenyl)-2-phenyl-4,5-dihydro-1,2,4-triazin-3(2H)-one, 2-(2-cyanophenyl)-4-(2-hydroxyphenyl)-6-(2-pyridyl)-4,5-dihydro-1,2,4-triazin-3(2*H*)-one, 2-(2-cyanophenyl)-6-(2-methoxyphenyl)-4-phenyl-4,5-dihydro-1,2,4-triazin-3(2*H*)-one, 4-(2-cyanophenyl)-6-(2-methoxyphenyl)-2-phenyl-4,5-dihydro-1,2,4-triazin-3(2*H*)-one, 2-(2-cyanophenyl)-6-(2-methoxyphenyl)-4-(2-pyridyl)-4,5-dihydro-1,2,4-triazin-3(2*H*)-one, 2-(2-cyanophenyl)-4-phenyl-6-(2-pyridyl)-4,5-dihydro-1,2,4-triazin-3(2*H*)-one, 2-(2-cyanophenyl)-6-(2-pyridyl)-4-(thiophen-3-yl)-4,5-dihydro-1,2,4-triazin-3(2H)-one, 2-(2-cyanophenyl)-6-(2-pyridyl)-4-(2-pyridyl)-4,5-dihydro-1,2,4-triazin-3(2*H*)-one, 2-(2-cyanophenyl)-6-(2-pyridyl)-4-(3-pyridyl)-4,5-dihydro-1,2,4-triazin-3(2H)-one, 4-(2-cyanophenyl)-2-phenyl-6-(2-pyridyl)-4,5-dihydro-1,2,4-triazin-3(2*H*)-one, 2-phenyl-6-(2-pyridyl)-4-(thiophen-3-yl)-4,5-dihydro-1,2,4-triazin-3(2*H*)-one, 2-(2-bromophenyl)-6-(2-pyridyl)-4-(thiophen-3-yl)-4,5-dihydro-1,2,4-triazin-3(2*H*)-one, 4-(2,4-dimethoxyphenyl)-2-phenyl-6-(2-pyridyl)-4,5-dihydro-1,2,4-triazin-3(2*H*)-one, 2-(2-bromophenyl)-6-(2-methoxyphenyl)-4-(thiophen-3-yl)-4,5-dihydro-1,2,4-triazin-3(2*H*)-one, 2-phenyl-6-(2-pyridyl)-4-(3-pyridyl)-4,5-dihydro-1,2,4-triazin-3(2*H*)-one, 2-(2-bromophenyl)-6-(2-pyridyl)-4-(3-pyridyl)-4,5-dihydro-1,2,4-triazin-3(2*H*)-one, 2-(2-bromophenyl)-4-(2-cyanophenyl)-6-(2-pyridyl)-4,5-dihydro-1,2,4-triazin-3(2*H*)-one, 2-(2-bromophenyl)-4,6-diphenyl-4,5-dihydro-1,2,4-triazin-3(2*H*)-one, 4-(2-bromophenyl)-2,6-diphenyl-4,5-dihydro-1,2,4-triazin-3(2*H*)-one, 2-(2-bromophenyl)-4-(2-bromophenyl)-6-phenyl-4,5-dihydro-1,2,4-triazin-3(2*H*)-one, 4-(2-bromophenyl)-6-(2-methoxyphenyl)-2-phenyl-4,5-dihydro-1,2,4-triazin-3(2*H*)-one, 2-(2-bromophenyl)-4-(2,5-dimethoxyphenyl)-6-(2-methoxyphenyl)-4,5-dihydro-1,2,4-triazin-3(2*H*)-one, 4-(2,5-dimethoxyphenyl)-6-(2-methoxyphenyl)-2-phenyl-4,5-dihydro-1,2,4-triazin-3(2*H*)-one, 2-(2-bromophenyl)-6-(2-pyridyl)-4-(2-pyridyl)-4,5-dihydro-1,2,4-triazin-3(2*H*)-one, 2-phenyl-4-phenyl-6-(2-pyrimidinyl)-4,5-dihydro-1,2,4-triazin-3(2*H*)-one, 2-(2-bromophenyl)-4-(4-biphenyl)-6-(2-pyridyl)-4,5-dihydro-1,2,4-triazin-3(2*H*)-one, 2-(2-bromophenyl)-4-(3-nitrophenyl)-6-(2-pyridyl)-4,5-dihydro-1,2,4-triazin-3(2*H*)-one, 2-(2-bromophenyl)-4-(4-fluorophenyl)-6-(2-pyridyl)-4,5-dihydro-1,2,4-triazin-3(2*H*)-one, 2-(2-bromophenyl)-4-(3-formylphenyl)-6-(2-pyridyl)-4,5-dihydro-1,2,4-triazin-3(2*H*)-one, 2-(2-bromophenyl)-4-(3-tolyl)-6-(2-pyridyl)-4,5-dihydro-1,2,4-triazin-3(2*H*)-one, 2-(2-bromophenyl)-4-(4-thiomethoxyphenyl)-6-(2-pyridyl)-4,5-dihydro-1,2,4-triazin-3(2H)-one, 2-(2-bromophenyl)-4-(2-chloropyridin-5-yl)-6-(2-pyridyl)-4,5-dihydro-1,2,4-triazin-3(2*H*)-one, 2-(2-cyanophenyl)-4-(3-nitrophenyl)-6-(2-pyridyl)-4,5-dihydro-1,2,4-triazin-3(2*H*)-one, 2-(2-cyanophenyl)-4-(3-aminophenyl)-6-(2-pyridyl)-4,5-dihydro-1,2,4-triazin-3(2*H*)-one, and 2-(2-chlorophenyl)-4-phenyl-6-(2-pyrimidinyl)-4,5-dihydro-1,2,4-triazin-3(2*H*)-one.

7. A pharmaceutical composition comprising the compound represented by formula (VIII) in claim 1, a salt thereof or a hydrate of them as the active ingredient, and a pharmacologically acceptable carrier.

8. Use of the compound represented by formula (VIII) in claim 1, a salt thereof or a hydrate of them for producing an agent for treating or preventing a disease selected from multiple sclerosis and epilepsy.

## Patentansprüche

1. Verbindung der folgenden Formel, ein Salz davon oder ein Hydrat von diesen: 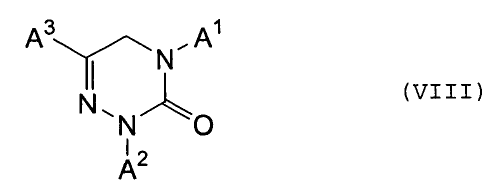 worin A¹, A² und A³ unabhängig voneinander sind und jeweils eine C₆₋₁₄ aromatische cyclische Kohlenwasserstoffgruppe oder eine 5- bis 14-gliedrige aromatische heterocyclische Gruppe darstellen, von denen jede mit einer oder mehreren Gruppen substituiert sein kann, ausgewählt aus der Gruppe bestehend aus (1) einer Hydroxylgruppe, (2) einem Halogenatom, (3) einer Nitrilgruppe, (4) einer Nitrogruppe, (5) einer C₁₋₆-Alkylgruppe, C₂₋₆-Alkenylgruppe oder C₂₋₆-Alkinylgruppe, von denen jede mit mindestens einer Gruppe substituiert sein kann, ausgewählt aus der Gruppe bestehend aus einer Hydroxylgruppe, Nitrilgruppe, einem Halogenatom, einer C₁-₆-Alkylaminogruppe, Di(C₁₋₆-alkyl)aminogruppe, C₂₋₆-Alkenylaminogruppe, Di(C₂₋₆-alkenyl)aminogruppe, C₂₋₆-Alkinylaminogruppe, Di(C₂₋₆-alkinyl)aminogruppe, N-C₁₋₆-Alkyl-N-C₂₋₆-alkenylaminogruppe, N-C₁₋₆-Alkyl-N-C₂₋₆-alkinylaminogruppe, N-C₂₋₆-Alkenyl-N-C₂₋₆-Alkinylaminogruppe, Aralkyloxygruppe, TBDMS-Oxygruppe, C₁₋₆-Alkylsulfonylaminogruppe, C₁₋₆-Alkylcarbonyloxygruppe, C₂₋₆-Alkenylcarbonyloxygruppe, C₂₋₆-Alkinylcarbonyloxygruppe, N-C₁₋₆-Alkylcarbamoylgruppe, N-C₂₋₆-Alkenylcarbamoylgruppe und N-C₂₋₆-Alkinylcarbamoylgruppe, (6) einer C₁₋₆-Alkoxygruppe, C₂₋₆-Alkenyloxygruppe oder C₂₋₆-Alkinyloxygruppe, von denen jede mit mindestens einer Gruppe substituiert sein kann, ausgewählt aus der Gruppe bestehend aus einer C₁₋₆-Alkylaminogruppe, Aralkyloxygruppe und Hydroxylgruppe, (7) einer C₁₋₆-Alkylthiogruppe, C₂₋₆-Alkenylthiogruppe oder C₂₋₆-Alkinylthiogruppe, von denen jede mit mindestens einer Gruppe substituiert sein kann, ausgewählt aus der Gruppe bestehend aus einer Hydroxylgruppe, Nitrilgruppe, einem Halogenatom, einer C₁₋₆-Alkylaminogruppe, Aralkyloxygruppe, TBDMS-Oxygruppe, C₁₋₆-Alkylsulfonylaminogruppe,C₁₋₆-Alkylcarbonyloxygruppe und C₁₋₆-Alkylcarbamoylgruppe, (8) einer Carbonylgruppe substituiert mit einer Gruppe ausgewählt aus der Gruppe bestehend aus einer C₁₋₆-Alkoxygruppe, Aminogruppe, C₁₋₆-Alkylaminogruppe, Di(C₁₋₆-alkyl)aminogruppe, C₂₋₆-Alkenylaminogruppe, Di(C₂₋₆-alkenyl)aminogruppe, C₂₋₆-Alkinylaminogruppe, Di(C₂₋₆-alkinyl)aminogruppe, N-C₁₋₆-Alkyl-N-C₂₋₆-alkenylaminogruppe, N-C₁₋₆-Alkyl-N-C₂₋₆-alkinylaminogruppe und N-C₂₋₆-Alkenyl-N-C₂₋₆-alkinylaminogruppe, (9) einer Aminogruppe, die mit einer oder zwei Gruppen substituiert sein kann, ausgewählt aus der Gruppe bestehend aus einer C₁₋₆-Alkylgruppe, C₂₋₆-Alkenylgruppe, C₂₋₆-Alkinylgruppe, C₁₋₆-Alkylsulfonylgruppe, C₂₋₆-Alkenylsulfonylgruppe, C₂₋₆-Alkinylsulfonylgruppe, C₁₋₆-Alkylcarbonylgruppe, C₂₋₆-Alkenylcarbonylgruppe und C₂₋₆-Alkinylcarbonylgruppe, (10) einer C₁₋₆-Alkylsulfonylgruppe, (11) einer C₂₋₆-Alkenylsulfonylgruppe, (12) einer C₂₋₆-Alkinylsulfonylgruppe, (13) einer C₁₋₆-Alkylsulfinylgruppe, (14) einer C₂₋₆-Alkenylsulfinylgruppe, (15) einer C₂₋₆-Alkinylsulfinylgruppe, (16) einer Formylgruppe, (17) einer C₃₋₈-Cycloalkylgruppe oder C₃₋₈-Cycloalkenylgruppe, von denen jede mit mindestens einer Gruppe substituiert sein kann, ausgewählt aus der Gruppe bestehend aus einer Hydroxylgruppe, einem Halogenatom; einer Nitrilgruppe, C₁₋₆-Alkylgruppe, C₁₋₆-Alkoxygruppe, C₁₋₆-Alkoxy-C₁₋₆-alkylgruppe und Aralkylgruppe, (18) einer 5- bis 14-gliedrigen nichtaromatischen heterocyclischen Gruppe, die mit mindestens einer Gruppe substituiert sein kann, ausgewählt aus der Gruppe bestehend aus einer Hydroxylgruppe, einem Halogenatom, einer Nitrilgruppe, C₁₋₆-Alkylgruppe, C₁₋₆-Alkoxygruppe, C₁₋₆-Alkoxy-C₁₋₆-alkylgruppe und Aralkylgruppe, (19) einer C₆₋₁₄ aromatischen cyclischen Kohlenwasserstoffgruppe, die mit mindestens einer Gruppe substituiert sein kann, ausgewählt aus der Gruppe bestehend aus einer Hydroxylgruppe, einem Halogenatom, einer Nitrilgruppe, C₁₋₆-Alkylgruppe, C₁₋₆-Alkoxygruppe, C₁₋₆-Alkoxy-C₁₋₆-alkylgruppe und Aralkylgruppe, (20) einer 5- bis 14-gliedrigen aromatischen heterocyclischen Gruppe, die mit mindestens einer Gruppe substituiert sein kann, ausgewählt aus der Gruppe bestehend aus einer Hydroxylgruppe, einem Halogenatom, einer Nitrilgruppe, C₁₋₆-Alkylgruppe, C₁₋₆-Alkoxygruppe, C₁₋₆-Alkoxy-C₁₋₆-alkylgruppe und Aralkylgruppe, und (21) einer Thiolgruppe,
vorausgesetzt, daß die Verbindungen in den folgenden Fällen (1) bis (3) ausgeschlossen sind:
(1) der Fall, wo jedes von A¹, A² und A³ eine Phenylgruppe ist,
(2) der Fall, wo A¹ eine o,p-Dimethylphenylgruppe ist; A² eine o-Methylphenylgruppe ist und A³ eine Phenylgruppe ist, und
(3) der Fall, wo A¹ eine o-Methylphenylgruppe ist; A² eine p-Methoxyphenylgruppe ist und A³ eine Phenylgruppe ist.

2. Verbindung gemäß Anspruch 1, ein Salz davon oder ein Hydrat von diesen, worin A¹, A² und A³ unabhängig voneinander sind und jedes eine Phenylgruppe, Pyrroylgruppe, Pyridylgruppe, Pyridazinylgruppe, Pyrimidinylgruppe, Pyrazinylgruppe, Thienylgruppe, Thiazolylgruppe, Furylgruppe, Naphthylgruppe, Chinolylgruppe, Isochinolylgruppe, Indolylgruppe, Benzimidazolylgruppe, Benzothiazolylgruppe, Benzoxazolylgruppe, Imidazopyridylgruppe oder Carbazolylgruppe darstellt, von denen jede substituiert sein kann.

3. Verbindung gemäß Anspruch 1, ein Salz davon oder ein Hydrat von diesen, worin A¹, A² und A³ unabhängig voneinander sind und jeweils eine Gruppe der Formel: 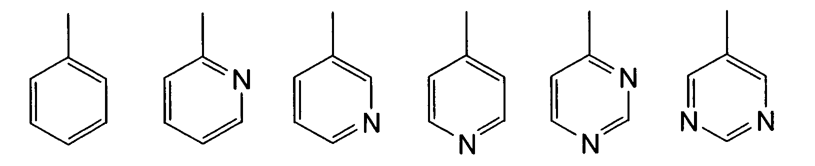 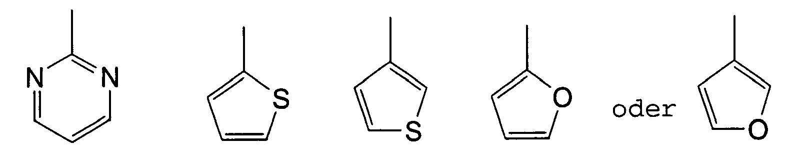 darstellen, von denen jede substituiert sein kann.

4. Verbindung gemäß Anspruch 1, ein Salz davon oder ein Hydrat von diesen, worin jedes von A¹, A² und A³ mit mindestens einer Gruppe substituiert sein kann, unabhängig ausgewählt aus der Gruppe bestehend aus einem Halogenatom, einer Nitrilgruppe, einer Hydroxylgruppe, Aminogruppe, Formylgruppe und Nitrogruppe.

5. Verbindung gemäß Anspruch 1, ein Salz davon oder ein Hydrat von diesen, worin die Bindungsstellen der Substituentengruppen an A¹, A² und/oder A³ α-Positionen der Kohlenstoffatome an A¹, A² und/oder A³ sind, von denen jedes direkt an den Triazinonring gebunden ist.

6. Verbindung gemäß Anspruch 1, ein Salz davon oder ein Hydrat von diesen, die eine Verbindung ist, ausgewählt aus:
2-(2-Bromphenyl)-4-(2-methoxyphenyl)-6-(2-pyridyl)-4,5-dihydro-1,2,4-triazin-3(2H)-on,
2-(2-Bromphenyl)-4-(2-hydroxyphenyl)-6-(2-pyridyl)-4,5-dihydro-1,2,4-triazin-3(2H)-on,
2-(2-Cyanophenyl)-4-(2-methoxyphenyl)-6-(2-pyridyl)-4,5-dihydro-1,2,4-triazin-3(2H)-on,
2-(2-Bromphenyl)-4-phenyl-6-(2-pyridyl)-4,5-dihydro-1,2,4-triazin-3(2H)-on,
2-(2-Bromphenyl)-6-(2-methoxyphenyl)-4-phenyl-4,5-dihydro-1,2,4-triazin-3(2H)-on,
2-(2-Bromphenyl)-6-(2-hydroxyphenyl)-4-phenyl-4,5-dihydro-1,2,4-triazin-3(2H)-on,
2-(2-Bromphenyl)-6-(2-dimethylaminoethoxyphenyl)-4-phenyl-4,5-dihydro-1,2,4-triazin-3(2H)-on,
2-(2-Bromphenyl)-6-(2-methoxyphenyl)-4-(2-pyridyl)-4,5-dihydro-1,2,4-triazin-3(2H)-on,
2-(2-Cyanophenyl)-6-(2-hydroxyphenyl)-4-phenyl-4,5-dihydro-1,2,4-triazin-3(2H)-on,
2-(2-Bromphenyl)-4-(2,5-dihydroxyphenyl)-6-(2-hydroxyphenyl)-4,5-dihydro-1,2,4-triazin-3(2H)-on,
4-(2,5-Dihydroxyphenyl)-6-(2-hydroxyphenyl)-2-phenyl-4,5-dihydro-1,2,4-triazin-3(2H)-on,
2-(2-Cyanophenyl)-4-(2-hydroxyphenyl)-6-(2-pyridyl)-4,5-dihydro-1,2,4-triazin-3(2H)-on,
2-(2-Cyanophenyl)-6-(2-methoxyphenyl)-4-phenyl-4,5-dihydro-1,2,4-triazin-3(2H)-on,
4-(2-Cyanophenyl)-6-(2-methoxyphenyl)-2-phenyl-4,5-dihydro-1,2,4-triazin-3(2H)-on,
2-(2-Cyanophenyl-6-(2-methoxyphenyl)-4-(2-pyridyl)-4,5-dihydro-1,2,4-triazin-3(2H)-on,
2-(2-Cyanophenyl)-4-phenyl-6-(2-pyridyl)-4,5-dihydro-1,2,4-triazin-3(2H)-on,
2-(2-Cyanophenyl)-6-(2-pyridyl)-4-(thiophen-3-yl)-4,5-dihydro-1,2,4-triazin-3(2H)-on,
2-(2-Cyanophenyl)-6-(2-pyridyl)-4-(2-pyridyl)-4,5-dihydro-1,2,4-triazin-3(2H)-on,
2-(2-Cyanophenyl)-6-(2-pyridyl)-4-(3-pyridyl)-4,5-dihydro-1,2,4-triazin-3(2H)-on,
4-(2-Cyanophenyl)-2-phenyl-6-(2-pyridyl)-4,5-dihydro-1,2,4-triazin-3(2H)-on,
2-Phenyl-6-(2-pyridyl)-4-(thiophen-3-yl)-4,5-dihydro-1,2,4-triazin-3(2H)-on,
2-(2-Bromphenyl)-6-(2-pyridyl)-4-(thiophen-3-yl)-4,5-dihydro-1,2,4-triazin-3(2H)-on,
4-(2,4-Dimethoxyphenyl)-2-phenyl-6-(2-pyridyl)-4,5-dihydro-1,2,4-triazin-3(2H)-on,
2-(2-Bromphenyl)-6-(2-methoxyphenyl)-4-(thiophen-3-yl)-4,5-dihydro-1,2,4-triazin-3(2H)-on,
2-Phenyl-6-(2-pyridyl)-4-(3-pyridyl)-4,5-dihydro-1,2,4-triazin-3(2H)-on,
2-(2-Bromphenyl)-6-(2-pyridyl)-4-(3-pyridyl)-4,5-dihydro-1,2,4-triazin-3(2H)-on,
2-(2-Bromphenyl)-4-(2-cyanophenyl)-6-(2-pyridyl)-4,5-dihydro-1,2,4-triazin-3(2H)-on,
2-(2-Bromphenyl)-4,6-diphenyl-4,5-dihydro-1,2,4-triazin-3(2H)-on,
4-(2-Bromphenyl)-2,6-diphenyl-4,5-dihydro-1,2,4-triazin-3(2H)-on,
2-(2-Bromophenyl)-4-(2-bromphenyl)-6-phenyl-4,5-dihydro-1,2,4-triazin-3(2H)-on,
4-(2-Bromphenyl)-6-(2-methoxyphenyl)-2-phenyl-4,5-dihydro-1,2,4-triazin-3(2H)-on,
2-(2-Bromphenyl)-4-(2,5-dimethoxyphenyl)-6-(2-methoxyphenyl)-4,5-dihydro-1,2,4-triazin-3(2H)-on,
4-(2,5-Dimethoxyphenyl)-6-(2-methoxyphenyl)-2-phenyl-4,5-dihydro-1,2,4-triazin-3(2H)-on,
2-(2-Bromphenyl)-6-(2-pyridyl)-4-(2-pyridyl)-4,5-dihydro-1,2,4-triazin-3(2H)-on,
2-Phenyl-4-phenyl-6-(2-pyrimidinyl)-4,5-dihydro-1,2,4-triazin-3(2H)-on,
2-(2-Bromphenyl)-4-(4-biphenyl)-6-(2-pyridyl)-4,5-dihydro-1,2,4-triazin-3(2H)-on,
2-(2-Bromphenyl)-4-(3-nitrophenyl)-6-(2-pyridyl)-4,5-dihydro-1,2,4-triazin-3(2H)-on,
2-(2-Bromphenyl)-4-(4-fluorphenyl)-6-(2-pyridyl)-4,5-dihydro-1,2,4-triazin-3(2H)-on,
2-(2-Bromphenyl)-4-(3-formylphenyl)-6-(2-pyridyl)-4,5-dihydro-1,2,4-triazin-3(2H)-on,
2-(2-Bromphenyl)-4-(3-tolyl)-6-(2-pyridyl)-4,5-dihydro-1,2,4-triazin-3(2H)-on,
2-(2-Bromphenyl)-4-(4-thiomethoxyphenyl)-6-(2-pyridyl)-4,5-dihydro-1,2,4-triazin-3(2H)-on,
2-(2-Bromphenyl)-4-(2-chlorpyridin-5-yl)-6-(2-pyridyl)-4,5-dihydro-1,2,4-triazin-3(2H)-on,
2-(2-Cyanophenyl)-4-(3-nitrophenyl)-6-(2-pyridyl)-4,5-dihydro-1,2,4-triazin-3(2H)-on,
2-(2-Cyanophenyl)-4-(3-aminophenyl)-6-(2-pyridyl)-4,5-dihydro-1,2,4-triazin-3(2H)-on und
2-(2-Chlorphenyl)-4-phenyl-6-(2-pyrimidinyl)-4,5-dihydro-1,2,4-triazin-3(2H)-on.

7. Pharmazeutische Zusammensetzung umfassend die Verbindung dargestellt durch die Formel (VIII) in Anspruch 1, ein Salz davon oder ein Hydrat von diesen als wirksamen Bestandteil und einen pharmakologisch akzeptablen Träger.

8. Verwendung einer Verbindung dargestellt durch die Formel (VIII) in Anspruch 1, eines Salzes davon oder eines Hydrat von diesen zur Herstellung eines Mittels für die Behandlung oder Verhinderung einer Erkrankung ausgewählt aus multipler Sklerose und Epilepsie.

## Revendications

1. Composé représenté par la formule suivante, sel de celui-ci ou hydrate de ceux-ci : 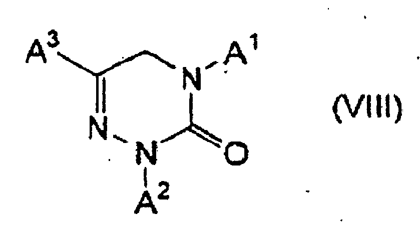 dans laquelle A¹, A² et A³ sont indépendants les uns des autres et chacun représente un groupe cyclique hydrocarbure aromatique en C₆₋₁₄ ou un groupe hétérocyclique aromatique de 5 à 14 éléments, dont chacun peut être substitué avec un ou plusieurs groupes choisi(s) parmi le groupe consistant en (1) un groupe hydroxyle, (2) un atome d'halogène, (3) un groupe nitrile, (4) un groupe nitro, (5) un groupe alkyle en C₁₋₆, un groupe alcényle en C₂₋₆ ou un groupe alcynyle en C₂₋₆, dont chacun peut être substitué avec au moins un groupe choisi parmi le groupe consistant en un groupe hydroxyle, un groupe nitrile, un atome d'halogène, un groupe alkylamino en C₁₋₆, un groupe di(alkyl en C₁₋₆) amino, un groupe alcénylamino en C₂₋₆, un groupe di(alcényl en C₂₋₆) amino, un groupe alcynylamino en C₂₋₆, un groupe di (alcynyl en C₂₋₆) amino, un groupe N-alkyl en C₁₋₆-N-alcénylamino en C₂₋₆, un groupe N-alkyl en C₁₋₆-N-alcynylamino en C₂₋₆, un groupe N-alcényl en C₂₋₆-N-alcynylamino en C₂₋₆, un groupe aralkyloxy, un groupe TBDMS oxy, un groupe alkylsulfonylamino en C₁₋₆, un groupe alkylcarbonyloxy en C₁₋₆, un groupe alcénylcarbonyloxy en C₂₋₆, un groupe alcynylcarbonyloxy en C₂₋₆, un groupe N-alkylcarbamoyle en C₁₋₆, un groupe N-alcénylcarbamoyle en C₂₋₆ et un groupe N-alcynylcarbamoyle en C₂₋₆, (6) un groupe alcoxy en C₁₋₆, un groupe alcényloxy en C₂₋₆ ou un groupe alcynyloxy en C₂₋₆, dont chacun peut être substitué avec au moins un groupe choisi parmi le groupe consistant en un groupe alkylamino en C₁₋₆, un groupe aralkyloxy et un groupe hydroxyle, (7) un groupe alkylthio en C₁₋₆, un groupe alcénylthio en C₂₋₆ ou un groupe alcynylthio en C₂₋₆, dont chacun peut être substitué avec au moins un groupe choisi parmi le groupe consistant en un groupe hydroxyle, un groupe nitrile, un atome d'halogène, un groupe alkylamino en C₁₋₆, un groupe aralkyloxy, un groupe TBDMS oxy, un groupe alkylsulfonylamino en C₁₋₆, un groupe alkylcarbonyloxy en C₁₋₆ et un groupe alkylcarbamoyle en C₁₋₆, (8) un groupe carbonyle substitué avec un groupe choisi parmi le groupe consistant en un groupe alcoxy en C₁₋₆, un groupe amino, un groupe alkylamino en C₁₋₆, un groupe di(alkyl en C₁₋₆)amino, un groupe alcénylamino en C₂₋₆, un groupe di(alcényl en C₂₋₆) amino, un groupe alcynylamino en C₂₋₆, un groupe di (alcynyl en C₂₋₆)amino, un groupe N-alkyle en C₁₋₆-N-alcénylamino en C₂₋₆, un groupe N-alkyle en C₁₋₆-N-alcynylamino en C₂₋₆ et un groupe N-alcényle en C₂₋₆-N-alcynylamino en C₂₋₆, (9) un groupe amino qui peut être substitué avec un ou deux groupe(s) choisi(s) parmi le groupe consistant en un groupe alkyle en C₁₋₆, un groupe alcényle en C₂₋₆, un groupe alcynyle en C₂₋₆, un groupe alkylsulfonyle en C₁₋₆, un groupe alcénylsulfonyle en C₂₋₆, un groupe alcynylsulfonyle en C₂₋₆, un groupe alkylcarbonyle en C₁₋₆, un groupe alcénylcarbonyle en C₂₋₆, et un groupe alcynylcarbonyle en C₂₋₆, (10) un groupe alkylsulfonyle en C₁₋₆, (11) un groupe alcénylsulfonyle en C₂₋₆, (12) un groupe alcynylsulfonyle en C₂₋₆, (13) un groupe alkylsulfinyle en C₁₋₆, (14) un groupe alcénylsulfinyle en C₁₋₆, (15) un groupe alcynylsulfinyle en C₂₋₆, (16) un groupe formyle, (17) un groupe cycloalkyle en C₃₋₈ ou un groupe cycloalcényle en C₃₋₈, dont chacun peut être substitué avec au moins un groupe choisi parmi le groupe consistant en un groupe hydroxyle, un atome d'halogène, un groupe nitrile, un groupe alkyle en C₁₋₆, un groupe alcoxy en C₁₋₆, un groupe alcoxy en C₁₋₆-alkyle en C₁₋₆ et un groupe aralkyle, (18) un groupe hétérocyclique non aromatique de 5 à 14 éléments qui peut être substitué avec au moins un groupe choisi parmi le groupe consistant en un groupe hydroxyle, un atome d'halogène, un groupe nitrile, un groupe alkyle en C₁₋₆, un groupe alcoxy en C₁₋₆, un groupe alcoxy en C₁₋₆-alkyle en C₁₋₆ et un groupe aralkyle, (19) un groupe cyclique hydrocarbure aromatique en C₅₋₁₄ qui peut être substitué avec au moins un groupe choisi parmi le groupe consistant en un groupe hydroxyle, un atome d'halogène, un groupe nitrile, un groupe alkyle en C₁₋₆, un groupe alcoxy en C₁₋₆, un groupe alcoxy en C₁₋₆-alkyle en C₁₋₆ et un groupe aralkyle, et (20) un groupe hétérocyclique aromatique de 5 à 14 éléments qui peut être substitué avec au moins un groupe choisi parmi le groupe consistant en un groupe hydroxyle, un atome d'halogène, un groupe nitrile, un groupe alkyle en C₁₋₆, un groupe alcoxy en C₁₋₆, un groupe alcoxy en C₁₋₆-alkyle en C₁₋₆ et un groupe aralkyle, et (21) un groupe thiol,
sous réserve que les composés dans les cas (1) à (3) suivants sont exclus :
(1) le cas dans lequel chacun de A¹, A² et A³ est un groupe phényle,
(2) le cas dans lequel A¹ est un groupe o,p-diméthylphényle ; A² est un groupe o-méthylphényle ; et A³ est un groupe phényle, et
(3) le cas dans lequel A¹ est un groupe o-méthylphényle ; A² est un groupe p-méthoxyphényle ; et A³ est un groupe phényle.

2. Composé selon la revendication 1, sel de celui-ci ou hydrate de ceux-ci, dans lequel A¹, A² et A³ sont indépendants les uns des autres et chacun représente un groupe phényle, un groupe pyrrolyle, un groupe pyridyle, un groupe pyridazinyle, un groupe pyrimidinyle, un groupe pyrazinyle, un groupe thienyle, un groupe thiazolyle, un groupe furyle, un groupe naphtyle, un groupe quinolyle, un groupe isoquinolyle, un groupe indolyle, un groupe benzimidazolyle, un groupe benzothiazolyle, un groupe benzoxazolyle, un groupe imidazopyridyle, ou un groupe carbazolyle, dont chacun peut être substitué.

3. Composé selon la revendication 1, sel de celui-ci ou hydrate de ceux-ci, dans lequel A¹, A² et A³ sont indépendants les uns des autres et chacun représente un groupe représenté par la formule : 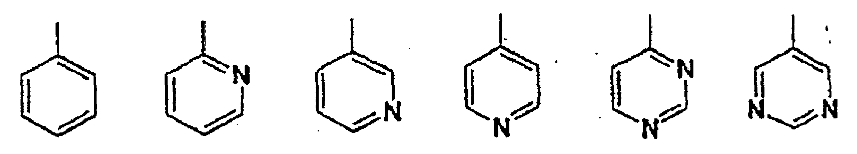 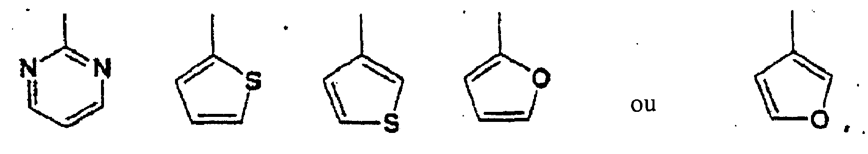 dont chacun peut être substitué.

4. Composé selon la revendication 1, sel de celui-ci ou hydrate de ceux-ci, dans lequel chacun de A¹, A² et A³ peut être substitué avec au moins un groupe choisi indépendamment parmi le groupe consistant en un atome d'halogène, un groupe nitrile, un groupe hydroxyle, un groupe amino, un groupe formyle et un groupe nitro.

5. Composé selon la revendication 1, sel de celui-ci ou hydrate de ceux-ci, dans lequel les positions de liaison des groupes substituants sur A¹, A² et/ou A³ sont les positions α des atomes de carbone sur A¹, A² et/ou A³, dont chacun est lié directement au cycle triazinone.

6. Composé selon la revendication 1, sel de celui-ci ou hydrate de ceux-ci, qui est un composé choisi parmi :
la 2-(2-bromophényl)-4-(2-méthoxyphényl)-6-(2-pyridyl)-4,5-dihydro-1,2,4-triazin-3(2*H*)-one,
la 2-(2-bromophényl)-4-(2-hydroxyphényl)-6-(2-pyridyl)-4,5-dihydro-1,2,4-triazin-3(2*H*)-one,
la 2-(2-cyanophényl)-4-(2-méthoxyphényl)-6-(2-pyridyl)-4,5-dihydro-1,2,4-triazin-3(2*H*)-one,
la 2-(2-bromophényl)-4-phényl-6-(2-pyridyl)-4,5-dihydro-1,2,4-triazin-3(2*H*)-one,
la 2-(2-bromophényl)-6-(2-méthoxyphényl)-4-phényl-4,5-dihydro-1,2,4-triazin-3(2*H*)-one,
la 2-(2-bromophényl)-6-(2-hydroxyphényl)-4-phényl-4,5-dihydro-1,2,4-triazin-3(2*H*)-one,
la 2-(2-bromophényl)-6-(2-diméthylaminoéthoxyphényl)-4-phényl-4,5-dihydro-1,2,4-triazin-3(2H)-one,
la 2-(2-bromophényl)-6-(2-méthoxyphényl)-4-(2-pyridyl)-4,5-dihydro-1,2,4-triazin-3(2*H*)-one,
la 2-(2-cyanophényl)-6-(2-hydroxyphényl)-4-phényl-4,5-dihydro-1,2,4-triazin-3(2*H*)-one,
la 2-(2-bromophényl)-4-(2,5-dihydroxyphényl)-6-(2-hydroxyphényl)-4,5-dihydro-1,2,4-triazin-3(2H)-one,
la 4-(2,5-dihydroxyphényl)-6-(2-hydroxyphényl)-2-phényl-4,5-dihydro-1,2,4-triazin-3(2*H*)-one,
la 2-(2-cyanophényl)-4-(2-hydroxyphény)-6-(2-pyridyl)-4,5-dihydro-1,2,4-triazin-3(2*H*)-one,
la 2-(2-cyanophényl)-6-(2-méthoxyphényl)-4-phényl-4,5-dihydro-1,2,4-triazin-3(2*H*)-one,
la 4-(2-cyanophényl)-6-(2-méthoxyphényl)-2-phényl-4,5-dihydro-1,2,4-triazin-3(2*H*)-one,
la 2-(2-cyanophényl)-6-(2-méthoxyphényl)-4-(2-pyridyl)-4,5-dihydro-1,2,4-triazin-3(2*H*)-one,
la 2-(2-cyanophényl)-4-phényl-6-(2-pyridyl)-4,5-dihydro-1,2,4-triazin-3(2*H*)-one,
la 2-(2-cyanophényl)-6-(2-pyridyl)-4-(thiophen-3-yl)-4,5-dihydro-1,2,4-triazin-3(2*H*)-one,
la 2-(2-cyanophényl)-6-(2-pyridyl)-4-(2-pyridyl)-4,5-dihydro-1,2,4-triazin-3(2*H*)-one,
la 2-(2-cyanophényl)-6-(2-pyridyl)-4-(3-pyridyl)-4,5-dihydro-1,2,4-triazin-3(2*H*)-one,
la 4-(2-cyanophényl)-2-phényl-6-(2-pyridyl)-4,5-dihydro-1,2,4-triazin-3(2*H*)-one,
la 2-phényl-6-(2-pyridyl)-4-(thiophen-3-yl)-4,5-dihydro-1,2,4-triazin-3(2*H*)-one,
la 2-(2-bromophényl)-6-(2-pyridyl)-4-(thiophen-3-yl)-4,5-dihydro-1,2,4-triazin-3(2*H*)-one,
la 4-(2,4-diméthoxyphényl)-2-phényl-6-(2-pyridyl)-4,5-dihydro-1,2,4-triazin-3(2*H*)-one,
la 2-(2-bromophényl)-6-(2-méthoxyphényl)-4-(thiophen-3-yl)-4,5-dihydro-1,2,4-triazin-3(2*H*)-one,
la 2-phényl-6-(2-pyridyl)-4-(3-pyridyl)-4,5-dihydro-1,2,4-triazin-3(2*H*)-one,
la 2-(2-bromophényl)-6-(2-pyridyl)-4-(3-pyridyl)-4,5-dihydro-1,2,4-triazin-3(2*H*)-one,
la 2-(2-bromophényl)-4-(2-cyanophényl)-6-(2-pyridyl)-4,5-dihydro-1,2,4-triazin-3(2*H*)-one,
la 2-(2-bromophényl)-4,6-diphényl-4,5-dihydro-1,2,4-triazin-3(2*H*)-one,
la 4-(2-bromophényl)-2,6-diphényl-4,5-dihydro-1,2,4-triazin-3(2*H*)-one,
la 2-(2-bromophényl)-4-(2-bromophényl)-6-phényl-4,5-dihydro-1,2,4-triazin-3(2*H*)-one,
la 4-(2-bromophényl)-6-(2-méthoxyphényl)-2-phényl-4,5-dihydro-1,2,4-triazin-3(2*H*)-one,
la 2-(2-bromophényl)-4-(2,5-diméthoxyphényl)-6-(2-méthoxyphényl)-4,5-dihydro-1,2,4-triazin-3(2H)-one,
la 4-(2,5-diméthoxyphényl)-6-(2-méthoxyphényl)-2-phényl-4,5-dihydro-1,2,4-triazin-3(2*H*)-one,
la 2-(2-bromophényl)-6-(2-pyridyl)-4-(2-pyridyl)-4,5-dihydro-1,2,4-triazin-3(2*H*)-one,
la 2-phényl-4-phényl-6-(2-pyrimidinyl)-4,5-dihydro-1,2,4-triazin-3(2*H*)-one,
la 2-(2-bromophényl)-4-(4-biphényl)-6-(2-pyridyl)-4,5-dihydro-1,2,4-triazin-3(2*H*)-one,
la 2-(2-bromophényl)-4-(3-nitrophényl)-6-(2-pyridyl)-4,5-dihydro-1,2,4-triazin-3(2*H*)-one,
la 2-(2-bromophényl)-4-(4-fluorophényl)-6-(2-pyridyl)-4,5-dihydro-1,2,4-triazin-3(2*H*)-one,
la 2-(2-bromophényl)-4-(3-formylphényl)-6-(2-pyridyl)-4,5-dihydro-1,2,4-triazin-3(2*H*)-one,
la 2-(2-bromophényl)-4-(3-tolyl)-6-(2-pyridyl)-4,5-dihydro-1,2,4-triazin-3(2*H*)-one,
la 2-(2-bromophényl)-4-(4-thiométhoxyphényl)-6-(2-pyridyl)-4,5-dihydro-1,2,4-triazin-3(2*H*)-one,
la 2-(2-bromophényl)-4-(2-chloropyridin-5-yl)-6-(2-pyridyl)-4,5-dihydro-1,2,4-triazin-3(2*H*)-one,
la 2-(2-cyanophényl)-4-(3-nitrophenyl)-6-(2-pyridyl)-4,5-dihydro-1,2,4-triazin-3(2*H*)-one,
la 2-(2-cyanophényl)-4-(3-aminophenyl)-6-(2-pyridyl)-4,5-dihydro-1,2,4-triazin-3(2*H*)-one, et
la 2-(2-chlorophényl)-4-phényl-6-(2-pyrimidinyl)-4,5-dihydro-1,2,4-triazin-3(2*H*)-one.

7. Composition pharmaceutique comprenant le composé représenté par la formule (VIII) dans la revendication 1, un sel de celui-ci ou un hydrate de ceux-ci en tant qu'ingrédient actif, et un véhicule pharmacologiquement acceptable.

8. Utilisation du composé représenté par la formule (VIII) dans la revendication 1, d'un sel de celui-ci ou d'un hydrate de ceux-ci pour produire un agent pour traiter ou prévenir une maladie choisie parmi la sclérose en plaques et l'épilepsie.
